# EUROPEAN PATENT APPLICATION

(11) **EP 3 502 109 A1**
(43) Date of publication of application: **26.06.2019**
(21) Application number: 17382868.2
(22) Date of filing: 20.12.2017
(51) Int. Cl.: C07D 417/04, C07D 417/14, A61K 31/426, A61P 33/02

(54) **TRIAZOLE-PHENYL-THIAZOLE HETEROCYCLES AS INNOVATIVE INHIBITORS OF TRYPANOTHIONE REDUCTASE AND THEIR USE AS LEISHMANICIDES**

(71) Applicant: Consejo Superior De Investigaciones Científicas, 28006 Madrid (ES); Universidad de Alcalá, 28801 Madrid (ES)
(72) Inventor: CAMARASA RIUS, Mª José, 28006 MADRID (ES); VELÁZQUEZ DÍAZ, Sonsoles, 28006 MADRID (ES); REVUELTO PÉREZ, Alejandro, 28006 MADRID (ES); GAGO BADENAS, Federico, 28801 ALCALÁ DE HENARES (MADRID) (ES); JIMÉNEZ RUIZ, Antonio, 28801 ALCALÁ DE HENARES (MADRID) (ES); TORO LONDOÑO, Miguel Ángel, 28801 ALCALÁ DE HENARES (MADRID) (ES); SÁNCHEZ MURCIA, Pedro Alejandro, 28801 ALCALÁ DE HENARES (MADRID) (ES); LUCIO ORTEGA, Héctor Elessar de, 28801 ALCALÁ DE HENARES (MADRID) (ES)
(74) Representative: Pons

(57) **Abstract**

The present invention refers to new compounds useful in the treatment of leishmaniasis and, more particularly, to a series of 5-6-5 triazole-phenyl-thiazole heterocycles capable of inhibiting both activity and dimerization of L. *infantum* TryR in enzymatic assays at low micromolar concentrations and endowed with potent in vitro activity against promastigote and amastigote forms of *Leishmania* which indicates a good permeability across the plasma membrane of the parasites.

## Description

The present invention refers to new compounds useful in the treatment of leishmaniasis, and more particularly, to a 5-6-5 triazole-phenyl-thiazole heterocycles capable of inhibiting both activity and dimerization of *Leishmania infantum* trypanothione reductase (TryR) in enzymatic assays at low micromolar concentrations and are endowed with potent in vitro activity against the promastigote and amastigote forms of *Leishmania* which indicates a good permeability across the plasma membrane of the parasites.

### BACKGROUND ART

Leishmaniasis is a parasitic disease caused by protozoan of the genus *Leishmania spp.* which belong to the kinetoplastids. These parasites are usually transmitted to humans by the bites of female sandflies. More than 20 different *Leishmania* species have been found to cause human leishmaniasis. Based on its clinical manifestations and on the *Leishmania* species involved, the disease is classified into three types: cutaneous (CL), mucocutaneous and visceral leishmaniasis (VL). The VL form also known as kala-azar, causes the most severe disease, which can be fatal if not properly treated. VL is a systemic infection that is predominantly caused by *Leishmania donovani* and *Leishmania infantum* species and affects the liver, spleen and bone marrow. The World Health Organization (WHO) classified leishmaniasis as a major tropical disease, ranking second after malaria. About 300.000 new cases of VL arise annually and at least 35 countries have reported the occurrence of HIV coinfection which gives a significantly higher mortality rate and is an increasing concern in Europe.

As there is no vaccine currently available against leishmaniasis, drugs are the only available tool for treatment and control of both VL and CL.

Pentavalent antimonials were developed in the 1940s and have saved thousands of lives for several decades. Other drugs such as amphotericin B, pentamidine and more recently miltefosine, the only oral drug licensed, have been repositioned to treat leishmaniasis. Unfortunately, the therapeutic arsenal for leishmaniasis has become quite limited and outdated. Antimonials have many toxic effects including cardiac, hepatic, pancreatic and renal toxicity. Parenteral administration, high cost, long-term treatment and development of resistance have been associated with use of amphotericin B. Cases of resistance to miltefosine with increased treatment failures have also been reported. Therefore, there is an urgent need to find new effective innovative drugs to treat leishmaniasis.

One of the strategies to develop potent and specific antileishmanial agents is to exploit the metabolic pathways that are essential for the survival of the parasite in the host. Trypanothione reductase (TryR) is a vital enzyme for antioxidant defense in trypanosomatids and the trypanothione reductase /trypanothione (TryR/Try) couple is analogous to but distinct from the mammalian redox defense system based on the glutathione/glutathione reductase (GR) couple. The absence of TryR in mammalian cells, the significant differences between the host and parasite enzymes (the active sites of the two enzymes have opposite net charges) and its vital role for the parasite make TryR an atractive target for rational drug design. TryR is a genetically validated drug target, one important criterion in drug target assessment. Since the identification of TryR a range of potent and selective competitive TryR inhibitors that are good trypanocides *in vitro* have been described but there are scarce reports of compounds that are also effective *in vivo.* All known inhibitors target the active site through covalent inactivation of the catalytic cysteine residues or binding of policationic species in the active site. One of the disadvantages of TryR is that the survival of the parasite is only affected when its activity is reduced by more than 90%. This implies that competitive inhibitors must have a very high affinity. For this reason, non-competitive or irreversible inhibitors are known to be more attractive alternatives for this enzyme.
In this regard, an alternative inhibition strategy has been recently reported that aims to disrupt the dimer interface of the enzyme by means of designed protein-protein interaction (PPI) inhibitors of *Leishmania infantum* TryR (Li-TryR). Potential druggable sites for PPIs disruptors were explored by analysis of the interface by a combination of molecular modelling and site-directed mutagenesis studies. From a small library of linear peptides of different length derived from an α-helix spanning residues P435 to M447, which contains the identified hot spot E436, the 13-*mer* peptides PEIIQSVGIS-Nle-K-Nle and PKIIQSVGIS-Nle-K-Nle, emerged as potent Li-TryR dimerization inhibitors in the low micromolar range (ChemBioChem, 2013, 14(10), 1212-1217). However, both the linear peptide prototypes and their second-generation cyclic peptide analogues were inactive against *L. infantum* axenic amastigotes and promastigotes due to the inability of these molecules to cross the cell membranes. We later demonstrated *in vitro* activity against *L*. *infantum* parasites when these peptide-based inhibitors of TryR dimerization were conjugated with cationic cell-penetrating peptides. These results suggest that protein-protein interaction inhibition of the homodimeric TryR of *L. infantum* represents a novel alternative mechanism of inhibition of this enzyme that may also be a viable route for the development of new leishmanicidal agents. However, the design of small-molecules with better drug-like properties than the prototype peptides would be desirable.

### SUMMARY OF THE INVENTION

The present invention discloses a series of novel 5-6-5 triazole-phenyl-thiazole heterocycles of general formula **I** which encompasses two families of compounds represented by general formulae **I-A** and **I-B** that can be used to treat leishmaniasis.

These molecules represent the first non-peptidic inhibitors of TryR of *L*. *infantum* that disrupt the PPIs of this homodimeric enzyme and exhibit potent leishmanicidal activity against extracellular and intracellular forms of the parasites.

The new compounds are able to inhibit both activity and dimerization of *L*. *infantum* TryR in enzymatic assays at low micromolar concentrations, and are endowed with potent *in vitro* activity against promastigotes and amastigotes forms of *Leishmania* parasites, which indicates a good permeability across the plasma membrane of these parasites. The novel compounds also showed significant leishmanicidal activity against intracellular amastigotes of *L. infantum.* Furthermore, the compounds of the invention displayed interesting intrinsic fluorescence properties (green or yellow), which allow us to visualize the location of the molecules inside the parasites. This study opens new possibilities in the field of neglected diseases such as leishmaniasis.

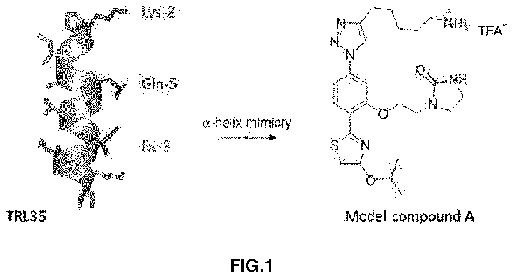

In addition, the new 5-6-5 triazole-phenyl-thiazole scaffold allows the introduction of substituents in a similar orientation to those of the side-chains of the key residues of the prototype peptides for interaction with the TryR dimer interface. Previous Ala-scanning studies and shortening of prototype linear peptides suggested that the side chains of residues Lys2, Gln5 and Ile9 in the peptides have a major contribution to their inhibitory potency. A first model compound of the invention, bearing appropriate substituents mimicking the three key residues, was designed **(****FIG. 1****).** Then, a library of 5-6-5 triazole-phenyl-thiazole derivatives with a variety of substituents (compounds of formula **(I-A),** Scheme 1) was prepared. Furthermore, triazolium cation salts have also been prepared, compounds of formula **(I-B)** (Scheme 3) by regioselective N-1 alkylation of the triazole system, in order to improve the water solubility of the compounds of the invention.

Thus, a first aspect of the present invention related to a compound of formula **(I),** a pharmaceutically acceptable salt, or solvate thereof, 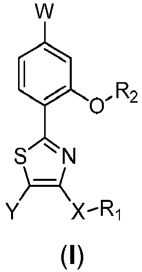 wherein:
X is selected from O, and -(CH₂)ₙ-, wherein n is a whole number in the range of from 0 to 3;
Y is selected from H, substituted or unsubstituted (C₁-C₁₀)alkyl, substituted or unsubstituted aryl, and substituted or unsubstituted heteroaryl;
R₁ is selected from substituted or unsubstituted (C₁-C₁₀)alkyl, substituted or unsubstituted aryl, and substituted or unsubstituted heteroaryl;
R₂ is selected from substituted or unsubstituted (C₁-C₄)alkyl, wherein the substituent is selected from an heterocyclylalkyl,NHCONH₂, and NHCONHR, wherein R is selected from (C₁-C₄)alkyl; and
W is a substituted or unsubstituted triazole.

In a preferred embodiment, X is -(CH₂)ₙ- and n is selected from 0 to 3.

Further preferred is a compound of formula **(I)** wherein Y is H or substituted or unsubstituted aryl; even more preferred is H or phenyl.

Further preferred is a compound of formula **(I)** wherein R₁ is selected from substituted or unsubstituted aryl, and substituted or unsubstituted heteroaryl, even more preferably is selected from the group consisting in phenyl, naphthyl, biphenyl, dihydrobenzofuranyl, tetrahydroquinolinyl, and dibenzofuranyl.

Further preferred is a compound of formula **(I)** wherein R₂ is selected from CH₃, 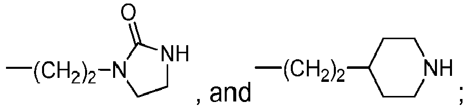 and even more preferred R₂ is

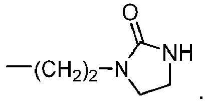

Further preferred is a compound of formula **(I),** wherein W is a substituted triazol, even more preferred is a triazole with the substituent in the position 4, and even more preferred is 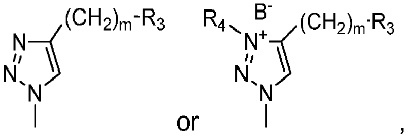 and wherein
R₃ is selected from NH₃⁺A⁻, an optionally substituted guanidinium salt, and other basic groups positively charged at physiological pH such as an optionally substituted secondary or optionally substituted tertiary amines salts and wherein the substituent is a C(₁-C₄)alkyl;
R₄ is selected from substituted or unsubstituted (C₁-C₅)alkyl and substituted or unsubstituted aryl, more preferred is (C₁-C₅)alkyl, and even more preferred is methyl or ethyl, propyl, and butyl, and even more preferred R₄ is methyl or ethyl;
m is selected from 1 to 5, more preferred from 2 to 4, and even more preferably m is 3 or 4; and
A⁻ and B⁻ independently are a counter-ion, preferably selected from trifluoroacetic acid anion, hydrochloric acid anion, sulphuric acid anion, acetic acid anion, trifluoromethanesulfonic acid anion, hydroiodic acid anion, and benzenesulfonic acid anion.

In a preferred embodiment, the invention relates to a compound of formula (**I-A),** a pharmaceutically acceptable salt, or solvate thereof, 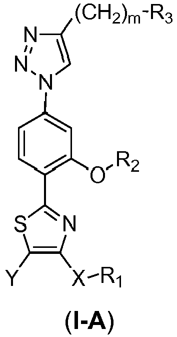 wherein:
X, Y, R₁ R₂, m and R₃ are as defined above.

Further preferred is a compound of formula **(I-A)** wherein R₃ is NH₃⁺A⁻ or an optionally substituted guanidinium salt; and even more preferred is NH₃⁺A⁻, being A⁻ as defined above.

Further preferred is a compound of formula **(I-A)** wherein m is selected from 1 to 5, and even more preferred m is 3 or 4.

Further preferred the compound of formula **(I-A)** are selected from the group as listed:
1-(2-(5-(4-(4-Aminobutyl)-1H-1,2,3-triazol-1-yl)-2-(4-isopropoxythiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-neopentylthiazol-2-yl)phenoxy)ethyl)imidazolidin-2-one 2,2,2 trifluoroacetate,
1-(2-(5-(4-(4-aminobutyl)-1H-1,2,3-triazol-1-yl)-2-(4-phenylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-benzylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-phenethylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(3-phenylpropyl)thiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(naphthalen-2-yl)ethyl)thiazol-2-yl)phenoxy)ethyl)imidazolidin-2-one 2,2,2 trifluoroacetate,
1-(2-(2-(4-(2-([1,1'-Biphenyl]-4-yl)ethyl)thiazol-2-yl)-5-(4-(4-aminobutyl)-1H-1,2,3-triazol-1-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(3,4-dihydro-2H-1λ²-quinolin-5-yl)ethyl)thiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(2,3-dihydrobenzofuran-6-yl)ethyl)thiazol-2-yl)phenoxy)ethyl)imidazolidin-2-one 2,2,2 trifluoroacetate,
1-(2-(5-(4-(4-aminobutyl)-1H-1,2,3-triazol-1-yl)-2-(4-(2-(dibenzo[b,d]furan-1-yl)ethyl)thiazol-2-yl)phenoxy)ethyl)imidazolidin-2-one 2,2,2 trifluoroacetate,
4-(1-(4-(4-phenethylthiazol-2-yl)-3-(2-(piperidin-4-yl)ethoxy)phenyl)-1H-1,2,3-triazol-4-yl)butan-1-amine 2,2,2 trifluoroacetate,
4-(1-(3-Methoxy-4-(4-phenethylthiazol-2-yl)phenyl)-1H-1,2,3-triazol-4-yl)butan-1-amine 2,2,2 trifluoroacetate,
1-(2-(5-(4-(3-aminopropyl)-1H-1,2,3-triazol-1-yl)-2-(4-phenethylthiazol-2-yl)phenoxy)ethyl)imidazolidin-2-one 2,2,2 trifluoroacetate,
1-(2-(2-(4-(2-([1,1'-Biphenyl]-4-yl)ethyl)thiazol-2-yl)-5-(4-(3-aminopropyl)-1H-1,2,3-triazol-1-yl)phenoxy)ethyl)imidazolidin-2-one 2,2,2 trifluoroacetate,
1-(2-(5-(4-(4-Aminobutyl)-1H-1,2,3-triazol-1-yl)-2-(4,5-diphenylthiazol-2-yl)phenoxy)ethyl)imidazolidin-2-one 2,2,2 trifluoroacetate, and
1-(2-(5-(4-(4-Aminobutyl)-1H-1,2,3-triazol-1-yl)-2-(4-(3-phenylpropyl)thiazol-2-yl)phenoxy)ethyl)imidazolidin-2-one hydrochloride salt.

In another preferred embodiment, the invention relates to a compound of formula **(I-B),** a pharmaceutically acceptable salt, or solvate thereof, 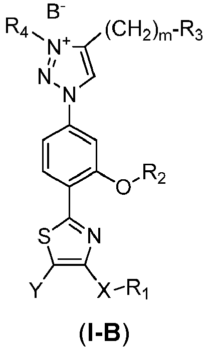 wherein:
X, Y, R₁, R₂, m, R₃, R₄, and B⁻ are as defined above.

Further preferred is a compound of formula **(I-B)** wherein R₃ is NH₃⁺TFA⁻, or an optionally substituted guanidinium salt; and even more preferred is NH₃⁺A⁻, being A⁻ as defined above.

Further preferred is a compound of formula **(I-B)** wherein R₄ is a substituted or unsubstituted (C₁-C₅)alkyl selected from a group consisting in methyl, ethyl, propyl, and butyl; even more preferred R₄ is methyl or ethyl.

Further preferred is a compound of formula **(I-B)** wherein B⁻ is trifluoromethanesulfonic acid anion or hydrochloride acid anion; even more preferred B⁻ is trifluoromethanesulfonic acid

Further preferred the compound of formula **(I-B)** are selected from the group as listed:
4-(4-Ammoniobutyl)-3-methyl-1-(3-(2-(2-oxoimidazolidin-1-yl)ethoxy)-4-(4-phenylthiazol-2-yl)phenyl)-1*H*-1,2,3-triazol-3-ium 2,2,2-trifluoroacetate trifluoromethanesulfonate,
4-(4-Ammoniobutyl)-3-methyl-1-(3-(2-(2-oxoimidazolidin-1-yl)ethoxy)-4-(4-phenethylthiazol-2-yl)phenyl)-1*H*-1,2,3-triazol-3-ium 2,2,2-trifluoroacetate trifluoromethanesulfonate,
4-(4-Ammoniobutyl)-3-methyl-1-(4-(4-(2-(naphthalen-2-yl)ethyl)thiazol-2-yl)-3-(2-(2-oxoimidazolidin-1-yl)ethoxy)phenyl)-1*H*-1,2,3-triazol-3-ium 2,2,2-trifluoroacetate trifluoromethanesulfonate,
4-(4-Ammoniobutyl)-1-(4-(4-(2-(2,3-dihydrobenzofuran-5-yl)ethyl)thiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-methyl-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-ethyl-1*H*-1,2,3-triazol-3-ium 2,2,2-trifluoroacetate trifluoromethanesulfonate,
4-(4-Ammoniobutyl)-1-(4-(4-(4-methoxyphenyl)thiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate,
4-(4-Ammoniobutyl)-1-(4-(4-(4-(diethylamino)phenyl)thiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate,
4-(3-Ammoniopropyl)-3-methyl-1-(3-(2-(2-oxoimidazolidin-1-yl)ethoxy)-4-(4-phenethylthiazol-2-yl)phenyl)-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(3-ammoniopropyl)-3-methyl-1*H*-1,2,3-triazol-3-ium 2,2,2-trifluoroacetate trifluoromethanesulfonate,
Mono (4-(2-(5-(4-(4-ammoniobutyl)-3-methyl-1*H*-1,2,3-triazol-3-ium-1-yl)-2-(4-phenethylthiazol-2-yl)phenoxy)ethyl)piperidin-1-ium) mono(2,2,2-trifluoroacetate) mono(trifluoromethanesulfonate), and
4-(4-Ammoniobutyl)-1-(4-(4,5-diphenylthiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate.

The term "alkyl" as used herein refers to a straight or branched hydrocarbon chain radical consisting solely of carbon and hydrogen atoms, containing no unsaturation, having from 1 to 10 carbon atoms, preferably from 1 to 6, and more preferably from 1 to 4 carbon atoms, and which is attached to the rest of the molecule by a single bond, e.g., methyl, ethyl, n-propyl, 1-methylethyl (isopropyl), n-butyl, n-pentyl, and 1,1-dimethylethyl (t-butyl).

The term "alkenyl" refers to an aliphatic hydrocarbon group containing a carbon-carbon double bond and which may be a straight or branched or branched chain having about 2 to about 10 carbon atoms, e.g., ethenyl, 1-propenyl, 2-propenyl (allyl), iso-propenyl, 2-methyl-1-propenyl, 1-butenyl, and 2-butenyl.

The term "alkynyl" refers to a straight or branched chain hydrocarbyl radicals having at least one carbon-carbon triple bond, and having in the range of about 2 up to 10 carbon atoms, e.g., ethynyl, propynyl, and butynyl.

The term "aryl" refers to aromatic radicals having in the range of 6 up to 20 carbon atoms such as phenyl, naphthyl, anthracenyl and biphenyl.

The term "heteroaryl" refers to an optionally substituted 5 to 14 members aromatic ring having one or more heteroatoms selected from N, O, and S as ring atoms. The heteroaryl may be a mono-, bi- or tricyclic ring system. Examples of such "heterocyclic ring" or "heteroaryl" radicals include, but are not limited to, oxazolyl, thiazolyl, imidazolyl, pyrrolyl, furanyl, pyridinyl, pyrimidinyl, pyrazinyl, benzofuranyl, indolyl, benzothiazolyl, benzoxazolyl, carbazolyl, quinolyl, tetrahydroquinolyl, isoquinolyl, azetidinyl, acridinyl, benzodioxolyl, benzodioxanyl, dihydrobenzofuranyl, dibenzofuranyl, dibenzofuranyl carbazolyl, cinnolinyl, dioxolanyl, indolizinyl, naphthyridinyl, perhydroazepinyl, phenazinyl, phenothiazinyl, phenoxazinyl, phthalazinyl, pteridinyl, purinyl, quinazolinyl, quinoxalinyl, tetrazoyl, tetrahydroisoquinolyl, piperidinyl, piperazinyl, 2-oxopiperazinyl, 2-oxopiperidinyl, 2-oxopyrrolidinyl, 2-oxoazepinyl, azepinyl, 4-piperidonyl, pyrrolidinyl, pyridazinyl, oxazolinyl, oxazolidinyl, triazolyl, indanyl, isoxazolyl, isoxazolidinyl, morpholinyl, thiazolinyl, thiazolidinyl, isothiazolyl, quinuclidinyl, isothiazolidinyl, isoindolyl, indolinyl, isoindolinyl, octahydroindolyl, octahydroisoindolyl, decahydroisoquinolyl, benzimidazolyl, thiadiazolyl, benzopyranyl, tetrahydrofuryl, tetrahydropyranyl, thienyl, benzothienyl, thiamorpholinyl, thiamorpholinyl sulfoxide, thiamorpholinyl sulfone, dioxaphospholanyl, oxadiazolyl, chromanyl, and isochromanyl. The heteroaryl ring radical may be attached to the main structure at any heteroatom or carbon atom that results in the creation of a stable structure. The term "substituted heteroaryl" also includes ring systems substituted with one or more oxide (-O-) substituents, such as pyridinyl N-oxides.

The term "heterocyclylalkyl" refers to a heterocylic ring radical as defined above directly bonded to an alkyl group. The heterocyclylalkyl radical may be attached to the main structure at carbon atom in the alkyl group that results in the creation of a stable strncture. Examples of such heterocycloalkyl radicals include, but are not limited to, dioxolanyl, thienyl[1 ,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1, 1-dioxothiomorpholinyl.

The term "substituted" unless otherwise specified, refers to substitution with any one or any combination of the following substituents and may be the same or different which one or more are selected from the groups such as OR', =O, SR', SOR', SO₂R', NO₂, NHR', N(R')₂, =N-R', NHCOR', N(COR')₂, NHSO₂R', NR'C(=NR')NHR', CN, halogen, C(O)R', COOR', OC(O)R', CONHR', CON(R')₂, substituted or unsubstituted (C₁-C₁₀)alkyl, substituted or unsubstituted (C₂-C₁₀)alkenyl, substituted or unsubstituted (C₂-C₁₀)alkynyl, substituted or unsubstituted (C₆-C₁₈)aryl, and substituted or unsubstituted (C₅-C₁₈)heteroaryl; wherein each R' is independently selected from H, OH, NO₂, NH₂, SH, CN, halogen, O, C(O)H, C(O)alkyl, COOH, substituted or unsubstituted (C₁-C₁₀)alkyl, substituted or unsubstituted (C₂-C₁₀)alkenyl, substituted or unsubstituted (C₂-C₁₀)alkynyl, substituted or unsubstituted (C₆-C₁₈)aryl, and substituted or unsubstituted (C₅-C₁₈)heteroaryl.

The term "halogen" as used herein refers to fluoro, chloro, bromo or iodo.

The term "guanidinium salt" refers to a compound of the following formula: 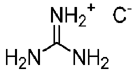 where C⁻ is an anion (e.g., trifluoroacetic acid anion, halide, nitrate, acetate, and the like). Example guanidinium salts include, for example, guanidinium trifluoroacetic, guanidinium chloride, guanidinium bromide, guanidinium iodide, guanidinium hydroxide, guanidinium acetate, guanidinium thiocyanate, guanidinium nitrate, and the like.

The term "counter-ion", as used herein, refers to an ionic moiety that carries a charge negative to the charge on the NH₃⁺. In one embodiment, the counter-ion is trifluoromethanesulfonic acid anion (triflate or TfO⁻), benzenesulfonic acid anion, trifluoroacetic acid anion (TFA⁻), or sulphuric acid anion, acetic acid anion, hydroiodic acid anion, and hydrochloric acid anion.

Pharmaceutically acceptable salts forming part of this invention include salts derived from inorganic bases such as Li, Na, K, Ca, Mg, Fe, Cu, Zn, and Mn; salts of organic bases such as N,N'-diacetylethylenediamine, glucamine, triethylamine, choline, hydroxide, dicyclohexylamine, metformin, benzylamine, trialkylamine, and thiamine; chiral bases such as alkylphenylamine, glycinol, and phenyl glycinol; salts of natural amino acids such as glycine, alanine, valine, leucine, isoleucine, norleucine, tyrosine, cystine, cysteine, methionine, proline, hydroxy proline, histidine, ornithine, lysine, arginine, and serine; quaternary ammonium salts of the compounds of invention with alkyl halides, alkyl sulphates such as Mel and (Me)₂SO₄; non-natural amino acids such as D-isomers or substituted amino acids; guanidine; and substituted guanidine wherein the substituents are selected from nitro, amino, alkyl, alkenyl, alkynyl, ammonium or substituted ammonium salts and aluminium salts. Salts may include acid addition salts where appropriate which are sulphates, nitrates, phosphates, perchlorates, borates, hydrohalides, acetates, tartrates, maleates, citrates, fumar ates, succinates, palmo ates, methanesulphonates, benzoates, salicylates, benzenesulfonates, ascorbates, glycerophosphates, and ketoglutarates.

Another aspect of the present invention related to a pharmaceutical composition comprising one or more compounds of the present invention, a pharmaceutically acceptable salt, or solvate thereof. The pharmaceutical composition may include one or more additional active ingredients as described herein. The pharmaceutical composition may be administered for any of the disorders described herein.

The subject pharmaceutical compositions are typically formulated to provide a therapeutically effective amount of a compound of the present invention as the active ingredient, or a pharmaceutically acceptable salt, or solvate thereof. Where desired, the pharmaceutical compositions contain a compound of the present invention as the active ingredient or a pharmaceutically acceptable salt and/or coordination complex thereof, and one or more pharmaceutically acceptable excipients, carriers, such as inert solid diluents and fillers, diluents, including sterile aqueous solution and various organic solvents, permeation enhancers, solubilizers and adjuvants.

The subject pharmaceutical compositions can be administered alone or in combination with one or more other agents, which are also typically administered in the form of pharmaceutical compositions. Where desired, the subject compounds and other agent(s) may be mixed into a preparation or both components may be formulated into separate preparations to use them in combination separately or at the same time. Some of this pharmaceutical agents may comprise, but not limit to, any of the following: pentavalent antimonials, amphotericin B deoxycholate, paromomycin, pentamidine isetionate, miltefosina, ketoconazole, fluconazole, itraconazole, pentamidine, suramin, eflornithine, melarsoprol, nifurtimox, antricide, prosal of antricide, piritidium bromide, protidium, isometadium chloride, homidiun bromide, cimelarsan, diminazine aceturate and isometamidium-type drugs.

Methods include administration of an inhibitor by itself, or in combination as described herein, and in each case optionally including one or more suitable diluents, fillers, salts, disintegrants, binders, lubricants, glidants, wetting agents, controlled release matrices, colorants/flavouring, carriers, excipients, buffers, stabilizers, solubilizers, and combinations thereof.

Preparations of various pharmaceutical compositions are known in the art. See, e.g., Handbook of Clinical Drug Data, Tenth Edition, McGraw-Hill, 2002; Pratt and Taylor, eds., Principies of Drug Action, Third Edition, Churchill Livingston, N.Y., 1990; Katzung, ed., Basic and Clinical Pharmacology, Ninth Edition, McGraw Hill, 2003; Goodman and Gilman, eds., The Phamlacological Basis of Therapeutics, Tenth Edition, McGraw Hill, 2001; Remingtons Pharmaceutical Sciences, 20th Ed., Lippincott Williarns & Wilkins., 2000; Martindale, The Extra Pharmacopoeia, Thirty-Second Edition (The Pharmaceutical Press, London, 1999), all of which are incorporated by reference herein in their entirety.

The compounds or pharmaceutical composition of the present invention can be administered by any route that enables delivery of the compounds to the site of action, such as oral routes, intraduodenal routes, parenteral injection (including intravenous, intraarterial, subcutaneous, intramuscular, intravascular, intraperitoneal or infusion), topical administration (e.g. transdermal application), rectal administration, via local delivery by catheter or stent or through inhalation. The compounds can also be administered intraadiposally or intrathecally.

The compositions can be administered in solid, semi-solid, liquid or gaseous form, or may be in dried powder, such as lyophilized form. The pharmaceutical compositions can be packaged in forms convenient for delivery, including, for example, so lid dosage forms such as capsules, sachets, cachets, gelatines, papers, tablets, capsules, suppositories, pellets, pills, troches, and lozenges. The type of packaging will generally depend on the desired route of administration. Implantable sustained release formulations are also contemplated, as are transdermal formulations.

A third aspect of the present invention refers to the compounds of the invention, namely, compounds of formula **(I), (I-A)** and **(I-B),** as defined above, and their pharmaceutically acceptable salts, or solvates thereof, for use as a medicament. More preferably, for use in the treatment of a disease related to an infection caused by a parasite, more preferably for use in the treatment of a disease selected from Chagas disease, trypanosomiasis or sleeping sickness, and leishmaniasis; and even more preferably for use in the treatment of leishmaniasis.

Diseases caused by parasites such as kinetoplastids are well known in the state of the art, it comprises the genuses *Trypanosoma sp.* and *Leishmania sp.* The protozoan parasite *Trypanosoma brucei* is the etiologic agent of African trypanosomiasis or sleeping sickness in human or nagana in domestic livestock. *Trypanosoma cruzi* is the etiological agent of American trypanosomiasis or Chagas disease. Leishmaniasis comprises a series of pathologies caused by different species of the *Leishmania* genus. *Leishmania donovani* is responsible for visceral leishmaniasis, one of the most severe forms of the disease.

Other kinetoplastids parasite species are: *L. major, L. tropica, L. aethiopica, L. mexicana, L. (Viannia) braziliensis, L. donovani, L. infantum, L. chagasi, L. amazonensis, L. panamensis, T. brucei, T. congolense, T. equinum, T. equiperdum, T. evansi, T. gambiense, T. simiae, T. rhodesiense, T. vivax.* All of them can be perjudicial to livestock or human, and thus infection caused by these parasites can be treated with the compounds of the invention.

Another aspect of the invention refers to a method of treatment of a trypanosomatid disease related to the inactivation of the trypanothione reductase (TryR), preferably selected from Chagas disease, trypanosomiasis or sleeping sickness, and leishmaniasis, and more preferably for the treatment of leishmaniasis, which comprises administering to a subject the compounds of the invention, namely, compounds of formula **(I), (I-A)** and **(I-B),** as defined above, and their pharmaceutically acceptable salts, or solvates thereof.

Another aspect of the invention is a method of preparing the compounds of formula **(I-A),** comprising the following steps:
(a) nucleophilic aromatic substitution of 4-bromo-2-fluorobenzonitrile with alcohols to give intermediates of formula **(II)** 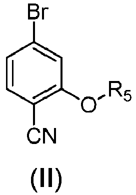 wherein R₅ is selected from substituted or unsubstituted (C₁-C₄)alkyl, wherein the substituent is an heterocyclylalkyl, NHCONH₂, and NHCONHR, wherein R is selected from (C₁-C₄)alkyl; preferably, R₅ is selected from CH₃, 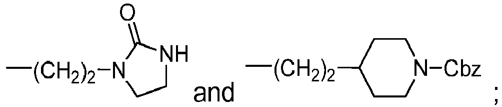
(b) azidation of intermediates of formula **(II)** to give intermediates of formula **(III)** 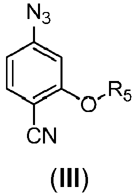 wherein R₅ is as defined above;
(c) "Click" 1,4-regioselective synthesis of compounds of formula **(IV)** by the Cu-catalyzed Huisgen 1,3-dipolar cycloaddition of azide intermediates **(III)** with terminal alkynes 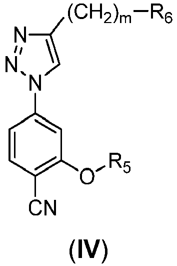 wherein m is a whole number in the range of from 1 to 5; more preferred from 2 and 4, and even more preferably m is 3 or 4;
   R₆ is selected from NHPG, in which PG is an amino-protecting group, and a protected guanidinium group; and
   R₅ is as defined above;
(d) treatment of triazole intermediates of formula **(IV)** with aqueous ammonium sulfide to give intermediates of formula **(V)** 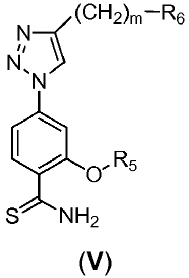 wherein m, R₅ and R₆ are as defined above;
(e) Hantsch thiazole synthesis of thioamides of formula **(V)** with various α-bromo ketones yielded the protected compounds of formula **(VI)** 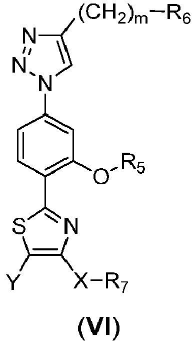
   wherein X is absent or is selected from O, -(CH₂)ₙ-, wherein n is a whole number in the range of from 1 to 3, -(CH=CH)-, and -(CH=CH)-CH₂-;
   Y is H, substituted or unsubstituted (C₁-C₁₀)alkyl, substituted or unsubstituted aryl, and substituted or unsubstituted heteroaryl;
   R₇ is selected from substituted or unsubstituted (C₁-C₁₀)alkyl, substituted or unsubstituted aryl, and substituted or unsubstituted heteroaryl; and
   m, R₅ and R₆ are as defined above;
(f) deprotection by TFA treatment or catalytic hydrogenation of compounds of formula **(VI)** furnished the final deprotected compounds of formula **(I-A).**

Another aspect of the invention is a method of preparing the compounds of formula **(I-B),** comprising the following steps:
(a) regioselective alkylation of the 1,2,3-triazole precursors of formula **(VI)** with alkyl trifluoromethane sulfonates yielded the protected compounds of formula **(VII)** 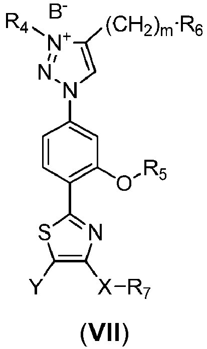
   wherein R₄ is selected from substituted or unsubstituted (C₁-C₅)alkyl and substituted or unsubstituted aryl;
   B- is a counter-ion selected from trifluoromethanesulfonic acid anion, benzenesulfonic acid anion, trifluoroacetic acid, or sulphuric acid anion;
   R₅ is selected from substituted or unsubstituted (C₁-C₄)alkyl, wherein the substituent is an heterocyclylalkyl, NHCONH₂, and NHCONHR, wherein R is selected from (C₁-C₄)alkyl; preferably, R₅ is selected from CH₃, 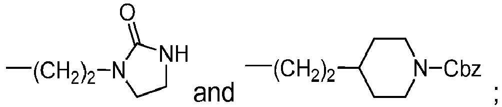
   m is a whole number in the range of from 1 to 5 preferably m is 3 or 4;
   R₆ is selected from NHPG, in which PG is an amino-protecting group, and a protected guanidinium group;
   X is absent or is selected from O, -(CH₂)ₙ-, wherein n is a whole number in the range of from 1 to 3, -(CH=CH)-, and -(CH=CH)-CH₂-;
   Y is selected from H, substituted or unsubstituted (C₁-C₁₀)alkyl, substituted or unsubstituted aryl, and substituted or unsubstituted heteroaryl; and
   R₇ is selected from substituted or unsubstituted (C₁-C₁₀)alkyl, substituted or unsubstituted aryl, and substituted or unsubstituted heteroaryl;
(b) treatment with TFA or catalytic hydrogenation of compounds of formula **(VII)** to yield the desired unprotected triazolium dicationic salts of formula **(I-B).**

A further aspect of the invention is a compound of formula **(VI),** a pharmaceutically acceptable salt, or solvate thereof, 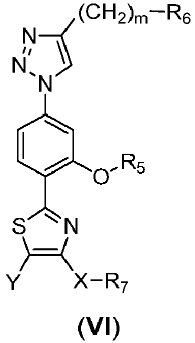 wherein:
X is absent or is selected from O, -(CH₂)ₙ-, wherein n is a whole number in the range of from 1 to 3, -(CH=CH)-, and -(CH=CH)-CH₂-;
Y is selected from H, substituted or unsubstituted (C₁-C₁₀)alkyl, substituted or unsubstituted aryl, and substituted or unsubstituted heteroaryl;
m is a whole number in the range of from 1 to 5
R₅ is selected from substituted or unsubstituted (C₁-C₄)alkyl, wherein the substituent is an heterocyclylalkyl, NHCONH₂, and NHCONHR, wherein R is selected from (C₁-C₄)alkyl;
R₆ is selected from NHPG, in which PG is an amino-protecting group, and a protected guanidinium group; and
R₇ is selected from substituted or unsubstituted (C₁-C₁₀)alkyl, substituted or unsubstituted aryl, and substituted or unsubstituted heteroaryl.

In a preferred embodiment, m is selected from 3 and 4, and even more preferred m is 4.

Further preferred is a compound of formula **(VI)** wherein R₆ is NHPG, in which PG is an amino-protecting group.

Further preferred is a compound of formula **(VI)** wherein X is -(CH=CH)-, and -(CH=CH)-CH₂-.

Further preferred is a compound of formula **(VI)** wherein Y is H or substituted or unsubstituted aryl; even more preferred is H or phenyl.

Further preferred is a compound of formula **(VI)** wherein R₇ is selected from substituted or unsubstituted aryl, and substituted or unsubstituted heteroaryl, even more preferably is selected from de group consisting in phenyl, naphthyl, biphenyl, benzofuranyl, quinolinyl, and dibenzofuranyl.

Further preferred is a compound of formula **(VI)** wherein R₅ is selected from CH₃ and 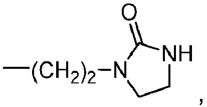 and even more preferred R₅ is

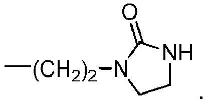

Another aspect of the invention is a compound of formula **(VII),** a pharmaceutically acceptable salt, or solvate thereof, 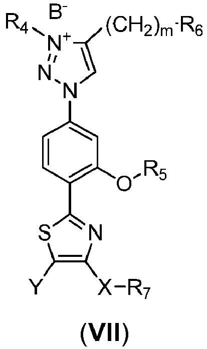 wherein:
R₄ is selected from substituted or unsubstituted (C₁-C₅)alkyl and substituted or unsubstituted aryl;
R₅ is selected from substituted or unsubstituted (C₁-C₄)alkyl, wherein the substituent is an heterocyclylalkyl, NHCONH₂, and NHCONHR, wherein R is selected from (C₁-C₄)alkyl;
m is a whole number in the range of from 1 to 5;
R₆ is selected from NHPG, in which PG is an amino-protecting group, and a protected guanidinium group;
X is absent or is selected from O, -(CH₂)ₙ-, wherein n is a whole number in the range of from 1 to 3, -(CH=CH)-, and -(CH=CH)-CH₂-; and
Y is selected from H, substituted or unsubstituted (C₁-C₁₀)alkyl, substituted or unsubstituted aryl, and substituted or unsubstituted heteroaryl;
R₇ is selected from substituted or unsubstituted (C₁-C₁₀)alkyl, substituted or unsubstituted aryl, and substituted or unsubstituted heteroaryl; and
B- is a counter-ion selected from trifluoromethanesulfonic acid anion, benzenesulfonic acid anion, trifluoroacetic acid, or sulphuric acid anion.

In a preferred embodiment, m is selected from 3 and 4, and even more preferred m is 4.

Further preferred is a compound of formula **(VII)** wherein R₆ is NHPG, in which PG is an amino-protecting group.

Further preferred is a compound of formula **(VII)** wherein X is -(CH=CH)-.

Further preferred is a compound of formula **(VII)** wherein Y is H or substituted or unsubstituted aryl; even more preferred is H or phenyl.

Further preferred is a compound of formula **(VII)** wherein R₇ is selected from substituted or unsubstituted aryl, and substituted or unsubstituted heteroaryl, even more preferably is selected from de group consisting in phenyl, naphthyl, biphenyl, benzofuranyl, quinolinyl, and dibenzofuranyl.
Further preferred is a compound of formula **(VII)** wherein R₅ is

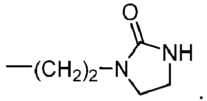

Further preferred is a compound of formula **(VII)** wherein R₄ is a substituted or unsubstituted (C₁-C₅)alkyl selected from a group consisting in methyl, ethyl, propyl, and butyl; and even more preferred, R₄ is methyl.

The term "protecting group" or "PG" refers to a substituent that is employed to block or protect a particular functionality. Other functional groups on the compound may remain reactive. In this particular case, the "amino-protecting group" is a substituent attached to an amino group that blocks or protects the amino functionality in the compound. Suitable aminoprotecting groups include, but are not limited to, acetyl, trifluoroacetyl, tert-butoxycarbonyl (BOC), benzyloxycarbonyl (CBz) and 9-fluorenylmethylenoxycarbonyl (Fmoc).

The term "comprising" (and related terms such as "comprise" or "comprises" or "having" or "including") includes those embodiments, for example, an embodiment of any composition of matter, composition, method, or process, or the like, that "consist of or "consist essentially of" the described features.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**FIG. 1****.** Design of model compound A.

### EXAMPLES

### Example 1. Synthesis of compounds of formula (I-A)

A detailed description of the synthesis compounds of formula **(I-A)** is depicted in **Scheme 1.** The synthetic route involves a nucleophilic aromatic substitution of 4-bromo-2-fluorobenzonitrile with alcohols to give intermediates **1** followed by azidation. "Click" 1,4-regioselective synthesis of 1,2,3-triazole **3** by the Cu-catalyzed Huisgen 1,3-dipolar cycloaddition of azide intermediates **2** with terminal alkynes was next carried out. Subsequent treatment of triazole intermediates **3** with aqueous ammonium sulfide followed by Hantsch thiazole synthesis of thioamides **4** with various α-bromo ketones yielded the protected compounds **7.** Further deprotection by TFA treatment or catalytic hydrogenation furnished the final deprotected compounds **8** in good yields isolated as monocationic trifluoroacetate salts. In **Scheme 1** the variety of R₁, R₂ and R₃ substituents that mimic the Lys-2, Gln-5 and Ile-9 residues of the prototype peptides are indicated. The synthesis of non-commercially available α-bromo ketones is depicted in **Scheme 2.**

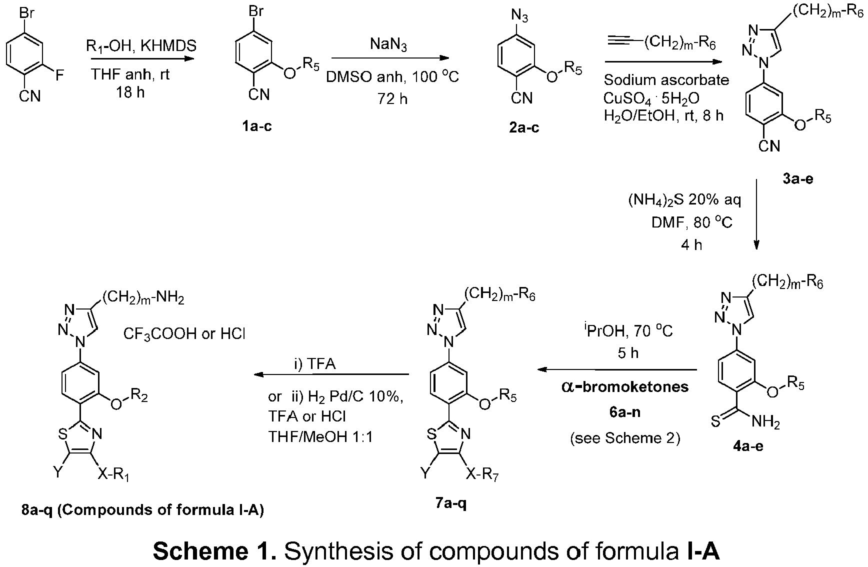

### A) Aromatic nucleophilic substitution (SNAr)

*General procedure.* 4-bromo-2-fluorobenzonitrile (3.25 mmol) was reacted with the corresponding alcohol (3.5 mmol) in the presence of a 0.5 M solution of KHMDS (3.75 mmol) in toluene (40mL). The resulting mixture was stirred at room temperature until complete dissapareance of starting material and the solvent was removed under reduced pressure. The residue was dissolved in dichloromethane (50 mL) and was successively washed with HCl aq (3 x 10 mL) and brine (3 x 10 mL). The organic phase was dried (MgSO₄), filtered and evaporated to dryness. The final residue was purified by flash column chromatography in silica gel (CH₂Cl₂/MeOH, 9.75:0.25 for **1a,** Hexane/AcOEt, 8:2 for **1b)** to give intermediate **1a,b** as a yellow solid and colorless oil in 71% and 94% yields, respectively.

### 4-Bromo-2-(2-(2-oxoimidazolidin-1-yl)ethoxy)benzonitrile (1a)

**M.p:** 165-171 °C; **IR (KBr), v (cm⁻¹):** 3250 (NH-st), 3097 (Cₛₚ-H st), 2227 (C=N st), 1685 (C=O st); **¹H-NMR (DMSO-d₆, 400 MHz) δ (ppm):** 7.69 (d, *J* = 8.2 Hz, 1H, Ar), 7.56 (d, *J* = 1.8 Hz, 1H, Ar), 7.32 (dd, *J* = 8.2, 1.7 Hz, 1H, Ar), 6.42 (bs, 1H, NH), 4.26 (t, *J* = 5.2 Hz, 2H, OCH₂), 3.51 (m, 2H, CH₂CH₂NHCON), 3.44 (t, *J* = 5.2 Hz, 2H, OCH₂CH₂), 3.23 (t, *J* = 7.8 Hz, 2H, CH₂CH₂NHCON); **¹³C-NMR (DMSO-d₆, 75 MHz) δ (ppm):** 162.1 (NHCON), 160.5 (OC_{Ar}), 134.9 (CH_{Ar}), 128.6 (C_{Ar}), 124.4 (CH_{Ar}), 116.5 (CH_{Ar}), 115.7 (CN), 100.0 (C_{Ar}), 68.7 (OCH₂), 45.9 (CH₂CH₂NHCON), 42.4 (OCH₂CH₂), 37.5 (CH₂CH₂NHCON); **MS (ESI, positive mode) m/z:** 332.0 [M+Na]⁺, 310.0 [M+H]⁺, with a Br isotopic pattern

### Benzyl 4-(2-(5-bromo-2-cyanophenoxy)ethyl)piperidine-1-carboxylate (1b)

**¹H-NMR (CDCl₃, 400 MHz) δ (ppm):** 7.40 (d, *J* = 8.2 Hz, 1H, Ar), 7.38 - 7.22 (m, 5H, Ar), 7.15 (dd, *J* = 8.2, 1.7 Hz, 1H, Ar), 7.11 (d, *J* = 1.7 Hz, 1H, Ar), 5.12 (s, 2H, NCOOCH₂), 4.21 (m, 2H, H'HCN(Cbz)CHH'), 4.10 (t, *J* = 5.2 Hz, 2H, OCH₂), 2.81 (m, 2H, H'HCN(Cbz)CHH'), 1.88 - 1.64 (m, 5H, CH, CH(CHH')₂), OCH₂CH₂), 1.21 (m, 2H, CH(CHH')₂); **¹³C-NMR (CDCl₃, 100 MHz) δ (ppm):** 161.0 (NCOO), 155.3 (OC_{Ar}), 137.0 (C_{Ar}), 134.5 (CH_{Ar}), 128.9 (C_{Ar}), 128.6 (CH_{Ar}), 128.0 (CH_{Ar}), 127.9 (CH_{Ar}), 124.3 (CH_{Ar}), 116.1 (CH_{Ar}), 115.8 (CN), 101.2 (C_{Ar}), 67.1 (NCOOCH₂), 67.0 (OCH₂), 44.1 (H'HCN(Cbz)CHH'), 35.3 (OCH₂CH₂), 32.8 (CH), 32.0 (CH(CHH')₂); **HRMS (ES, positive mode) m/z:** calculated for C₂₂H₂₃BrN₂O₃ 442.0892; Found 442.0900 (1.92 ppm)

### B) Synthesis of azide intermediates

*General procedure.* The corresponding bromide **1a,b** or commercially available 4-bromo-2-methoxybenzonitrile **1c** (1.61 mmol) was treated with NaN₃ (24.2 mmol) in the presence of molecular sieves (4 Å) in anhydride DMSO (35 mL) at room temperature under argon atmosphere. Then, the reaction was stirred at 100 °C for 72 h. The disappearance of starting material was monitored by analytical HPLC (2% to 95% acetonitrile gradient). The reaction was allowed to cool to room temperature, water was added (60 mL) and the aqueous phase was extracted with dichloromethane (3 x 50 mL). The combined organic layers were dried (MgSO₄), filtered, evaporated to dryness and lyophilized. The final residue was purified by flash column chromatography in silica gel (CH₂Cl₂/MeOH, 9.7:0.3 for **1a,** Hex/AcOEt 8:2 for **1b** and CH₂Cl₂/MeOH, 10 : 0.1 for **1c**) to give intermediates **2a-c** as a yellow solid, colorless oil and solid in 49%, 85% and 74% yields, respectively.

### 4-Azido-2-(2-(2-oxoimidazolidin-1-yl)ethoxy)benzonitrile (2a)

**M.p:** 158-161 °C; **IR (KBr), v (cm⁻¹):** 3230 (NH-st), 3090 (Cₛₚ-H st), 2223 (C=N st), 2125 (N=N=N st), 1702 (C=O st); **¹H-NMR (CDCl₃, 300 MHz) δ (ppm):** 7.53 (d, *J* = 8.3 Hz, 1H, Ar), 6.70 (dd, *J* = 8.3, 2.0 Hz, 1H, Ar), 6.53 (d, *J =* 1.8 Hz, 1H, Ar), 4.71 (bs, 1H, NH), 4.20 (t, *J* = 4.7 Hz, 2H, OCH₂), 3.79 (m, 2H, CH₂CH₂NHCON), 3.64 (t, *J* = 4.9 Hz, 2H, OCH₂CH₂), 3.44 (t, *J* = 8.1 Hz, 2H, CH₂CH₂NHCON); **¹³C-NMR (CDCl₃, 75 MHz) δ (ppm):** 162.8 (NHCON), 161.8 (OC_{Ar}), 146.8 (C_{Ar}), 134.9 (CH_{Ar}), 116.2 (CN), 111.8 (CH_{Ar}), 103.2 (CH_{Ar}), 98.3 (C_{Ar}), 69.4 (OCH₂), 47.8 (CH₂CH₂NHCON), 43.2 (OCH₂CH₂), 38.6 (CH₂CH₂NHCON); **MS (ESI, positive mode) m/z:** 567.2 [2M+Na]⁺, 295.0 [M+Na]⁺, 273.0 [M+H]⁺

### Benzyl 4-(2-(5-azido-2-cyanophenoxy)ethyl)piperidine-1-carboxylate (2b)

**¹H-NMR (CDCl₃, 400 MHz) δ (ppm): ¹H-NMR (CDCl₃, 400 MHz) δ (ppm):** 7.44 (d, *J* = 8.3 Hz, 1H, Ar), 7.35 - 7.16 (m, 5H, Ar), 6.61 (dd, *J* = 8.3, 2.0 Hz, 1H, Ar), 6.44 (d, *J* = 2.1 Hz, 1H, Ar), 5.05 (s, 2H, NCOOCH₂), 4.08 (m, 2H, H'HCN(Cbz)CHH'), 4.02 (t, *J* = 5.2 Hz, 2H, OCH₂), 2.74 (m, 2H, H'HCN(Cbz)CHH'), 1.78 - 1.56 (m, 5H, CH, CH(CHH')₂), OCH₂CH₂), 1.16 (m, 2H, CH(CHH')₂); **¹³C-NMR (CDCl₃, 75 MHz) δ (ppm):** 162.0 (NCOO), 159.3 (N₃C_{Ar}), 155.3 (OC_{Ar}), 137.0 (C_{Ar}), 135.0 (CH_{Ar}), 128.9 (CH_{Ar}), 128.6 (CH_{Ar}), 128.0 (CH_{Ar}), 127.9 (CH_{Ar}), 111.4 (CN), 103.2 (CH_{Ar}), 98.5 (C_{Ar}), 67.1 (NCOOCH₂), 66.8 (OCH₂), 44.1 (H'HCN(Cbz)CHH'), 35.3 (OCH₂CH₂), 32.8 (CH), 32.0 (CH(CHH')₂); **HRMS (ES, positive mode) m/z:** calculated for C₂₂H₂₃N₅O₃ 405.1801; Found 405.1814 (3.22 ppm)

### 4-Azido-2-methoxybenzonitrile (2c)

**¹H-NMR (CDCl₃, 400 MHz) δ (ppm):** 7.53 (d, *J* = 8.3 Hz, 1H, Ar), 6.69 (dd, *J* = 8.3, 2.0 Hz, 1H, Ar), 6.54 (d, *J* = 2.0 Hz, 1H, Ar), 3.92 (s, 3H, OCH₃); **¹³C-NMR (CDCl₃, 75 MHz) δ (ppm):** 162.7 (OC_{Ar}), 146.7 (C_{Ar}), 135.1 (CH_{Ar}), 116.2 (CN), 111.4 (CH_{Ar}), 102.6 (CH_{Ar}), 98.3 (C_{Ar}), 56.4 (OCH₃); **MS (ESI, positive mode) m/z:** 197.0 [M+Na]⁺, 175.0 [M+H]⁺

### C) Synthesis of 1,2,3-triazole derivatives by 1,3-dipolar cycloaddition between azides and terminal alkynes

*General procedure.* The azide intermediate **2a-c** (0.92 mmol) was treated with the corresponding terminal alkyne (1.19 mmol) and CuSO₄·5H₂O (0.09 mmol) in ethanol (30 mL). Water (30 mL) and sodium ascorbate (0.40 mmol) was added and the reaction mixture was stirred at room temperature for 20 h in darkness conditions. Then, the ethanol was evaporated to dryness, the residue was dissolved in dichloromethane (50 mL) and washed with water (3 x 50 mL). The combined organic layers were dried (MgSO₄), filtered and evaporated to dryness. The final residue was purified by flash column chromatography in silica gel (CH₂Cl₂/MeOH, 9.8:0.2 and 10 : 0.1 for a,c and **b** compounds, respectively) to give 1,2,3-triazole intermediates **3a-e** as a colorless oil in 48-88% yields.

### tert-Butyl (4-(1-(4-cyano-3-(2-(2-oxoimidazolidin-1-yl)ethoxy)phenyl)-1H-1,2,3-triazol-4-yl)butyl)carbamate (3a)

**M.p:** 139-142 °C; **IR (KBr), v (cm⁻¹):** 3396 (NH-st), 2226 (C=N st), 1691 (C=O st); **¹H-NMR (CDCl₃, 300 MHz) δ (ppm):** 7.93 (s, 1H, Ar), 7.68 (dd, *J* = 8.4, 2.4 Hz, 1H, Ar), 7.57 (s, 1H, Ar), 7.39 (d, *J* = 7.7 Hz, 1H, Ar), 4.78 (bs, 1H, NH), 4.69 (bs, 1H, NH), 4.33 (m, 2H, OCH₂), 3.75 (t, *J* = 7.1 Hz, 2H, CH₂CH₂NHCON), 3.65 (m, 2H, OCH₂CH₂), 3.45 (t, *J* = 7.4 Hz, 2H, CH₂CH₂NHCON), 3.15 (q, *J* = 6.0 Hz, 2H, CH₂NHBoc), 2.82 (t, *J* = 6.6 Hz, 2H, TrizCH₂), 1.76 (quin, *J* = 7.7 Hz, 2H, TrizCH₂CH₂), 1.57 (quin, *J* = 7.5 Hz, 2H, CH₂CH₂NHBoc), 1.42 (s, 9H, CH₃); **¹³C-NMR (CDCl₃, 75 MHz) δ (ppm):** 162.8 (NHCON), 161.5 (OC_{Ar}), 156.2 (NHCOO), 149.3 (C_{Ar}), 141.6 (C_{Ar}), 135.1 (CH_{Ar}), 119.1 (CH_{Ar}), 115.7 (CN), 112.1 (CH_{Ar}), 104.1 (CH_{Ar}), 101.6 (C_{Ar}), 79.3 (OC(CH₃)₃), 69.2 (OCH₂), 47.4 (CH₂CH₂NHCON), 43.0 (OCH₂CH₂), 40.3 (CH₂NHBoc), 38.6 (CH₂CH₂NHCON), 29.5 (CH₂CH₂NHBoc), 28.5 (CH₃), 26.3 (TrizCH₂CH₂), 25.2 (TrizCH₂); **MS (ESI, positive mode) m/z:** 492.3 [M+Na]⁺, 470.3 [M+H]⁺

### Benzyl (4-(1-(4-cyano-3-((1-(2-oxoimidazolidin-1-yl)ethoxy)phenyl)-1H-1,2,3-triazol-4-yl)butyl)carbamate (3b)

**¹H-NMR (CDCl₃, 400 MHz) δ (ppm):** 7.86 (s, 1H, Ar), 7.61 (d, *J* = 8.3 Hz, 1H, Ar), 7.49 (s, 1H, Ar), 7.34 (d, *J* = 7.1 Hz, 1H, Ar), 7.32 - 7.17 (m, 5H, Ar), 5.05 (bs, 1H, NH), 5.02 (s, 2H, OCH₂Ph), 4.68 (bs, 1H, NH), 4.26 (t, *J* = 5.2 Hz, 2H, OCH₂), 3.66 (t, *J* = 7.9 Hz, 2H, CH₂CH₂NHCON), 3.57 (t, *J* = 5.2 Hz, 2H, OCH₂CH₂), 3.33 (t, *J* = 8.3 Hz, 2H, CH₂CH₂NHCON), 3.18 (q, *J* = 6.7 Hz, 2H, CH₂NHCbz), 2.76 (t, *J* = 7.3 Hz, 2H, TrizCH₂), 1.71 (quin, *J* = 7.4 Hz, 2H, TrizCH₂CH₂), 1.52 (quin, *J* = 7.2 Hz, 2H, CH₂CH₂NHCbz); **¹³C-NMR (CDCl₃, 75 MHz) δ (ppm):** 162.8 (NHCON), 161.5 (OCAr), 156.6 (NHCOO), 149.2 (C_{Ar}), 141.5 (C_{Ar}), 136.6 (CH_{Ar}), 135.1 (CH_{Ar}), 128.6 (CH_{Ar}), 128.2 (CH_{Ar}), 119.1 (CH_{Ar}), 115.7 (CN), 112.1 (CH_{Ar}), 104.1 (CH_{Ar}), 101.5 (C_{Ar}), 69.0 (OCH₂), 66.7 (OCH₂Ph), 47.3 (CH₂CH₂NHCON), 42.8 (OCH₂CH₂), 40.8 (CH₂NHCbz), 38.5 (CH₂CH₂NHCON), 29.4 (CH₂CH₂NHCbz), 26.1 (TrizCH₂CH₂), 25.1 (TrizCH₂); **HRMS (ES, positive mode) m/z:** calculated for C₂₆H₂₉N₇O₄ 503.2281; Found 503.2279 (-0.33 ppm)

### Benzyl 4-(1-(5-(4-(4-(((benzyloxy)carbonyl)amino)butyl)-1H-1,2,3-triazol-1-yl)-2-cyanophenoxy)ethyl)piperidine-1-carboxylate (3c)

**¹H-NMR (CDCl₃, 400 MHz) δ (ppm):** 7.84 (s, 1H, Ar), 7.69 (d, *J* = 8.4 Hz, 1H, Ar), 7.58 (d, *J* = 1.9 Hz, 1H, Ar), 7.46 - 7.22 (m, 10H, Ar), 7.24 (dd, *J* = 8.3, 2.0 Hz, 1H, Ar), 5.15 (s, 2H, NCOOCH₂), 5.11 (s, 2H, NCOOCH₂), 4.90 (bs, 1H, NH), 4.23 (m, 2H, H'HCN(Cbz)CHH'), 4.25 (t, *J* = 6.0 Hz, 2H, OCH₂), 3.27 (q, *J* = 6.7 Hz, 2H, CH₂NHCbz), 2.92 - 2.75 (m, 4H, TrizCH₂, H'HCN(Cbz)CHH'), 1.95 - 1.73 (m, 7H, TrizCH₂CH₂, CH, CH(CHH')₂), OCH₂CH₂), 1.63 (m, 2H, CH₂CH₂NHCbz), 1.24 (m, 2H, CH(CHH')₂); **¹³C-NMR (CDCl₃, 75 MHz) δ (ppm):** 161.8 (OC_{Ar}), 156.6 (NCOO), 155.6 (NCOO), 149.2 (C_{Ar}), 141.4 (C_{Ar}), 137.0 (C_{Ar}), 136.6 (C_{Ar}), 135.1 (CH_{Ar}), 128.6 (CH_{Ar}), 128.6 (CH_{Ar}), 128.2 (CH_{Ar}), 128.1 (CH_{Ar}), 128.0 (CH_{Ar}), 127.9 (CH_{Ar}), 119.0 (CH_{Ar}), 115.5 (CN), 111.4 (CH_{Ar}), 104.3 (CH_{Ar}), 101.9 (C_{Ar}), 67.3 (NCOOCH₂), 67.1 (NCOOCH₂), 66.7 (OCH₂), 44.2 (H'HCN(Cbz)CHH'), 40.9 (CH₂NHCbz), 35.3 (OCH₂CH₂), 32.8 (CH), 32.1 (CH(CHH')₂), 29.5 (CH₂CH₂NHCbz), 26.3 (TrizCH₂CH₂), 25.1 (TrizCH₂); **HRMS (ES, positive mode) m/z:** calculated for C₃₆H₄₀N₆O₅ 636.3060; Found 636.3060 (-0.1 ppm)

### tert-Butyl (4-(1-(4-cyano-3-methoxyphenyl)-1H-1,2,3-triazol-4-yl)butyl)carbamate (3d)

**¹H-NMR (CDCl₃, 400 MHz) δ (ppm):** 7.85 (s, 1H, Ar), 7.68 (d, *J* = 8.3 Hz, 1H, Ar), 7.57 (d, *J* = 1.9 Hz, 1H, Ar), 7.28 (dd, *J* = 8.3, 1.9 Hz, 1H, Ar), 4.59 (bs, 1H, NH), 4.03 (s, 3H, OCH₃), 3.16 (q, *J* = 6.8 Hz, 2H, CH₂NHBoc), 2.82 (t, *J* = 7.5 Hz, 2H, TrizCH₂), 1.75 (quin, *J* = 7.5 Hz, 2H, TrizCH₂CH₂), 1.58 (quin, *J* = 7.5 Hz, 2H, CH₂CH₂NHBoc), 1.42 (s, 9H, CH₃); **¹³C-NMR (CDCl₃, 75 MHz) δ (ppm):** 162.5 (OC_{Ar}), 156.2 (NHCOO), 149.3 (C_{Ar}), 141.5 (C_{Ar}), 135.1 (CH_{Ar}), 119.1 (CH_{Ar}), 115.6 (CN), 111.5 (CH_{Ar}), 103.6 (CH_{Ar}), 101.7 (C_{Ar}), 79.3 (OC(CH₃)₃), 56.7 (OCH₃), 40.3 (CH₂NHBoc), 29.5 (CH₂CH₂NHBoc), 28.5 (CH₃), 26.4 (TrizCH₂CH₂), 25.2 (TrizCH₂); **HRMS (ES, positive mode) m/z:** calculated for C₁₉H₂₅N₅O₃ 371.1957; Found 371.1968 (2.81 ppm)

### Benzyl (3-(1-(4-cyano-3-((1-(2-oxoimidazolidin-1-yl)ethoxy)phenyl)-1H-1,2,3-triazol-4-yl)propyl)carbamate (3e)

**¹H-NMR (CDCl₃, 400 MHz) δ (ppm):** 8.01 (s, 1H, Ar), 7.61 (d, *J* = 8.3 Hz, 1H, Ar), 7.55 (s, 1H, Ar), 7.38 (d, *J* = 8.4 Hz, 1H, Ar), 7.32 - 7.18 (m, 5H, Ar), 5.48 (bs, 1H, NH), 5.02 (s, 2H, OCH₂Ph), 4.70 (bs, 1H, NH), 4.26 (t, *J* = 5.7 Hz, 2H, OCH₂), 3.63 (t, *J* = 8.0 Hz, 2H, CH₂CH₂NHCON), 3.55 (t, *J* = 5.7 Hz, 2H, OCH₂CH₂), 3.25 (t, *J* = 8.0 Hz, 2H, CH₂CH₂NHCON), 3.16 (q, *J* = 6.6 Hz, 2H, CH₂NHCbz), 2.76 (t, *J* = 7.3 Hz, 2H, TrizCH₂), 1.82 (m, 2H, TrizCH₂CH₂); **¹³C-NMR (CDCl₃, 75 MHz) δ (ppm):** 162.9 (NHCON), 161.4 (OC_{Ar}), 156.7 (NHCOO), 148.4 (C_{Ar}), 141.5 (C_{Ar}), 136.5 (CH_{Ar}), 135.1 (CH_{Ar}), 128.7 (CH_{Ar}), 128.3 (CH_{Ar}), 119.7 (CH_{Ar}), 115.7 (CN), 112.0 (CH_{Ar}), 104.1 (CH_{Ar}), 101.4 (C_{Ar}), 68.6 (OCH₂), 66.9 (OCH₂Ph), 47.2 (CH₂CH₂NHCON), 42.6 (OCH₂CH₂), 39.8 (CH₂NHCbz), 38.5 (CH₂CH₂NHCON), 29.0 (TrizCH₂CH₂), 22.3 (TrizCH₂); **HRMS (ES, positive mode) m/z:** calculated for C₂₅H₂₇N₇O₄ 489.2124; Found 489.2126 (0.21 ppm)

### D) Synthesis of thioamides by thionation

*General procedure.* A solution of the corresponding benzonitriles **3a-e** (0.19 mmol) in DMF (10 mL) was treated with a 20% solution of (NH₄)₂S (13.8 mmol) in water. A change of yellow to dark blue color was instantly observed. The reaction mixture was heated at 80 °C for 3 h and then was allowed to cool to room temperature. Dichloromethane (50 mL) was added and the resulting mixture was washed with HCl 0.1 N (3 x 50 mL). The organic layers were dried (MgSO₄), filtered and evaporated to dryness. The residue (if neccesary) was purified by flash column chromatography in silica gel (CH₂Cl₂/MeOH, 9.8:0.2) to yield the thioamide compounds **4a-e** as yellow solids in quantitative yields.

### tert-Butyl (4-(1-(4-carbamothioyl-3-(2-(2-oxoimidazolidin-1-yl)ethoxy)phenyl)-1H-1,2,3-triazol-4-yl)butyl)carbamate (4a)

**IR (KBr), v (cm⁻¹):** 3351 (NH st), 1682 (C=O st); **¹H-NMR (DMSO-d₆, 400 MHz) δ (ppm):** 10.10 (bs, 1H, CSNH₂), 9.43 (bs, 1H, CSNH₂), 8.68 (s, 1H, Ar), 7.88 (dd, *J* = 8.4, 1.1 Hz, 1H, Ar), 7.56 (d, *J* = 1.6 Hz, 1H, Ar), 7.51 (dd, *J* = 8.4, 1.7 Hz, 1H, Ar), 6.82 (bs, 1H, NH), 6.38 (bs, 1H, NH), 4.25 (t, *J* = 5.2 Hz, 2H, OCH₂), 3.55 - 3.45 (m, 4H, CH₂CH₂NHCON, OCH₂CH₂), 3.22 (t, *J* = 7.8 Hz, 2H, CH₂CH₂NHCON), 2.95 (m, 2H, CH₂NHBoc), 2.71 (t, *J* = 7.5 Hz, 2H, TrizCH₂), 1.65 (m, 2H, TrizCH₂CH₂), 1.46 (m, 2H, CH₂CH₂NHBoc), 1.36 (s, 9H, CH₃); **¹³C-NMR (DMSO-d₆, 75 MHz) δ (ppm):** 197.5 (CSNH₂), 162.2 (NHCON), 155.6 (NHCOO), 154.3 (OC_{Ar}), 148.1 (C_{Ar}), 138.5 (C_{Ar}), 132.5 (CH_{Ar}), 129.8 (C_{Ar}), 120.3 (CH_{Ar}), 111.1 (CH_{Ar}), 103.9 (CH_{Ar}), 77.3 (OC(CH₃)₃), 67.7 (OCH₂), 45.6 (CH₂CH₂NHCON), 42.6 (OCH₂CH₂), 37.6 (CH₂CH₂NHCON), 29.0 (CH₂CH₂NHBoc), 28.3 (CH₃), 26.0 (TrizCH₂CH₂), 24.7 (TrizCH₂); **MS (ESI, positive mode) m/z:** 526.3 [M+Na]⁺ , 504.3 [M+H]⁺

### Benzyl (4-(1-(4-carbamothioyl-3-((1-(2-oxoimidazolidin-1-yl)ethoxy)phenyl)-1H-1,2,3-triazol-4-yl)butyl)carbamate (4b)

**¹H-NMR (CDCl₃, 400 MHz) δ (ppm):** 9.63 (bs, 1H, CSNH₂), 9.50 (bs, 1H, CSNH₂), 8.71 (d, *J* = 8.7 Hz, 1H, Ar), 7.78 (s, 1H, Ar), 7.45 (d, *J* = 2.0 Hz, 1H, Ar), 7.36 - 7.17 (m, 5H, Ar), 7.10 (dd, *J* = 8.7, 2.0 Hz, 1H, Ar), 5.96 (bs, 1H, NH), 5.02 (s, 2H, OCH₂Ph), 4.98 (bs, 1H, NH), 4.16 (t, *J* = 4.4 Hz, 2H, OCH₂), 3.64 (t, *J* = 4.4 Hz, 2H, OCH₂CH₂), 3.50 (dd, J = 9.7, 6.7 Hz, 2H, CH₂CH₂NHCON), 3.40 (t, *J* = 9.1, 6.2 Hz, 2H, CH₂CH₂NHCON), 3.17 (q, *J =* 6.7 Hz, 2H, CH₂NHCbz), 2.73 (t, *J* = 7.5 Hz, 2H, TrizCH₂), 1.70 (quin, *J* = 7.5 Hz, 2H, TrizCH₂CH₂), 1.53 (quin, *J* = 7.5 Hz, 2H, CH₂CH₂NHCbz); **¹³C-NMR (CDCl₃, 75 MHz) δ (ppm):** 196.5 (CSNH₂), 163.5 (NHCON), 156.3 (NHCOO), 149.1 (C_{Ar}), 140.3 (C_{Ar}), 138.4 (CH_{Ar}), 136.7 (CH_{Ar}), 128.7 (CH_{Ar}), 128.2 (CH_{Ar}), 125.1 (C_{Ar}), 119.1 (CH_{Ar}), 111.3 (CH_{Ar}), 104.0 (CH_{Ar}), 66.8 (OCH₂), 66.6 (OCH₂Ph), 45.4 (CH₂CH₂NHCON), 43.2 (OCH₂CH₂), 40.9 (CH₂NHCbz), 38.3 (CH₂CH₂NHCON), 29.8 (CH₂CH₂NHCbz), 26.4 (TrizCH₂CH₂), 25.3 (TrizCH₂); **MS (ESI, positive mode) m/z:** 538.3 [M+H]⁺

### Benzyl 4-(1-(5-(4-(4-(((benzyloxy)carbonyl)amino)butyl)-1H-1,2,3-triazol-1-yl)-2-carbamothioylphenoxy)ethoxy)piperidine-1-carboxylate (4c)

**¹H-NMR (CDCl₃, 400 MHz) δ (ppm):** 8.86 (bs, 1H, CSNH₂), 8.65 (d, *J* = 8.7 Hz, 1H, Ar), 8.11 (bs, 1H, CSNH₂), 7.76 (s, 1H, Ar), 7.53 (d, *J* = 2.0 Hz, 1H, Ar), 7.38 - 7.17 (m, 10H, Ar), 7.13 (dd, *J* = 8.7, 2.0 Hz, 1H, Ar), 5.05 (s, 2H, NCOOCH₂), 5.02 (s, 2H, NCOOCH₂), 4.84 (bs, 1H, NH), 4.20 (t, *J* = 6.6 Hz, 2H, OCH₂), 4.13 (m, 2H, H'HCN(Cbz)CHH'), 3.18 (q, *J* = 6.7 Hz, 2H, CH₂NHCbz), 2.77 - 2.65 (m, 4H, TrizCH₂, H'HCN(Cbz)CHH'), 1.82 - 1.61 (m, 7H, TrizCH₂CH₂, CH, CH(CHH')₂), OCH₂CH₂), 1.55 (m, 2H, CH₂CH₂NHCbz), 1.25 - 1.02 (m, 2H, CH(CHH')₂); **¹³C-NMR (CDCl₃, 75 MHz) δ (ppm):** 197.9 (CSNH₂), 156.5 (NCOO), 155.3 (NCOO), 149.1 (C_{Ar}), 140.5 (C_{Ar}), 137.9 (CH_{Ar}), 136.9 (C_{Ar}), 136.7 (C_{Ar}), 128.6 (CH_{Ar}), 128.6 (CH_{Ar}), 128.2 (CH_{Ar}), 128.2 (CH_{Ar}), 128.1 (CH_{Ar}), 128.0 (CH_{Ar}), 124.9 (C_{Ar}), 119.0 (CH_{Ar}), 111.4 (CH_{Ar}), 104.5 (CH_{Ar}), 67.8 (OCH₂Ph), 67.2 (OCH₂Ph), 66.7 (OCH₂), 44.1 (H'HCN(Cbz)CHH'), 40.8 (CH₂NHCbz), 35.6 (OCH₂CH₂), 33.2 (CH), 32.1 (CH(CHH')₂), 29.6 (CH₂CH₂NHCbz), 26.4 (TrizCH₂CH₂), 25.2 (TrizCH₂); **HRMS (ES, positive mode) m/z:** calculated for C₃₆H₄₂N₆O₅S 670.2937; Found 670.2931 (-0.92 ppm)

### tert-Butyl (3-(1-(4-carbamothioyl-3-methoxyphenyl)-1H-1,2,3-triazol-4-yl)propyl)carbamate (4d)

**¹H-NMR (CDCl₃, 400 MHz) δ (ppm):** 8.99 (bs, 1H, CSNH₂), 8.76 (d, *J* = 8.7 Hz, 1H, Ar), 8.19 (bs, 1H, CSNH₂), 7.86 (s, 1H, Ar), 7.62 (d, *J* = 2.0 Hz, 1H, Ar), 7.23 (dd, *J* = 8.7, 2.0 Hz, 1H, Ar), 4.62 (bs, 1H, NH), 4.06 (s, 3H, OCH₃), 3.17 (q, *J* = 6.7 Hz, 2H, CH₂NHBoc), 2.82 (t, *J* = 7.5 Hz, 2H, TrizCH₂), 1.77 (quin, *J* = 7.5 Hz, 2H, TrizCH₂CH₂), 1.59 (quin, *J* = 7.5 Hz, 2H, CH₂CH₂NHBoc), 1.43 (s, 9H, CH₃); **¹³C-NMR (CDCl₃, 75 MHz) δ (ppm):** 197.7 (CSNH₂), 157.3 (OC_{Ar}), 156.2 (NHCOO), 149.2 (C_{Ar}), 138.1 (CH_{Ar}), 124.5 (C_{Ar}), 119.1 (CH_{Ar}), 111.4 (CH_{Ar}), 103.7 (CH_{Ar}), 79.3 (OC(CH₃)₃), 56.8 (OCH₃), 40.3 (CH₂NHBoc), 29.6 (CH₂CH₂NHBoc), 28.5 (CH₃), 26.5 (TrizCH₂CH₂), 25.2 (TrizCH₂); **HRMS (ES, positive mode) m/z:** calculated for C₁₉H₂₇N₅O₃S 405.1835; Found 405.1839 (1.1 ppm)

### Benzyl (3-(1-(4-carbamothioyl-3-((1-(2-oxoimidazolidin-1 - yl)ethoxy)phenyl)-1H-1,2,3-triazol-4-yl)propyl)carbamate (4e)

**¹H-NMR (CDCl₃, 400 MHz) δ (ppm):** 9.69 (bs, 1H, CSNH₂), 9.49 (bs, 1H, CSNH₂), 8.69 (d, *J* = 8.7 Hz, 1H, Ar), 7.84 (s, 1H, Ar), 7.42 (s, 1H, Ar), 7.38 - 7.13 (m, 5H, Ar), 7.09 (dd, *J* = 8.7, 2.0 Hz, 1H, Ar), 6.03 (bs, 1H, NHCON), 5.18 (t, *J* = 6.2 Hz, 1H, NHCbz), 5.02 (s, 2H, OCH₂Ph), 4.13 (t, *J* = 4.3 Hz, 2H, OCH₂), 3.63 (t, *J* = 4.5 Hz, 2H, OCH₂CH₂), 3.49 (dd, *J* = 9.4, 6.4 Hz, 2H, CH₂CH₂NHCON), 3.38 (dd, *J* = 9.4, 6.5 Hz, 2H, CH₂CH₂NHCON), 3.19 (q, *J* = 6.7 Hz, 2H, CH₂NHCbz), 2.74 (t, *J* = 7.4 Hz, 2H, TrizCH₂), 1.85 (m, 2H, TrizCH₂CH₂); **¹³C-NMR (CDCl₃, 75 MHz) δ (ppm):** 196.9 (CSNH₂), 163.3 (NHCON), 156.8 (NHCOO), 156.2 (OC_{Ar}), 148.4 (C_{Ar}), 140.4 (C_{Ar}), 138.1 (CH_{Ar}), 137.0 (CH_{Ar}), 128.7 (CH_{Ar}), 128.2 (CH_{Ar}), 125.6 (C_{Ar}), 119.4 (CH_{Ar}), 111.6 (CH_{Ar}), 104.3 (CH_{Ar}), 66.9 (OCH₂), 66.8 (OCH₂Ph), 45.6 (CH₂CH₂NHCON), 43.3 (OCH₂CH₂), 40.5 (CH₂NHCbz), 38.4 (CH₂CH₂NHCON), 29.5 (CH₂CH₂NHCbz), 22.8 (TrizCH₂); **HRMS (ES, positive mode) m/z:** calculated for C₂₅H₂₉N₇O₄S 523.2002; Found 523.2007 (0.96 ppm)

### E) Synthesis of non-commercially available α-bromoketones (Scheme 2)

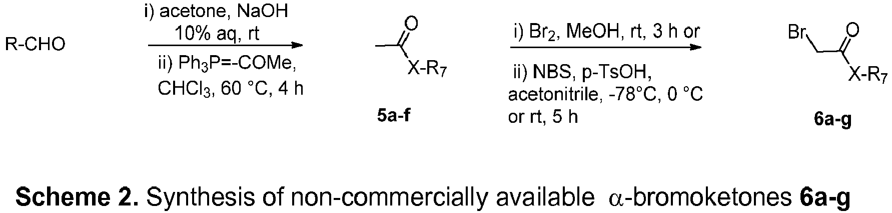

***i) General procedure of Claisen-Schmidt condensation.*** The commercially available arylcarboxaldehyde (6.4 mmol) was reacted with acetone (32 mmol) in methanol (15 mL) in the presence of NaOH 10% aq (32 mmol) as the base. The reaction mixture was stirred at room temperature for 2 h observing the appearance of a yellow precipitate. Then, the solid was filtered and recrystallize in EtOH to give intermediates **5a-c** as yellow solids in 64-91% yields.

### (E)-4-(Naphthalen-2-yl)but-3-en-2-one (5a)

**¹H-NMR (CDCl₃, 400 MHz) δ (ppm):** 7.96 (d, *J* = 1.8 Hz, 1H, Ar), 7.90 - 7.82 (m, 3H, Ar, CH=CHCO), 7.74 - 7.63 (m, 2H, Ar), 7.55 - 7.40 (m, 2H, Ar), 6.84 (d, *J* = 16.2 Hz, 1H, CH=CHCO), 2.43 (s, 3H, CH₃); **¹³C-NMR (CDCl₃, 75 MHz) δ (ppm):** 198.5 (CO), 143.7 (ArCH=CH), 134.5 (C_{Ar}), 133.4 (C_{Ar}), 132.1 (C_{Ar}), 130.5 (CH_{Ar}), 128.9 (CH_{Ar}), 128.7 (CH_{Ar}), 128.0 (CH_{Ar}), 127.5 (ArCH=CH), 127.4 (CH_{Ar}), 126.9 (CH_{Ar}), 123.6 (CH_{Ar}), 27.8 (CH₃); **MS (ESI, positive mode) m/z:** 219.0 [M+Na]⁺, 197.0 [M+H]⁺

### (E)-4-([1,1'-Biphenyl]-4-yl)but-3-en-2-one (5b)

**¹H-NMR (CDCl₃, 400 MHz) δ (ppm):** 7.67 - 7.60 (m, 6H, Ar), 7.56 (d, *J* = 16.3 Hz, 1H, CH=CHCO), 7.46 (t, *J* = 7.6 Hz, 2H, Ar), 7.45 - 7.31 (m, 1H, Ar), 6.76 (d, *J* = 16.2 Hz, 1H, CH=CHCO), 2.40 (s, 3H, CH₃)

### (E)-4-(2,3-Dihydrobenzofuran-5-yl)but-3-en-2-one (5c)

**¹H-NMR (CDCl₃, 400 MHz) δ (ppm):** 7.36 (d, *J* = 16.2 Hz, 1H, CH=CHCO), 7.31 (s, 1H, Ar), 7.20 (dd, *J* = 8.2, 1.9 Hz, 1H, Ar), 6.68 (d, *J* = 8.6 Hz, 1H, Ar), 6.47 (d, *J* = 16.2 Hz, 1H, CH=CHCO), 4.51 (t, *J* = 8.7 Hz, 2H, OCH₂), 3.12 (t, *J* = 8.7 Hz, 2H, OCH₂CH₂), 2.24 (s, 3H, CH₃); **¹³C-NMR (CDCl₃, 75 MHz) δ (ppm):** 198.3 (CO), 162.5 (OC_{Ar}), 143.7 (ArCH=CH), 129.9 (CH_{Ar}), 128.2 (C_{Ar}), 127.1 (C_{Ar}), 124.6 (ArCH=CH), 124.3 (CH_{Ar}), 109.7 (CH_{Ar}), 71.9 (OCH₂), 29.2 (OCH₂CH₂), 27.4 (CH₃); **MS (ESI, positive mode) m/z:** 399.2 [2M+Na]⁺, 211.0 [M+Na]⁺, 189.0 [M+H]⁺
***ii) General procedure of Wittig.*** A solution of phenylacetaldehyde (1.17 mL, 10 mmol) and 1-(triphenylphosphoranylidene)-2-propanone (2.87 g, 9 mmol) in chloroform (50 mL) was heated to 60 °C for 4 h. After this time, the solvent was evaporated and the residue was purified by silica gel flash column chromatography using a mixture of Hex/AcOEt 8:2 as eluent to give intermediates **5d-f in** 88-92% as yellow oil for **5d** and white solid for **5e-f.**

### (E)-5-Phenylpent-3-en-2-one and (E)-5-phenylpent-4-en-2-one (5d)

**¹H-NMR (CDCl₃, 400 MHz) δ (ppm) (*regioisomer 1*):** 7.41 - 7.01 (m, 5H, Ar), 6.83 (dt, *J* = 15.9, 6.8 Hz, 1H, CH=CHCO), 6.00 (dt, *J* = 15.9, 1.6 Hz, 1H, CH=CHCO), 3.46 (dd, *J* = 6.8, 1.6 Hz, 2H, CH₂), 2.16 (s, 3H, CH₃); **¹H-NMR (CDCl₃, 400 MHz) δ (ppm) *(regioisomer 2*):** 7.41 - 7.01 (m, 5H, Ar), 7.00 (dt, *J* = 15.9, 1.5 Hz, 1H, PhCH=CH), 6.83 (dt, *J* = 15.9, 7.1 Hz, 1H, PhCH=CH), 3.25 (dd, *J* = 7.1, 1.3 Hz, 2H, CH₂), 2.13 (s, 3H, CH₃)

### (E)-4-(Dibenzo[b,d]furan-2-yl)but-3-en-2-one (5e)

**¹H-NMR (CDCl₃, 400 MHz) δ (ppm):** 7.93 (d, *J* = 1.9 Hz, 1H, Ar), 7.80 (d, *J* = 8.5 Hz, 1H, Ar), 7.57 - 7.32 (m, 5H, Ar, CH=CHCO), 7.25 (td, *J* = 7.5, 2.1 Hz, 1H, Ar), 6.64 (d, *J* = 16.2 Hz, 1H, CH=CHCO), 2.29 (s, 3H, CH₃); **¹³C-NMR (CDCl₃, 75 MHz) δ (ppm):** 198.2 (CO), 157.5 (OC_{Ar}), 156.7 (OC_{Ar}), 143.5 (ArCH=CH), 129.4 (C_{Ar}), 127.9 (CH_{Ar}), 127.5 (ArCH=CH), 126.3 (CH_{Ar}), 125.1 (C_{Ar}), 123.6 (C_{Ar}), 123.2 (CH_{Ar}), 120.8 (CH_{Ar}), 120.8 (CH_{Ar}), 112.2 (CH_{Ar}), 111.9 (CH_{Ar}), 27.6 (CH₃)

### (E)-4-(quinolin-6-yl)but-3-en-2-one (5f)

**¹H-NMR (CDCl₃, 400 MHz) δ (ppm):** 7.94 (dd, *J* = 8.4, 1.8 Hz, 1H, Ar), 8.18 (dd, *J* = 8.3, 1.7 Hz, 1H, Ar), 8.12 (d, *J* = 8.6 Hz, 1H, Ar), 8.00 - 7.85 (m, 2H, Ar), 7.68 (d, *J* = 16.3 Hz, 1H, CH=CHCO), 7.45 (dd, *J* = 8.3, 4.3 Hz, 1H, Ar), 6.86 (d, *J* = 16.3 Hz, 1H, CH=CHCO), 2.43 (s, 3H, CH₃); **¹³C-NMR (CDCl₃, 75 MHz) δ (ppm):** 198.2 (CO), 151.6 (NCH_{Ar}), 149.3 (NC_{Ar}), 142.4 (ArCH=CH), 136.6 (CH_{Ar}), 132.9 (C_{Ar}), 130.5 (CH_{Ar}), 129.7 (CH_{Ar}), 128.4 (C_{Ar}), 128.3 (ArCH=CH), 127.5 (CH_{Ar}), 122.1 (CH_{Ar}), 28.0 (CH₃)

### iii) General procedure of ketones bromination.

**Bromination with Br₂.** To a stirred solution of commercially available 4,4-dimethylpentan-2-one **5g** (1 mmol) in MeOH (20 mL) was added dropwise a Br₂ solution at 0 °C. Then, the reaction mixture was allowed to warm to room temperature and was stirred for 3 h. After quenching the reaction with water (20 mL), the aqueous phase was extracted with Et₂O (3 x 30 mL). The combined organic layers were dried (MgSO₄), filtered and carefully concentrated to give the corresponding α-bromo methyl ketone intermediate **6a** as colorless oil in quantitative yields.

### 1-bromo-4,4-dimethylpentan-2-one (6a)

**¹H-NMR (CDCl₃, 400 MHz) δ (ppm):** 3.86 (s, 2H, CH₂Br), 2.52 (s, 2H, CH₂-^{t}Bu), 1.03 (s, 9H, CH₃); **¹³C-NMR (CDCl₃, 75 MHz) δ (ppm):** 201.1 (CO), 52.0 (CH₂Br), 36.3 (CH₂-^{t}Bu), 31.3 (C(CH₃)₃), 29.6 (CH₃)

**Bromination with NBS.** A solution of the corresponding α-methyl ketons **5a-f** in acetonitrile (80mL) were reacted with N-bromosuccinimide in the presence of p-toluensulfonic acid at -78 °C (**6d-f**), or room temperature (**6b, 6c and 6g**) for 5 hours. Then, the reaction mixture was quenched with water (20 ml) and the acetonitrile was removed at reduced pressure. The aqueous residue was extracted with AcOEt (3 x 20 ml) and the combined organic layers were dried (Na₂SO₄), filtered and evaporated to dryness. The final residue was purified by flash column chromatography using silica gel (Hex/AcOEt 8:2) to give the appropriate α-bromo methyl ketones compounds **6a-g** in yields ranging from 17% to 55%. In the case of the quinolin derivative **6g**, the corresponding bromide was not isolated and it was used immediately in the next step without purification.

### (E)-1-bromo-4-(naphthalen-2-yl)but-3-en-2-one (6b)

**¹H-NMR (CDCl₃, 400 MHz) δ (ppm):** 8.00 (d, *J* = 1.8 Hz, 1H, Ar), 7.94 - 7.83 (m, 4H, Ar, CH=CHCO), 7.71 (dd, *J* = 8.5, 1.8 Hz, 1H, Ar), 7.66 - 7.49 (m, 2H, Ar), 7.07 (d, *J* = 15.9 Hz, 1H, CH=CHCO), 4.12 (s, 2H, CH₂Br); **¹³C-NMR (CDCl₃, 75 MHz) δ (ppm):** 191.4 (CO), 145.6 (ArCH=CH), 134.8 (C_{Ar}), 133.4 (C_{Ar}), 131.6 (C_{Ar}), 131.3 (CH_{Ar}), 129.0 (CH_{Ar}), 128.9 (CH_{Ar}), 128.0 (CH_{Ar}), 127.8 (ArCH=CH), 127.0 (CH_{Ar}), 123.6 (CH_{Ar}), 122.4 (CH_{Ar}), 33.3 (CH₂Br); **MS (ESI, positive mode) m/z:** 297 [M+Na]⁺, 275.0 [M+H]⁺, both with a Br isotopic pattern.

### (E)-4-([1,1'-biphenyl]-4-yl)-1-bromobut-3-en-2-one (6c)

**¹H-NMR (CDCl₃, 400 MHz) δ (ppm):** 7.74 (d, *J* = 16.0 Hz, 1H, CH=CHCO), 7.67 - 7.55 (m, 6H, Ar), 7.52 - 7.43 (m, 2H, Ar), 7.41 - 7.32 (m, 1H, Ar), 6.99 (d, *J* = 16.0 Hz, 1H, CH=CHCO), 4.10 (s, 2H, CH₂Br); **¹³C-NMR (CDCl₃, 75 MHz) δ (ppm):** 191.1 (CO), 145.1 (ArCH=CH), 144.0 (C_{Ar}), 140.1 (C_{Ar}), 133.0 (C_{Ar}), 129.3 (CH_{Ar}), 129.1 (CH_{Ar}), 128.2 (CH_{Ar}), 127.8 (ArCH=CH), 127.2 (CH_{Ar}), 122.1 (CH_{Ar}), 33.3 (CH₂Br)

### (E)-1-bromo-4-(2,3-dihydrobenzofuran-5-yl)but-3-en-2-one (6d)

**¹H-NMR (CDCl₃, 400 MHz) δ (ppm):** 7.65 (d, *J* = 15.9 Hz, 1H, CH=CHCO), 7.46 (d, *J* = 2.0 Hz, 1H, Ar), 7.36 (dd, *J* = 8.3, 1.9 Hz, 1H, Ar), 6.80 (d, *J* = 8.2 Hz, 1H, Ar), 6.64 (d, *J* = 15.9 Hz, 1H, CH=CHCO), 4.64 (t, *J* = 8.7 Hz, 2H, OCH₂), 4.05 (s, 2H, CH₂Br), 3.24 (t, *J* = 8.7 Hz, 2H, OCH₂CH₂); **¹³C-NMR (CDCl₃, 75 MHz) δ (ppm):** 191.1 (CO), 163.2 (OC_{Ar}), 145.8 (ArCH=CH), 130.7 (CH_{Ar}), 128.5 (C_{Ar}), 126.9 (C_{Ar}), 125.2 (ArCH=CH), 119.3 (CH_{Ar}), 110.0 (CH_{Ar}), 72.2 (OCH₂), 33.3 (CH₂Br), 29.3 (OCH₂CH₂); **MS (ESI, positive mode) m/z:** 289.0 [M+Na]⁺, 267.0 [M+H]⁺, both with a Br isotopic pattern.

### (E)-1-bromo-5-phenylpent-3-en-2-one (6e)

**¹H-NMR (CDCl₃, 400 MHz) δ (ppm):** 7.37 - 7.31 (m, 2H, Ar), 7.29 - 7.23 (m, 1H, Ar), 7.20 - 7.16 (m, 2H, Ar), 7.12 (dt, *J* = 15.7, 6.8 Hz, 1H, CH=CHCO), 6.28 (dt, *J* = 15.8, 1.6 Hz, 1H, CH=CHCO), 3.98 (s, 2H, CH₂Br), 3.59 (dd, *J* = 7.0, 1.7 Hz, 2H, PhCH₂), **¹³C-NMR (CDCl₃, 75 MHz) δ (ppm):** 191.1 (CO), 148.7 (CH₂CH=CH), 137.3 (C_{Ar}), 129.0 (CH_{Ar}), 127.4 (CH_{Ar}), 127.1 (CH₂CH=CH), 39.0 (Ar-CH₂), 32.7 (CH₂Br)

### (E)-1-bromo-4-(dibenzo[b,d]furan-2-yl)but-3-en-2-one (6f)

**¹H-NMR (CDCl₃, 400 MHz) δ (ppm):** 8.17 (d, *J* = 1.8 Hz, 1H, Ar), 7.98 (dd, *J* = 7.7, 1.4 Hz, 1H, Ar), 7.87 (d, *J* = 16.0 Hz, 1H, CH=CHCO), 7.70 (dd, *J* = 8.5, 1.8 Hz, 1H, Ar), 7.62 - 7.57 (m, 2H, Ar), 7.51 (td, *J* = 8.0, 1.3 Hz, 1H, Ar), 7.39 (td, *J* = 7.6, 1.0 Hz, 1H, Ar), 7.02 (d, *J* = 16.0 Hz, 1H, CH=CHCO), 4.12 (s, 2H, CH₂Br); **¹³C-NMR (CDCl₃, 100 MHz) δ (ppm):** 191.0 (CO), 158.0 (OC_{Ar}), 156.9 (OC_{Ar}), 145.7 (ArCH=CH), 143.3 (CH_{Ar}), 129.1 (C_{Ar}), 128.1 (CH_{Ar}), 128.0 (ArCH=CH), 125.1 (C_{Ar}), 123.6 (C_{Ar}), 123.4 (CH_{Ar}), 121.4 (CH_{Ar}), 121.0 (CH_{Ar}), 112.5 (CH_{Ar}), 112.1 (CH_{Ar}), 33.3 (CH₂Br); **MS (ESI, positive mode) m/z:** 329.0 [M+Na]⁺ with a Br isotopic pattern.

### F) Hantzsch synthesis of thiazoles

*General procedure.* A solution of the corresponding thioamide **4a-e** (1 mmol) in isopropanol (25 mL) was treated with the appropriated α-bromoketones **6a-g** (1.1 mmol). The commercially available **isopropyl 2-bromoacetate (6h), 2-bromo-1-phenylethan-1-one (6i); 1-bromo-3-phenylpropan-2-one (6j), 1-bromo-4-phenylbutan-2-one (6k), 2-bromo-1-(4-(diethylamino)phenyl)ethan-1-one (6l), 2-bromo-1-(4-methoxyphenyl)ethan-1-one (6m) and 2-bromo-1,2-diphenylethan-1-one (6n) were also used.** The reaction mixture was stirred at 70 °C for 4 h in a tube pressure. Then, it was allowed to cool to room temperature and it was concentrated to dryness. The residue was purified by flash column chromatography using silica gel (CH₂Cl₂/MeOH 10:1) to obtain the corresponding thiazoles **7a-q** as colorless oils in yields ranging from 40-96%.

### tert-Butyl (4-(1-(4-(4-isopropoxythiazol-2-yl)-3-(2-(2-oxoimidazolidin-1-yl)ethoxy)phenyl)-1H-1,2,3-triazol-4-yl)butyl)carbamate (7a)

**¹H-NMR (DMSO-d₆, 400 MHz) δ (ppm):** 8.72 (s, 1H, Ar), 8.34 (d, *J* = 8.6 Hz, 1H, Ar), 7.73 (d, *J* = 1.6 Hz, 1H, Ar), 7.66 (dd, *J* = 8.6, 1.8 Hz, 1H, Ar), 6.81 (s, 1H, Ar), 6.64 (bs, 1H, NH), 6.56 (bs, 1H, NH), 4.73 (quin, *J* = 6.1 Hz, 1H, OCH(CH₃)₂), 4.47 (t, *J* = 5.6 Hz, 2H, OCH₂), 3.63 (t, *J* = 5.6 Hz, 2H, OCH₂CH₂), 3.49 (t, *J* = 7.8 Hz, 2H, CH₂CH₂NHCON), 3.23 (t, *J* = 7.9 Hz, 2H, CH₂CH₂NHCON), 2.95 (m, 2H, CH₂NHBoc), 2.72 (m, 2H, TrizCH₂), 1.65 (m, 2H, TrizCH₂CH₂), 1.46 (m, 2H, CH₂CH₂NHBoc), 1.36 (s, 9H, C(CH₃)₃), 1.32 (d, *J* = 6.1 Hz, 6H, CH(CH₃)₂); **¹³C-NMR (DMSO-d₆, 100 MHz) δ (ppm):** 162.2 (NHCON), 156.7 (OC_{Ar}), 155.9 (OC_{Ar}), 155.6 (NHCOO), 148.2 (C_{Ar}), 137.9 (C_{Ar}), 128.6 (CH_{Ar}), 120.9 (CH_{Ar}), 120.3 (C_{Ar}), 112.0 (CH_{Ar}), 104.3 (CH_{Ar}), 93.1 (CH_{Ar}), 77.4 (OC(CH₃)₃), 71.8 (OCH(CH₃)₂), 67.6 (OCH₂), 45.3 (CH₂CH₂NHCON), 42.4 (OCH₂CH₂), 37.6 (CH₂CH₂NHCON), 29.0 (CH₂CH₂NHBoc), 28.3 (C(CH₃)₃), 26.1 (TrizCH₂CH₂), 24.7 (TrizCH₂), 21.9 (CH(CH₃)₂); **MS (ESI, positive mode) m/z:** 1193.5 [2M+Na]⁺, 608.3 [M+Na]⁺, 586.3 [M+H]⁺

### tert-Butyl (4-(1-(4-(4-neopentylthiazol-2-yl)-3-(2-(2-oxoimidazolidin-1-yl)ethoxy)phenyl)-1H-1,2,3-triazol-4-yl)butyl)carbamate (7b)

**¹H-NMR (CD₃OD, 400 MHz) δ (ppm):** 8.45 (s, 1H, Ar), 8.44 (d, *J* = 8.6 Hz, 1H, Ar), 7.72 (d, *J* = 2.0 Hz, 1H, Ar), 7.58 (dd, *J* = 8.6, 2.0 Hz, 1H, Ar), 7.21 (s, 1H, Ar), 4.50 (t, *J* = 5.5 Hz, 2H, OCH₂), 3.77 (t, *J* = 5.5 Hz, 2H, OCH₂CH₂), 3.62 (dd, *J* = 9.2, 6.9 Hz, 2H, CH₂CH₂NHCON), 3.38 (dd, *J* = 9.3, 6.9 Hz, 2H, CH₂CH₂NHCON), 3.10 (t, *J* = 6.9 Hz, 2H, CH₂NHBoc), 2.82 (t, *J* = 7.5 Hz, 2H, TrizCH₂), 2.75 (s, 2H, CH₂-^{t}Bu), 1.77 (quin, *J* = 7.6 Hz, 2H, TrizCH₂CH₂), 1.59 (quin, *J* = 7.6 Hz, 2H, CH₂CH₂NHBoc), 1.43 (s, 9H, OC(CH₃)₃), 1.00 (s, 9H, CH₂C(CH₃)₃); **¹³C-NMR (CD₃OD, 75 MHz) δ (ppm):** 165.1 (NHCON), 161.1 (OC_{Ar}), 157.4 (C_{Ar}), 155.8 (C_{Ar}), 150.0 (C_{Ar}), 139.7 (C_{Ar}), 130.8 (CH_{Ar}), 123.9 (C_{Ar}), 121.5 (CH_{Ar}), 118.4 (CH_{Ar}), 113.6 (CH_{Ar}), 105.8 (CH_{Ar}), 79.9 (OC(CH₃)₃), 68.4 (OCH₂), 46.9 (CH₂CH₂NHCON), 45.7 (CH₂-^{t}Bu), 43.9 (OCH₂CH₂), 41.0 (CH₂NHBoc), 39.3 (CH₂CH₂NHCON), 32.4 (CH₂C(CH₃)₃), 30.4 (CH₂CH₂NHBoc), 30.0 (CH₂C(CH₃)₃), 28.8 (OC(CH₃)₃), 27.6 (TrizCH₂CH₂), 26.0 (TrizCH₂); **HRMS (ES, positive mode) m/z:** calculated for C₃₀H₄₃N₇O₄S 597.3097; Found 597.3096 (-0.18 ppm)

### Benzyl (4-(1-(3-(2-(2-oxoimidazolidin-1-yl)ethoxy)-4-(4-phenylthiazol-2-yl)phenyl)-1H-1,2,3-triazol-4-yl)butyl)carbamate (7c)

**¹H-NMR (DMSO-d₆, 400 MHz) δ (ppm):** 8.74 (s, 1H, Ar), 8.60 (d, *J* = 8.6 Hz, 1H, Ar), 8.21 (s, 1H, Ar), 8.10 (dd, *J* = 7.7, 2.0 Hz, 2H, Ar), 8.09 (s, 1H, Ar), 7.77 (d, *J* = 1.9 Hz, 1H, Ar), 7.72 (dd, *J* = 8.6, 1.9 Hz, 1H, Ar), 7.48 (t, *J* = 7.8 Hz, 1H, Ar), 7.42 - 7.24 (m, 6H, Ar), 6.41 (bs, 1H, NH), 5.01 (s, 2H, OCH₂Ph), 4.50 (t, *J* = 5.7 Hz, 2H, OCH₂), 3.67 (t, *J* = 5.6 Hz, 2H, OCH₂CH₂), 3.51 (dd, *J* = 9.0, 6.6 Hz, 2H, CH₂CH₂NHCON), 3.23 (dd, *J* = 9.0, 6.6 Hz, 2H, CH₂CH₂NHCON), 3.07 (q, *J* = 6.6 Hz, 2H, CH₂NHCbz), 2.74 (t, *J* = 7.5 Hz, 2H, TrizCH₂), 1.70 (quin, *J* = 7.5 Hz, 2H, TrizCH₂CH₂), 1.51 (quin, *J* = 6.6 Hz, 2H, CH₂CH₂NHCbz); **¹³C-NMR (DMSO-d₆, 75 MHz) δ (ppm):** 162.2 (NHCON), 162.1 (OC_{Ar}), 160.1 (C_{Ar}), 156.1 (C_{Ar}), 155.8 (C_{Ar}), 153.4 (C_{Ar}), 148.1 (C_{Ar}), 138.3 (C_{Ar}), 137.3 (C_{Ar}), 134.1 (CH_{Ar}), 129.2 (CH_{Ar}), 128.8 (CH_{Ar}), 128.3 (CH_{Ar}), 128.1 (CH_{Ar}), 127.7 (CH_{Ar}), 126.2 (C_{Ar}), 121.1 (CH_{Ar}), 120.3 (CH_{Ar}), 115.8 (CH_{Ar}), 112.1 (CH_{Ar}), 104.3 (CH_{Ar}), 67.6 (OCH₂), 65.1 (OCH₂Ph), 45.3 (CH₂CH₂NHCON), 42.4 (OCH₂CH₂), 37.6 (CH₂CH₂NHCON), 28.9 (CH₂CH₂NHCbz), 26.0 (TrizCH₂CH₂), 24.7 (TrizCH₂); **HRMS (ES, positive mode) m/z:** calculated for C₃₄H₃₅N₇O₄S 637.2471; Found 637.2474 (0.49 ppm)

### tert-Butyl (4-(1-(4-(4-benzylthiazol-2-yl)-3-(2-(2-oxoimidazolidin-1-yl)ethoxy)phenyl)-1H-1,2,3-triazol-4-yl)butyl)carbamate (7d)

**¹H-NMR (DMSO-d₆, 300 MHz) δ (ppm):** 8.72 (s, 1H, Ar), 8.40 (d, *J* = 8.6 Hz, 1H, Ar), 7.73 (d, *J* = 2.0 Hz, 1H, Ar), 7.66 (dd, *J* = 8.4, 2.0 Hz, 1H, Ar), 7.40 - 7.14 (m, 6H, Ar), 6.80 (bs, 1H, NH), 6.39 (bs, 1H, NH), 4.46 (t, *J* = 5.5 Hz, 2H, OCH₂), 4.14 (s, 2H, CH₂Ph), 3.62 (t, *J* = 5.5 Hz, 2H, OCH₂CH₂), 3.48 (m, 2H, CH₂CH₂NHCON), 3.20 (m, 2H, CH₂CH₂NHCON), 2.95 (m, 2H, CH₂NHBoc), 2.72 (t, *J* = 7.6 Hz, 2H, TrizCH₂), 1.75 - 1.60 (m, 2H, TrizCH₂CH₂), 1.48 (m, 2H, CH₂CH₂NHBoc), 1.36 (s, 9H, OC(CH₃)₃); **HRMS (ES, positive mode) m/z:** calculated for C₃₂H₃₉N₇O₄S 617.2784; Found 617.2775 (-1.44 ppm)

### Benzyl (4-(1-(3-(2-(2-oxoimidazolidin-1-yl)ethoxy)-4-(4-phenethylthiazol-2-yl)phenyl)-1H-1,2,3-triazol-4-yl)butyl)carbamate (7e)

**¹H-NMR (CDCl₃, 400 MHz) δ (ppm):** 8.46 (d, *J* = 8.5 Hz, 1H, Ar), 7.81 (s, 1H, Ar), 7.51 (d, *J* = 2.0 Hz, 1H, Ar), 7.38 - 7.06 (m, 11H, Ar), 6.86 (s, 1H, Ar), 5.06 (t, *J* = 6.0 Hz, 1H, NHCbz), 5.01 (s, 2H, OCH₂Ph), 4.34 (t, *J* = 5.7 Hz, 2H, OCH₂), 3.69 (t, *J* = 5.7 Hz, 2H, OCH₂CH₂), 3.50 (dd, *J* = 9.0, 6.7 Hz, 2H, CH₂CH₂NHCON), 3.26 (t, *J* = 7.9 Hz, 2H, CH₂CH₂NHCON), 3.18 (q, *J* = 7.4 Hz, 2H, CH₂NHCbz), 3.12 - 2.97 (m, 4H, CH₂CH₂Ph), 2.76 (t, *J* = 7.4 Hz, 2H, TrizCH₂), 1.71 (quin, *J* = 7.4 Hz, 2H, TrizCH₂CH₂), 1.59 (quin, *J* = 7.1 Hz, 2H, CH₂CH₂NHCbz); **¹³C-NMR (CDCl₃, 75 MHz) δ (ppm):** 162.8 (NHCON), 160.3 (OC_{Ar}), 156.6 (C_{Ar}), 156.0 (C_{Ar}), 155.9 (C_{Ar}), 148.7 (C_{Ar}), 141.7 (C_{Ar}), 138.3 (C_{Ar}), 136.7 (C_{Ar}), 130.0 (CH_{Ar}), 128.6 (CH_{Ar}), 128.6 (CH_{Ar}), 128.4 (CH_{Ar}), 128.2 (CH_{Ar}), 126.1 (CH_{Ar}), 122.7 (C_{Ar}), 119.1 (CH_{Ar}), 115.1 (CH_{Ar}), 112.4 (CH_{Ar}), 104.5 (CH_{Ar}), 67.8 (OCH₂), 66.7 (OCH₂Ph), 46.4 (CH₂CH₂NHCON), 43.0 (OCH₂CH₂), 40.8 (CH₂NHCbz), 38.4 (CH₂CH₂NHCON), 35.6 (CH₂CH₂Ph), 33.4 (CH₂CH₂Ph), 29.4 (CH₂CH₂NHCbz), 26.4 (TrizCH₂CH₂), 25.2 (TrizCH₂); **HRMS (ES, positive mode) m/z:** calculated for C₃₆H₃₉N₇O₄S 665.2784; Found 665.2791 (1.00 ppm)

### Benzyl (E)-(4-(1-(3-(2-(2-oxoimidazolidin-1-yl)ethoxy)-4-(4-(3-phenylprop-1-en-1-yl)thiazol-2-yl)phenyl)-1H-1,2,3-triazol-4-yl)butyl)carbamate (7f)

**¹H-NMR (CDCl₃, 400 MHz) δ (ppm):** 8.55 (d, *J* = 8.5 Hz, 1H, Ar), 7.87 (s, 1H, Ar), 7.57 (d, *J* = 1.9 Hz, 1H, Ar), 7.40 - 7.18 (m, 11H, Ar), 7.10 (s, 1H, Ar), 6.86 (dt, *J* = 15.5, 6.9 Hz, 1H, CH₂CH=CH), 6.50 (dt, *J* = 15.5, 1.6 Hz, 1H, CH₂CH=CH), 5.05 (bs, 1H, NH), 5.08 (s, 2H, OCH₂Ph), 4.73 (bs, 1H, NH), 4.41 (t, *J* = 5.7 Hz, 2H, OCH₂), 3.75 (t, *J* = 5.7 Hz, 2H, OCH₂CH₂), 3.61 - 3.52 (m, 4H, CH₂CH₂NHCON, CH₂CH=CH), 3.32 (t, *J* = 7.9 Hz, 2H, CH₂CH₂NHCON), 3.25 (q, *J* = 6.7 Hz, 2H, CH₂NHCbz), 2.82 (t, *J* = 7.3 Hz, 2H, TrizCH₂), 1.77 (quin, *J* = 7.5 Hz, 2H, TrizCH₂CH₂), 1.63 (quin, *J* = 7.3 Hz, 2H, CH₂CH₂NHCbz); **¹³C-NMR (CDCl₃, 100 MHz) δ (ppm):** 162.7 (NHCON), 160.6 (OC_{Ar}), 156.6 (C_{Ar}), 156.1 (C_{Ar}), 153.3 (C_{Ar}), 148.8 (C_{Ar}), 140.0 (C_{Ar}), 138.5 (C_{Ar}), 136.7 (C_{Ar}), 132.6 (CH_{Ar}), 130.2 (CH_{Ar}), 128.9 (CH_{Ar}), 128.6 (CH_{Ar}), 128.2 (CH_{Ar}), 126.3 (CH_{Ar}), 124.3 (CH_{Ar}), 122.5 (C_{Ar}), 119.1 (CH_{Ar}), 115.1 (CH_{Ar}), 112.4 (CH_{Ar}), 104.5 (CH_{Ar}), 67.8 (OCH₂), 66.7 (OCH₂Ph), 46.4 (CH₂CH₂NHCON), 43.0 (OCH₂CH₂), 40.9 (CH₂NHCbz), 39.3 (CH₂CH₂NHCON), 38.4 (PhCH₂CH=CH), 29.4 (CH₂CH₂NHCbz), 26.4 (TrizCH₂CH₂), 25.2 (TrizCH₂); **HRMS (ES, positive mode) m/z:** calculated for C₃₇H₃₉N₇O₄S 677.2784; Found 677.2781 (-0.53 ppm)

### Benzyl (E)-(4-(1-(4-(4-(2-(naphthalen-2-yl)vinyl)thiazol-2-yl)-3-(2-(2-oxoimidazolidin-1-yl)ethoxy)phenyl)-1H-1,2,3-triazol-4-yl)butyl)carbamate (7g)

**¹H-NMR (CDCl₃, 400 MHz) δ (ppm):** 8.61 (d, *J* = 8.5 Hz, 1H, Ar), 7.90 - 7.64 (m, 7H, Ar), 7.55 (s, 1H, Ar), 7.45 - 7.36 (m, 3H, Ar), 7.30 - 7.14 (m, 7H, Ar), 5.03 (s, 2H, OCH₂Ph), 4.97 (bs, 1H, NH), 4.48 (bs, 1H, NH), 4.39 (t, *J* = 5.7 Hz, 2H, OCH₂), 3.73 (t, *J* = 5.7 Hz, 2H, OCH₂CH₂), 3.52 (dd, *J* = 9.0, 6.6 Hz, 2H, CH₂CH₂NHCON), 3.28 (t, *J* = 7.9 Hz, 2H, CH₂CH₂NHCON), 3.20 (q, *J* = 6.7 Hz, 2H, CH₂NHCbz), 2.78 (t, *J* = 7.3 Hz, 2H, TrizCH₂), 1.74 (quin, *J* = 7.5 Hz, 2H, TrizCH₂CH₂), 1.56 (m, 2H, CH₂CH₂NHCbz); **¹³C-NMR (CDCl₃, 75 MHz) δ (ppm):** 162.7 (NHCON), 161.0 (OC_{Ar}), 156.6 (C_{Ar}), 156.2 (C_{Ar}), 153.5 (C_{Ar}), 148.8 (C_{Ar}), 138.6 (C_{Ar}), 136.7 (C_{Ar}), 134.7 (CH_{Ar}), 133.8 (C_{Ar}), 133.3 (C_{Ar}), 131.5 (CH_{Ar}), 130.3 (CH_{Ar}), 128.7 (CH_{Ar}), 128.4 (CH_{Ar}), 128.3 (CH_{Ar}), 128.2 (CH_{Ar}), 127.8 (CH_{Ar}), 127.2 (C_{Ar}), 126.5 (CH_{Ar}), 126.1 (CH_{Ar}), 123.6 (CH_{Ar}), 121.8 (CH_{Ar}), 119.2 (CH_{Ar}), 117.1 (CH_{Ar}), 112.5 (CH_{Ar}), 104.5 (CH_{Ar}), 67.7 (OCH₂), 66.8 (OCH₂Ph), 46.4 (CH₂CH₂NHCON), 43.0 (OCH₂CH₂), 40.9 (CH₂NHCbz), 38.4 (CH₂CH₂NHCON), 29.5 (CH₂CH₂NHCbz), 26.4 (TrizCH₂CH₂), 25.2 (TrizCH₂); **HRMS (ES, positive mode) m/z:** calculated for C₄₀H₃₉N₇O₄S 713.2784; Found 713.2779 (-0.72 ppm)

### Benzyl (E)-(4-(1-(4-(4-(2-([1,1'-biphenyl]-4-yl)vinyl)thiazol-2-yl)-3-(2-(2-oxoimidazolidin-1-yl)ethoxy)phenyl)-1H-1,2,3-triazol-4-yl)butyl)carbamate (7h)

**¹H-NMR (CDCl₃, 400 MHz) δ (ppm):** 8.57 (d, *J* = 8.5 Hz, 1H, Ar), 7.81 (s, 1H, Ar), 7.61 - 7.45 (m, 8H, Ar), 7.40 - 7.31 (m, 3H, Ar), 7.31 - 7.16 (m, 7H, Ar), 7.12 (d, *J* = 16.0 Hz, 1H, ThiazCH=CH), 5.02 (s, 2H, OCH₂Ph), 4.68 (bs, 1H, NH), 4.36 (t, *J* = 5.7 Hz, 2H, OCH₂), 3.70 (t, *J* = 5.8 Hz, 2H, OCH₂CH₂), 3.50 (dd, *J* = 9.0, 6.7 Hz, 2H, CH₂CH₂NHCON), 3.26 (t, *J* = 7.9 Hz, 2H, CH₂CH₂NHCON), 3.16 (q, *J* = 6.6 Hz, 2H, CH₂NHCbz), 2.76 (t, *J* = 7.3 Hz, 2H, TrizCH₂), 1.75 (quin, *J* = 7.5 Hz, 2H, TrizCH₂CH₂), 1.55 (quin, *J* = 7.3 Hz, 2H, CH₂CH₂NHCbz); **¹³C-NMR (CDCl₃, 75 MHz) δ (ppm):** 162.7 (NHCON), 160.9 (OC_{Ar}), 156.6 (C_{Ar}), 156.2 (C_{Ar}), 153.4 (C_{Ar}), 148.8 (C_{Ar}), 140.7 (C_{Ar}), 140.6 (C_{Ar}), 138.6 (CH_{Ar}), 136.7 (C_{Ar}), 136.2 (CH_{Ar}), 130.9 (CH_{Ar}), 130.3 (CH_{Ar}), 128.9 (CH_{Ar}), 128.6 (CH_{Ar}), 128.3 (CH_{Ar}), 127.5 (CH_{Ar}), 127.0 (CH_{Ar}), 122.4 (C_{Ar}), 121.5 (CH_{Ar}), 119.1 (CH_{Ar}), 117.4 (CH_{Ar}), 112.4 (CH_{Ar}), 104.4 (CH_{Ar}), 67.7 (OCH₂), 66.8 (OCH₂Ph), 46.4 (CH₂CH₂NHCON), 42.9 (OCH₂CH₂), 40.9 (CH₂NHCbz), 38.4 (CH₂CH₂NHCON), 29.4 (CH₂CH₂NHCbz), 26.4 (TrizCH₂CH₂), 25.2 (TrizCH₂); **HRMS (ES, positive mode) m/z:** calculated for C₄₂H₄₁N₇O₄S 739.2941; Found 739.2972 (4.21 ppm)

### Benzyl (E)-(4-(1-(3-(2-(2-oxoimidazolidin-1-yl)ethoxy)-4-(4-(2-(quinolin-6-yl)vinyl)thiazol-2-yl)phenyl)-1H-1,2,3-triazol-4-yl)butyl)carbamate (7i)

**¹H-NMR (CDCl₃, 400 MHz) δ (ppm):** 8.79 (d, *J* = 4.2 Hz, 1H, Ar), 8.59 (d, *J* = 8.6 Hz, 1H, Ar), 8.06 (dd, *J* = 6.5, 2.2 Hz, 1H, Ar), 8.01 (d, *J* = 8.8 Hz, 1H, Ar), 7.91 (dd, *J* = 8.9, 2.0 Hz, 1H, Ar), 7.83 (s, 1H, Ar), 7.80 (s, 1H, Ar), 7.69 (d, *J* = 15.9 Hz, 1H, ThiazCH=CH), 7.53 (s, 1H, Ar), 7.37 (d, *J* = 8.6 Hz, 1H, Ar), 7.34 - 7.15 (m, 8H, Ar), 5.06 (bs, 1H, NH), 5.02 (s, 2H, OCH₂Ph), 4.67 (bs, 1H,NHCON), 4.37 (t, *J* = 5.8 Hz, 2H, OCH₂), 3.71 (t, *J* = 5.9 Hz, 2H, OCH₂CH₂), 3.52 (dd, *J* = 9.0, 6.7 Hz, 2H, CH₂CH₂NHCON), 3.28 (t, *J* = 8.0 Hz, 2H, CH₂CH₂NHCON), 3.20 (q, *J* = 6.6 Hz, 2H, CH₂NHCbz), 2.77 (t, *J* = 7.4 Hz, 2H, TrizCH₂), 1.72 (quin, *J* = 7.6 Hz, 2H, TrizCH₂CH₂), 1.57 (quin, *J* = 7.4 Hz, 2H, CH₂CH₂NHCbz); **¹³C-NMR (CDCl₃, 100 MHz) δ (ppm):** 162.7 (NHCON), 161.1 (OC_{Ar}), 156.6 (C_{Ar}), 156.2 (C_{Ar}), 153.1 (NHCOO), 150.2 (C_{Ar}), 148.8 (C_{Ar}), 148.2 (C_{Ar}), 138.7 (C_{Ar}), 136.7 (C_{Ar}), 136.2 (CH_{Ar}), 135.5 (CH_{Ar}), 130.6 (CH_{Ar}), 130.3 (CH_{Ar}), 129.8 (C_{Ar}), 128.7 (CH_{Ar}), 128.6 (CH_{Ar}), 128.2 (CH_{Ar}), 127.4 (CH_{Ar}), 126.5 (CH_{Ar}), 122.9 (CH_{Ar}), 122.3 (C_{Ar}), 121.6 (CH_{Ar}), 119.1 (CH_{Ar}), 117.8 (CH_{Ar}), 112.5 (CH_{Ar}), 104.5 (CH_{Ar}), 67.7 (OCH₂), 66.8 (OCH₂Ph), 46.4 (CH₂CH₂NHCON), 43.0 (OCH₂CH₂), 40.9 (CH₂NHCbz), 38.4 (CH₂CH₂NHCON), 29.5 (CH₂CH₂NHCbz), 26.4 (TrizCH₂CH₂), 25.2 (TrizCH₂); **HRMS (ES, positive mode) m/z:** calculated for C₃₉H₃₈N₈O₄S 714.2737; Found 714.2734 (-0.38 ppm)

### Benzyl (E)-(4-(1-(4-(4-(2-(2,3-dihydrobenzofuran-5-yl)vinyl)thiazol-2-yl)-3-(2-(2-oxoimidazolidin-1-yl)ethoxy)phenyl)-1H-1,2,3-triazol-4-yl)butyl)carbamate (7j)

**¹H-NMR (CDCl₃, 400 MHz) δ (ppm):** 8.55 (d, *J* = 8.6 Hz, 1H, Ar), 7.81 (s, 1H, Ar), 7.51 (s, 1H, Ar), 7.46 (d, *J* = 15.8 Hz, 1H, ThiazCH=CH), 7.44 - 7.15 (m, 8H, Ar), 7.12 (s, 1H, Ar), 6.92 (d, *J* = 16.0 Hz, 1H, ThiazCH=CH), 6.71 (d, *J* = 8.2 Hz, 1H Ar), 5.04 (bs, 1H, NH), 5.03 (s, 2H, OCH₂Ph), 4.68 (bs, 1H, NH), 4.52 (t, *J* = 8.7 Hz, 2H, OCH₂CH₂Ph), 4.35 (t, *J* = 5.7 Hz, 2H, OCH₂), 3.69 (t, *J* = 5.6 Hz, 2H, OCH₂CH₂), 3.50 (t, *J* = 7.8 Hz, 2H, CH₂CH₂NHCON), 3.26 (t, *J* = 8.0 Hz, 2H, CH₂CH₂NHCON), 3.26 - 3.12 (m, 4H, CH₂NHCbz, OCH₂CH₂Ph), 2.76 (t, *J* = 7.3 Hz, 2H, TrizCH₂), 1.72 (quin, *J* = 7.3 Hz, 2H, TrizCH₂CH₂), 1.56 (quin, *J* = 7.6 Hz, 2H, CH₂CH₂NHCbz); **¹³C-NMR (CDCl₃, 100 MHz) δ (ppm):** 162.7 (NHCON), 160.7 (OC_{Ar}), 160.3 (OC_{Ar}), 156.6 (C_{Ar}), 156.1 (C_{Ar}), 153.8 (C_{Ar}), 148.8 (C_{Ar}), 138.5 (C_{Ar}), 136.7 (C_{Ar}), 131.5 (C_{Ar}), 130.3 (CH_{Ar}), 130.0 (CH_{Ar}), 128.6 (CH_{Ar}), 128.2 (CH_{Ar}), 128.2 (CH_{Ar}), 127.8 (CH_{Ar}), 127.5 (C_{Ar}), 123.0 (CH_{Ar}), 122.5 (C_{Ar}), 119.1 (CH_{Ar}), 118.9 (CH_{Ar}), 115.8 (CH_{Ar}), 112.5 (CH_{Ar}), 109.5 (CH_{Ar}), 104.5 (CH_{Ar}), 71.6 (OCH₂CH₂Ph), 67.8 (OCH₂), 66.8 (OCH₂Ph), 46.4 (CH₂CH₂NHCON), 43.0 (OCH₂CH₂), 40.9 (CH₂NHCbz), 38.4 (CH₂CH₂NHCON), 29.7 (OCH₂CH₂Ph), 29.5 (CH₂CH₂NHCbz), 26.4 (TrizCH₂CH₂), 25.2 (TrizCH₂); **HRMS (ES, positive mode) m/z:** calculated for C₃₈H₃₉N₇O₅S 705.2733; Found 705.2728 (-0.82 ppm)

### Benzyl (E)-(4-(1-(4-(4-(2-(dibenzo[b,d]furan-2-yl)vinyl)thiazol-2-yl)-3-(2-(2-oxoimidazolidin-1-yl)ethoxy)phenyl)-1H-1,2,3-triazol-4-yl)butyl)carbamate (7k)

**¹H-NMR (DMSO-d₆, 400 MHz) δ (ppm):** 8.70 (bs, 1H, NH), 8.60 (dd, *J* = 8.5, 2.2 Hz, 1H, Ar), 8.19 (d, *J* = 7.6 Hz, 1H, Ar), 7.88 - 7.66 (m, 8H, Ar), 7.60 - 7.50 (m, 1H, Ar), 7.47 - 7.24 (m, 8H, Ar), 6.31 (bs, 1H, NH), 5.02 (s, 2H, OCH₂Ph), 4.51 (t, *J* = 5.8 Hz, 2H, OCH₂), 3.67 (t, *J* = 5.7 Hz, 2H, OCH₂CH₂), 3.52 (dd, *J* = 8.8, 6.8 Hz, 2H, CH₂CH₂NHCON), 3.35 - 3.08 (m, 4H, CH₂CH₂NHCON, CH₂NHCbz), 2.76 (t, *J* = 7.5 Hz, 2H, TrizCH₂), 1.72 (quin, *J* = 7.2 Hz, 2H, TrizCH₂CH₂), 1.54 (quin, *J* = 7.2 Hz, 2H, CH₂CH₂NHCbz); **¹³C-NMR (DMSO-d₆, 75 MHz) δ (ppm):** 161.9 (NHCON), 160.1 (OC_{Ar}), 155.8 (OC_{Ar}), 155.8 (OC_{Ar}), 155.0 (NHCOO), 152.6 (C_{Ar}), 147.9 (C_{Ar}), 138.2 (C_{Ar}), 137.1 (C_{Ar}), 132.1 (C_{Ar}), 130.2 (CH_{Ar}), 129.1 (CH_{Ar}), 128.0 (CH_{Ar}), 127.5 (C_{Ar}), 127.4 (CH_{Ar}), 127.3 (CH_{Ar}), 126.1 (CH_{Ar}), 124.0 (C_{Ar}), 123.3 (C_{Ar}), 123.0 (CH_{Ar}), 121.2 (C_{Ar}), 121.1 (CH_{Ar}), 121.0 (CH_{Ar}), 120.0 (CH_{Ar}), 118.7 (CH_{Ar}), 117.4 (CH_{Ar}), 112.0 (CH_{Ar}), 111.6 (CH_{Ar}), 111.4 (CH_{Ar}), 104.5 (CH_{Ar}), 67.5 (OCH₂), 64.9 (OCH₂Ph), 45.1 (CH₂CH₂NHCON), 42.3 (OCH₂CH₂), 37.4 (CH₂CH₂NHCON), 28.7 (CH₂CH₂NHCbz), 25.8 (TrizCH₂CH₂), 24.5 (TrizCH₂); **HRMS (ES, positive mode) m/z:** calculated for C₄₂H₃₉N₇O₅S 753.2733; Found 753.2713 (-2.65 ppm)

### Benzyl (4-(1-(4-(4-(4-methoxyphenyl)thiazol-2-yl)-3-(2-(2-oxoimidazolidin-1-yl)ethoxy)phenyl)-1H-1,2,3-triazol-4-yl)butyl)carbamate (7l)

**¹H-NMR (CDCl₃, 400 MHz) δ (ppm):** 8.67 (d, *J* = 8.5 Hz, 1H, Ar), 7.95 (d, *J* = 8.7 Hz, 2H, Ar), 7.89 (s, 1H, Ar), 7.60 (s, 1H, Ar), 7.46 (s, 1H, Ar), 7.44 - 7.20 (m, 7H, Ar), 6.99 (d, *J* = 8.7 Hz, 1H, Ar), 5.08 (s, 2H, OCH₂Ph), 5.07 (bs, 1H, NH), 4.54 (bs, 1H, NH), 4.46 (t, *J* = 5.8 Hz, 2H, OCH₂), 3.86 (s, 3H, OMe), 3.79 (t, *J* = 5.8 Hz, 2H, OCH₂CH₂), 3.60 (dd, *J* = 8.9, 6.7 Hz, 2H, CH₂CH₂NHCON), 3.34 (t, *J* = 8.0 Hz, 2H, CH₂CH₂NHCON), 3.26 (q, *J* = 6.6 Hz, 2H, CH₂NHCbz), 2.85 (t, *J* = 7.3 Hz, 2H, TrizCH₂), 1.79 (quin, *J* = 7.4 Hz, 2H, TrizCH₂CH₂), 1.63 (m, 2H, CH₂CH₂NHCbz); **¹³C-NMR (CDCl₃, 75 MHz) δ (ppm):** 162.7 (NHCON), 160.5 (OC_{Ar}), 159.8 (OC_{Ar}), 156.6 (C_{Ar}), 156.1 (C_{Ar}), 154.5 (C_{Ar}), 148.8 (C_{Ar}), 138.5 (C_{Ar}), 136.7 (C_{Ar}), 130.2 (CH_{Ar}), 128.7 (CH_{Ar}), 128.3 (CH_{Ar}), 127.8 (CH_{Ar}), 127.6 (CH_{Ar}), 122.7 (CH_{Ar}), 119.2 (C_{Ar}), 114.2 (CH_{Ar}), 112.8 (CH_{Ar}), 112.5 (CH_{Ar}), 104.3 (CH_{Ar}), 67.8 (OCH₂), 66.8 (OCH₂Ph), 55.5 (OCH₃), 46.5 (CH₂CH₂NHCON), 43.0 (OCH₂CH₂), 40.9 (CH₂NHCbz), 38.4 (CH₂CH₂NHCON), 29.5 (CH₂CH₂NHCbz), 26.4 (TrizCH₂CH₂), 25.3 (TrizCH₂); **HRMS (ES, positive mode) m/z:** calculated for C₃₅H₃₇N₇O₅S 667.2577; Found 667.2557 (-3.02 ppm)

### Benzyl 4-(2-(5-(4-(4-(((benzyloxy)carbonyl)amino)butyl)-1H-1,2,3-triazol-1-yl)-2-(4-phenethylthiazol-2-yl)phenoxy)ethyl)piperidine-1-carboxylate (7m)

**¹H-NMR (CDCl₃, 400 MHz) δ (ppm):** 8.48 (d, *J* = 8.5 Hz, 1H, Ar), 7.73 (s, 1H, Ar), 7.52 (d, *J* = 2.0 Hz, 1H, Ar), 7.35 - 7.03 (m, 16H, Ar), 6.86 (s, 1H, Ar), 5.05 (s, 2H, NCOOCH₂), 5.02 (s, 2H, NCOOCH₂), 4.77 (bs, 1H, NH), 4.25 (t, *J* = 6.2 Hz, 2H, OCH₂), 4.10 (m, 2H, H'HCN(Cbz)CHH'), 3.19 (q, *J* = 6.7 Hz, 2H, CH₂NHCbz), 3.14 - 3.01 (m, 4H, CH₂CH₂Ph), 2.78 - 2.65 (m, 4H, TrizCH₂, H'HCN(Cbz)CHH'), 1.88 (q, *J* = 6.3 Hz, 2H, OCH₂CH₂), 1.82 - 1.66 (m, 5H, TrizCH₂CH₂, CH, CH(CHH')₂), 1.55 (m, 2H, CH₂CH₂NHCbz), 1.16 (m, 2H, CH(CHH')₂); **¹³C-NMR (CDCl₃, 100 MHz) δ (ppm):** 160.4 (C_{Ar}), 156.5 (NCOO), 156.4 (NCOO), 156.0 (C_{Ar}), 155.4 (OC_{Ar}), 148.8 (C_{Ar}), 141.7 (C_{Ar}), 138.3 (C_{Ar}), 137.0 (C_{Ar}), 136.7 (C_{Ar}), 129.8 (CH_{Ar}), 128.6 (CH_{Ar}), 128.6 (CH_{Ar}), 128.5 (CH_{Ar}), 128.2 (CH_{Ar}), 128.1 (CH_{Ar}), 128.0 (CH_{Ar}), 126.1 (CH_{Ar}), 122.7 (C_{Ar}), 119.0 (CH_{Ar}), 115.2 (CH_{Ar}), 111.8 (CH_{Ar}), 104.4 (CH_{Ar}), 67.1 (OCH₂Ph), 67.1 (OCH₂Ph), 66.8 (OCH₂), 44.3 (H'HCN(Cbz)CHH'), 40.9 (CH₂NHCbz), 35.7 (OCH₂CH₂), 35.6 (CH₂CH₂Ph), 33.5 (CH), 33.1 (CH₂CH₂Ph), 32.1 (CH(CHH')₂), 29.6 (CH₂CH₂NHCbz), 26.6 (TrizCH₂CH₂), 25.3 (TrizCH₂); **HRMS (ES, positive mode) m/z:** calculated for C₄₆H₅₀N₆O₅S 798.3563; Found 798.3571 (0.90 ppm)

### tert-Butyl (4-(1-(3-methoxy-4-(4-phenethylthiazol-2-yl)phenyl)-1H-1,2,3-triazol-4-yl)butyl)carbamate (7n)

**¹H-NMR (CDCl₃, 400 MHz) δ (ppm):** 8.47 (d, *J* = 8.5 Hz, 1H, Ar), 7.76 (s, 1H, Ar), 7.54 (d, *J* = 2.0 Hz, 1H, Ar), 7.28 - 7.04 (m, 6H, Ar), 6.87 (s, 1H, Ar), 4.52 (bs, 1H, NH), 4.04 (s, 3H, OCH₃), 3.16 - 2.97 (m, 6H, CH₂CH₂Ph, CH₂NHBoc), 2.77 (t, *J* = 7.5 Hz, 2H, TrizCH₂), 1.71 (quin, *J* = 7.6 Hz, 2H, TrizCH₂CH₂), 1.54 (quin, *J* = 7.5 Hz, 2H, CH₂CH₂NHBoc), 1.37 (s, 9H, OC(CH₃)₃); **¹³C-NMR (CDCl₃, 75 MHz) δ (ppm):** 160.5 (NHCON), 157.2 (OC_{Ar}), 156.2 (NHCOO), 156.0 (C_{Ar}), 148.9 (C_{Ar}), 141.7 (C_{Ar}), 138.3 (C_{Ar}), 129.6 (CH_{Ar}), 128.6 (CH_{Ar}), 128.5 (CH_{Ar}), 126.1 (CH_{Ar}), 122.7 (C_{Ar}), 119.1 (CH_{Ar}), 115.3 (C_{Ar}), 111.9 (CH_{Ar}), 103.9 (CH_{Ar}), 79.3 (OC(CH₃)₃), 56.2 (OCH₃), 40.4 (CH₂NHBoc), 35.7 (CH₂CH₂Ph), 33.5 (CH₂CH₂Ph), 29.7 (CH₂CH₂NHBoc), 28.6 (OC(CH₃)₃), 26.6 (TrizCH₂CH₂), 25.3 (TrizCH₂); **HRMS (ES, positive mode) m/z:** calculated for C₂₉H₃₅N₅O₃S 533.2461; Found 533.2469 (1.49 ppm)

### Benzyl (3-(1-(3-(2-(2-oxoimidazolidin-1-yl)ethoxy)-4-(4-phenethylthiazol-2-yl)phenyl)-1H-1,2,3-triazol-4-yl)propyl)carbamate (7o)

**¹H-NMR (CDCl₃, 400 MHz) δ (ppm):** 8.36 (d, *J* = 8.6 Hz, 1H, Ar), 7.85 (s, 1H, Ar), 7.46 (d, *J* = 2.0 Hz, 1H, Ar), 7.26 (dd, *J* = 8.6, 2.0 Hz, 1H, Ar), 7.24 - 6.97 (m, 10H, Ar), 6.75 (s, 1H, Ar), 5.30 (bs, 1H, NH), 4.93 (s, 2H, OCH₂Ph), 4.43 (bs, 1H, NH), 4.25 (t, *J* = 6.0 Hz, 2H, OCH₂), 3.56 (t, *J* = 6.0 Hz, 2H, OCH₂CH₂), 3.38 (t, *J* = 7.9 Hz, 2H, CH₂CH₂NHCON), 3.15 - 3.05 (m, 4H, CH₂CH₂NHCON, CH₂NHCbz), 3.01 - 2.82 (m, 4H, CH₂CH₂Ph), 2.69 (t, *J* = 7.1 Hz, 2H, TrizCH₂), 1.79 (quin, *J* = 6.9 Hz, 2H, TrizCH₂CH₂); **¹³C-NMR (CDCl₃, 100 MHz) δ (ppm):** 162.8 (NHCON), 160.3 (OC_{Ar}), 156.7 (C_{Ar}), 156.1 (C_{Ar}), 155.9 (C_{Ar}), 148.0 (C_{Ar}), 141.7 (C_{Ar}), 138.4 (C_{Ar}), 136.7 (C_{Ar}), 130.1 (CH_{Ar}), 128.7 (CH_{Ar}), 128.7 (CH_{Ar}), 128.6 (CH_{Ar}), 128.5 (C_{Ar}), 128.4 (CH_{Ar}), 128.3 (CH_{Ar}), 126.1 (CH_{Ar}), 122.7 (C_{Ar}), 119.6 (CH_{Ar}), 115.1 (CH_{Ar}), 112.4 (CH_{Ar}), 104.5 (CH_{Ar}), 67.6 (OCH₂), 66.9 (OCH₂Ph), 46.5 (CH₂CH₂NHCON), 42.8 (OCH₂CH₂), 40.0 (CH₂NHCbz), 38.4 (CH₂CH₂NHCON), 35.7 (CH₂CH₂Ph), 33.5 (CH₂CH₂Ph), 29.2 (TrizCH₂CH₂), 22.5 (TrizCH₂); **HRMS (ES, positive mode) m/z:** calculated for C₃₅H₃₇N₇O₄S 651.2628; Found 651.2623 (-0.74 ppm)

### Benzyl (E)-(3-(1-(4-(4-(2-([1,1'-biphenyl]-4-yl)vinyl)thiazol-2-yl)-3-(2-(2-oxoimidazolidin-1-yl)ethoxy)phenyl)-1H-1,2,3-triazol-4-yl)propyl)carbamate (7p)

**¹H-NMR (CDCl₃, 400 MHz) δ (ppm):** 8.59 (d, *J* = 8.6 Hz, 1H, Ar), 7.98 (s, 1H, Ar), 7.65 - 7.50 (m, 9H, Ar), 7.44 - 7.35 (m, 3H, Ar), 7.33 - 7.23 (m, 6H, Ar), 7.13 (d, *J* = 16.1 Hz, 1H, ThiazCH=CH), 5.41 (bs, 1H, NH), 5.04 (s, 2H, OCH₂Ph), 4.51 (bs, 1H, NH), 4.38 (t, *J* = 5.8 Hz, 2H, OCH₂), 3.69 (t, *J* = 6.1 Hz, 2H, OCH₂CH₂), 3.50 (t, *J* = 7.9 Hz, 2H, CH₂CH₂NHCON), 3.22 - 3.16 (m, 4H, CH₂CH₂NHCON, CH₂NHCbz), 2.81 (t, *J* = 7.1 Hz, 2H, TrizCH₂), 1.92 (quin, *J* = 7.3 Hz, 2H, TrizCH₂CH₂); **¹³C-NMR (CDCl₃, 100 MHz) δ (ppm):** 162.6 (NHCON), 161.0 (OC_{Ar}), 156.2 (C_{Ar}), 156.1 (C_{Ar}), 153.9 (C_{Ar}), 148.0 (C_{Ar}), 140.8 (C_{Ar}), 140.7 (C_{Ar}), 138.7 (C_{Ar}), 136.6 (C_{Ar}), 136.3 (C_{Ar}), 131.0 (CH_{Ar}), 130.4 (CH_{Ar}), 130.2 (CH_{Ar}), 128.9 (CH_{Ar}), 128.7 (CH_{Ar}), 128.4 (CH_{Ar}), 128.3 (CH_{Ar}), 127.5 (CH_{Ar}), 127.3 (CH_{Ar}), 127.1 (CH_{Ar}), 121.6 (CH_{Ar}), 119.7 (CH_{Ar}), 117.0 (CH_{Ar}), 112.5 (CH_{Ar}), 104.4 (CH_{Ar}), 67.5 (OCH₂), 66.9 (OCH₂Ph), 46.5 (CH₂CH₂NHCON), 42.8 (OCH₂CH₂), 40.0 (CH₂NHCbz), 38.4 (CH₂CH₂NHCON), 29.2 (TrizCH₂CH₂), 22.5 (TrizCH₂); **HRMS (ES, positive mode) m/z:** calculated for C₄₁H₃₉N₇O₄S 725.2784; Found 725.2797 (1.69 ppm)

### Benzyl (4-(1-(4-(4,5-diphenylthiazol-2-yl)-3-(2-(2-oxoimidazolidin-1-yl)ethoxy)phenyl)-1H-1,2,3-triazol-4-yl)butyl)carbamate (7q)

**¹H-NMR (CDCl₃, 400 MHz) δ (ppm):** 8.62 (d, *J* = 8.6 Hz, 1H, Ar), 7.89 (s, 1H, Ar), 7.64 - 7.57 (m, 3H, Ar), 7.44 - 7.23 (m, 14H, Ar), 5.19 (t, *J* = 6.0 Hz, 1H, NHCbz), 5.08 (s, 2H, OCH₂Ph), 4.87 (bs, 1H, NH), 4.44 (t, *J* = 5.9 Hz, 2H, OCH₂), 3.75 (t, *J* = 5.8 Hz, 2H, OCH₂CH₂), 3.57 (dd, *J* = 9.0, 6.7 Hz, 2H, CH₂CH₂NHCON), 3.29 (t, *J* = 8.0 Hz, 2H, CH₂CH₂NHCON), 3.25 (q, *J* = 6.9 Hz, 2H, CH₂NHCbz), 2.82 (t, *J* = 7.4 Hz, 2H, TrizCH₂), 1.75 (m, 2H, TrizCH₂CH₂), 1.62 (quin, *J* = 6.2 Hz, 2H, CH₂CH₂NHCbz); **¹³C-NMR (CDCl₃, 75 MHz) δ (ppm):** 162.2 (NHCON), 158.3 (OCAr), 156.6 (CAr), 155.9 (CAr), 149.3 (C_{Ar}), 148.7 (C_{Ar}), 138.4 (C_{Ar}), 136.7 (C_{Ar}), 135.1 (C_{Ar}), 134.3 (C_{Ar}), 132.2 (CH_{Ar}), 129.7 (CH_{Ar}), 129.6 (CH_{Ar}), 129.2 (CH_{Ar}), 129.1 (CH_{Ar}), 128.8 (CH_{Ar}), 128.6 (CH_{Ar}), 128.4 (CH_{Ar}), 128.2 (CH_{Ar}), 127.9 (C_{Ar}), 122.5 (CH_{Ar}), 119.1 (CH_{Ar}), 112.4 (CH_{Ar}), 104.4 (CH_{Ar}), 67.7 (OCH₂), 66.7 (OCH₂Ph), 46.4 (CH₂CH₂NHCON), 42.9 (OCH₂CH₂), 40.8 (CH₂NHCbz), 38.4 (CH₂CH₂NHCON), 29.4 (CH₂CH₂NHCbz), 26.3 (TrizCH₂CH₂), 25.2 (TrizCH₂); **HRMS (ES, positive mode) m/z:** calculated for C₄₀H₃₉N₇O₄S 713.2784; Found 713.2786 (0.23 ppm)

### G) Deprotection of the Boc group

*General procedure.* A solution of the corresponding Boc protected compounds **7a,b,d,n** (0.15mmol) in CH₂Cl₂ (20 mL) were treated with TFA (1 mL). After stirring at room temperature for 2 h, the excess of TFA was removed by coevaporation with CH₂Cl₂ and MeOH several times. The pure deprotected type **I** compounds **8a,b,d,n** were obtained as colorless oils in quantitative yields as 2,2,2 trifluoroacetate salts.

### H) Catalytic hydrogenation

*General procedure.* A solution of the corresponding Cbz protected compounds **7c,e-m,o-q** (0.15 mmol) in THF/MeOH 1:1 (30 mL) containing Pd/C (10%) (20% wt/wt) (20 mg) and TFA was hydrogenated (hydrogen balloon) at room temperature for 2 h. The reaction mixture was filtered (PTFE membrane filters) and the filtrate was evaporated to dryness under reduced pressure. The final residues were purified on a SP1 Isolera Biotage instrument using reverse phase columns to give compounds **8c,e-m,o-q** (colorless oils) in yields ranging from 13% to 55% as trifluoroacetate salts. Following a similar hydrogenation in the presence of HCl compound **8r** (idem substituents than **8e**) was obtained in quantitative yield as hydrochloride salt.

### 1-(2-(5-(4-(4-Aminobutyl)-1H-1,2,3-triazol-1-yl)-2-(4-isopropoxythiazol-2-yl)phenoxy)ethyl)imidazolidin-2-one 2,2,2 trifluoroacetate (Model compound A or 8a)

**¹H-NMR (DMSO-d₆, 400 MHz) δ (ppm):** 8.83 (s, 1H, Ar), 8.30 (d, *J* = 8.6 Hz, 1H, Ar), 7.75 (s, 1H, Ar), 7.65 (d, *J* = 8.3 Hz, 1H, Ar), 6.60 (s, 1H, Ar), 6.42 (bs, 1H, NH), 4.73 (quin, *J* = 6.1 Hz, 1H, OCH(CH₃)₂), 4.45 (m, 2H, OCH₂), 3.65 - 3.55 (m, 2H, OCH₂CH₂), 3.48 (t, *J* = 7.4 Hz, CH₂CH₂NHCON), 3.21 (t, *J* = 7.4 Hz, 2H, CH₂CH₂NHCON), 2.79 - 2.62 (m, 4H, TrizCH₂, CH₂NH₃⁺), 1.70 (m, 2H, TrizCH₂CH₂), 1.58 (m, 2H, CH₂CH₂NH₃⁺), 1.31 (d, *J* = 6.1 Hz, 6H, CH₃); **¹³C-NMR (DMSO-d₆, 100 MHz) δ (ppm):** 162.1 (NHCON), 156.7 (OC_{Ar}), 155.9 (OC_{Ar}), 148.0 (C_{Ar}), 137.9 (C_{Ar}), 128.5 (CH_{Ar}), 120.8 (CH_{Ar}), 120.5 (C_{Ar}), 111.9 (CH_{Ar}), 104.2 (CH_{Ar}), 93.0 (CH_{Ar}), 71.8 (OCH₂), 67.5 (OCH(CH₃)₂), 45.3 (CH₂CH₂NHCON), 42.4 (OCH₂CH₂), 37.6 (CH₂CH₂NHCON), 28.6 (CH₂CH₂NH₃⁺), 25.8 (TrizCH₂CH₂), 24.6 (TrizCH₂), 21.9 (CH₃); **HPLC:** Gradient 1, tᵣ = 4.3 min; **HRMS (ES, positive mode) m/z:** calculated for C₂₃H₃₁N₇O₃S 485.2203; Found 485.2209 (-1.19 ppm); **Elemental analysis:** calculated for C₂₅H₃₂F₃N₇O₅S: C. 50.08; H. 5.38; N. 16.35; S. 5.35; Found: C. 50.15; H. 5.16; N. 16.05; S. 5.65

### 1-(2-(5-(4-(4-Aminobutyl)-1H-1,2,3-triazol-1-yl)-2-(4-neopentylthiazol-2-yl)phenoxy)ethyl)imidazolidin-2-one 2,2,2 trifluoroacetate (8b)

**¹H-NMR (CD₃OD, 400 MHz) δ (ppm):** 8.46 (s, 1H, Ar), 8.46 (d, *J* = 8.6 Hz, 1H, Ar), 7.73 (d, *J* = 2.1 Hz, 1H, Ar), 7.59 (dd, *J* = 8.6, 2.1 Hz, 1H, Ar), 7.22 (s, 1H, Ar), 4.50 (t, *J* = 5.5 Hz, 2H, OCH₂), 3.77 (t, *J* = 5.5 Hz, 2H, OCH₂CH₂), 3.62 (dd, *J* = 9.2, 6.9 Hz, 2H, CH₂CH₂NHCON), 3.38 (dd, *J* = 9.3, 6.9 Hz, 2H, CH₂CH₂NHCON), 2.83 (t, *J* = 7.5 Hz, 2H, CH₂NH₃⁺), 2.75 (s, 2H, CH₂-^{t}Bu), 2.70 (t, *J* = 7.2 Hz, 2H, TrizCH₂), 1.79 (quin, *J* = 7.6 Hz, 2H, TrizCH₂CH₂), 1.58 (quin, *J* = 7.6 Hz, 2H, CH₂CH₂NH₃⁺), 1.00 (s, 9H, CH₃); **¹³C-NMR (CD₃OD, 75 MHz) δ (ppm):** 165.1 (NHCON), 161.1 (OCAr), 157.4 (C_{Ar}), 155.8 (C_{Ar}), 150.1 (C_{Ar}), 139.7 (C_{Ar}), 130.8 (CH_{Ar}), 123.9 (C_{Ar}), 121.5 (CH_{Ar}), 118.4 (CH_{Ar}), 113.6 (CH_{Ar}), 105.7 (CH_{Ar}), 68.4 (OCH₂), 46.9 (CH₂CH₂NHCON), 45.7 (CH₂-^{t}Bu), 43.9 (OCH₂CH₂), 42.2 (CH₂NH₃⁺), 39.3 (CH₂CH₂NHCON), 33.2 (CH₂CH₂NH₃⁺), 32.4 (CH₂C(CH₃)₃), 30.0 (CH₂C(CH₃)₃), 27.7 (TrizCH₂CH₂), 26.1 (TrizCH₂); **HPLC:** Gradient 1, tᵣ = 4.3 min; **HRMS (ES, positive mode) m/z:** calculated for C₂₅H₃₅N₇O₂S 497.2573; Found 497.2572 (-0.17 ppm); **Elemental analysis:** calculated for C₂₇H₃₆F₃N₇O₄S: C. 53.02; H. 5.93; N. 16.03; S. 5.24; Found: C. 52.97; H. 6.17; N. 16.29; S. 5.55

### 1-(2-(5-(4-(4-aminobutyl)-1H-1,2,3-triazol-1-yl)-2-(4-phenylthiazol-2-yl)phenoxy)ethyl)imidazolidin-2-one 2,2,2 trifluoroacetate (8c)

**¹H-NMR (CD₃OD, 400 MHz) δ (ppm):** 8.64 (d, *J* = 8.6 Hz, 1H, Ar), 8.46 (s, 1H, Ar), 8.54 (dd, *J* = 8.2, 1.3 Hz, 2H, Ar), 7.81 (s, 1H, Ar), 7.71 (d, *J* = 2.0 Hz, 1H, Ar), 7.61 (dd, *J* = 8.6, 2.0 Hz, 1H, Ar), 7.45 (dd, *J* = 8.3, 7.0 Hz, 2H, Ar), 7.36 (d, *J* = 7.4 Hz, 1H, Ar), 4.51 (t, *J* = 5.5 Hz, 2H, OCH₂), 3.78 (t, *J =* 5.5 Hz, 2H, OCH₂CH₂), 3.64 (dd, *J* = 9.3, 6.9 Hz, 2H, CH₂CH₂NHCON), 3.39 (dd, *J* = 9.2, 6.9 Hz, 2H, CH₂CH₂NHCON), 3.03 (t, *J* = 7.4 Hz, 2H, CH₂NH₃⁺), 2.87 (t, *J* = 7.2 Hz, 2H, TrizCH₂), 1.87 (m, 2H, TrizCH₂CH₂), 1.78 (m, 2H, CH₂CH₂NH₃⁺); **¹³C-NMR (CD₃OD, 100 MHz) δ (ppm):** 165.1 (NHCON), 162.0 (OC_{Ar}), 157.5 (C_{Ar}), 155.8 (C_{Ar}), 149.3 (C_{Ar}), 139.7 (C_{Ar}), 135.9 (C_{Ar}) 131.0 (CH_{Ar}), 129.8 (CH_{Ar}), 129.2 (CH_{Ar}), 127.4 (CH_{Ar}), 123.9 (C_{Ar}), 121.6 (CH_{Ar}), 116.0 (CH_{Ar}), 113.6 (CH_{Ar}), 105.6 (CH_{Ar}), 68.4 (OCH₂), 47.0 (CH₂CH₂NHCON), 43.9 (OCH₂CH₂), 40.4 (CH₂NH₃⁺), 39.3 (CH₂CH₂NHCON), 28.0 (CH₂CH₂NH₃⁺), 27.1 (TrizCH₂CH₂), 25.6 (TrizCH₂); **HPLC:** Gradient 1, tᵣ = 7.7 min; **HRMS (ES, positive mode) m/z:** calculated for C₂₆H₂₉N₇O₂S 503.2103; Found 503.2100 (-0.66 ppm); **Elemental analysis:** calculated for C₂₈H₃₀F₃N₇O₄S: C. 54.45; H. 4.90; N. 15.87; S. 5.19; Found: C. 54.66; H. 4.48; N. 15.90; S. 5.15

### 1-(2-(5-(4-(4-Aminobutyl)-1H-1,2,3-triazol-1-yl)-2-(4-benzylthiazol-2-yl)phenoxy)ethyl)imidazolidin-2-one 2,2,2 trifluoroacetate (8d)

**¹H-NMR (DMSO-d₆, 300 MHz) δ (ppm):** 8.73 (s, 1H, Ar), 8.39 (d, *J* = 8.6 Hz, 1H, Ar), 7.74 (s, 1H, Ar), 7.66 (d, *J* = 8.4 Hz, 1H, Ar), 7.55 - 7.05 (m, 5H, Ar), 7.19 (s, 1H, Ar), 6.43 (bs, 1H, NH), 4.46 (t, *J* = 5.4 Hz, 2H, OCH₂), 4.14 (s, 2H, CH₂Ph), 3.62 (t, *J* = 5.4 Hz, 2H, OCH₂CH₂), 3.48 (m, 2H, CH₂CH₂NHCON), 3.20 (m, 2H, CH₂CH₂NHCON), 2.94 (m, 2H, CH₂NH₃⁺), 2.72 (m, 2H, TrizCH₂), 1.67 (m, 2H, TrizCH₂CH₂), 1.48 - 1.37 (m, 2H, CH₂CH₂NH₃⁺); **¹³C-NMR (DMSO-d₆, 75 MHz) δ (ppm):** 162.1 (NHCON), 155.6 (C_{Ar}), 155.0 (C_{Ar}), 148.3 (C_{Ar}), 139.6 (C_{Ar}), 138.1 (C_{Ar}), 128.9 (CH_{Ar}), 128.8 (CH_{Ar}), 128.4 (CH_{Ar}), 126.1 (CH_{Ar}), 121.1 (C_{Ar}), 120.0 (CH_{Ar}), 116.7 (CH_{Ar}), 112.0 (CH_{Ar}), 104.3 (CH_{Ar}), 67.6 (OCH₂), 45.2 (CH₂CH₂NHCON), 42.3 (OCH₂CH₂), 40.4 (CH₂NH₃⁺), 37.5 (CH₂CH₂NHCON), 37.0 (CH₂Ph), 30.7 (CH₂CH₂NH₃⁺), 29.7 (TrizCH₂CH₂), 26.1 (TrizCH₂); **HPLC:** Gradient 1, tᵣ = 4.4 min; **HRMS (ES, positive mode) m/z:** calculated for C₂₇H₃₁N₇O₂S 517.2260; Found 517.2256 (-0.69 ppm); **Elemental analysis:** calculated for C₂₉H₃₂F₃N₇O₄S: C. 55.14; H. 5.11; N. 15.52; S. 5.08; Found: C. 55.02; H. 5.15; N. 15.63; S. 5.16

### 1-(2-(5-(4-(4-Aminobutyl)-1H-1,2,3-triazol-1-yl)-2-(4-phenethylthiazol-2-yl)phenoxy)ethyl)imidazolidin-2-one 2,2,2 trifluoroacetate (8e)

**¹H-NMR (CD₃OD, 400 MHz) δ (ppm):** 8.47 (s, 1H, Ar), 8.46 (d, *J* = 8.5 Hz, 1H, Ar), 7.70 (d, *J* = 2.0 Hz, 1H, Ar), 7.58 (dd, *J* = 8.6, 2.0 Hz, 1H, Ar), 7.33 - 7.17 (m, 5H, Ar), 7.15 (s, 1H, Ar), 4.48 (t, *J* = 5.5 Hz, 2H, OCH₂), 3.74 (t, *J* = 5.5 Hz, 2H, OCH₂CH₂), 3.60 (dd, *J* = 9.3, 6.9 Hz, 2H, CH₂CH₂NHCON), 3.36 (dd, *J* = 9.3, 6.9 Hz, 2H, CH₂CH₂NHCON), 3.15 - 3.03 (m, 4H, CH₂CH₂Ph), 3.00 (t, *J* = 7.4 Hz, 2H, CH₂NH₃⁺), 2.87 (t, *J* = 7.2 Hz, 2H, TrizCH₂), 1.85 (m, 2H, TrizCH₂CH₂), 1.76 (m, 2H, CH₂CH₂NH₃⁺); **¹³C-NMR (CD₃OD, 100 MHz) δ (ppm):** 165.1 (NHCON), 162.0 (OC_{Ar}), 157.4 (C_{Ar}), 157.1 (C_{Ar}), 149.3 (C_{Ar}), 142.8 (C_{Ar}), 139.7 (C_{Ar}), 130.8 (CH_{Ar}), 129.5 (CH_{Ar}), 129.4 (CH_{Ar}), 127.0 (CH_{Ar}), 123.8 (C_{Ar}), 121.6 (CH_{Ar}), 116.9 (CH_{Ar}), 113.6 (CH_{Ar}), 105.7 (CH_{Ar}), 68.4 (OCH₂), 47.0 (CH₂CH₂NHCON), 43.9 (OCH₂CH₂), 40.4 (CH₂NH₃⁺), 39.3 (CH₂CH₂NHCON), 36.7 (CH₂CH₂Ph), 34.3 (CH₂CH₂Ph), 28.0 (CH₂CH₂NH₃⁺), 27.1 (TrizCH₂CH₂), 25.6 (TrizCH₂); **HPLC:** Gradient 1, tᵣ = 4.5 min; **HRMS (ES, positive mode) m/z:** calculated for C₂₈H₃₃N₇O₂S 531.2416; Found 531.2424 (1.35 ppm); **Elemental analysis:** calculated for C₃₀H₃₄F₃N₇O₄S: C. 55.80; H. 5.31; N. 15.18; S. 4.97; Found: C. 55.94; H. 5.50; N. 15.08; S. 4.51

### 1-(2-(5-(4-(4-Aminobutyl)-1H-1,2,3-triazol-1-yl)-2-(4-(3-phenylpropyl)thiazol-2-yl)phenoxy)ethyl)imidazolidin-2-one 2,2,2 trifluoroacetate (8f)

**¹H-NMR (CD₃OD, 400 MHz) δ (ppm):** 8.50 (s, 1H, Ar), 8.48 (d, *J* = 8.5 Hz, 1H, Ar), 7.71 (d, *J* = 2.1 Hz, 1H, Ar), 7.58 (dd, *J* = 8.6, 2.0 Hz, 1H, Ar), 7.38 - 7.06 (m, 6H, Ar), 4.49 (t, *J* = 5.6 Hz, 2H, OCH₂), 3.75 (t, *J* = 5.5 Hz, 2H, OCH₂CH₂), 3.61 (dd, *J* = 9.3, 6.9 Hz, 2H, CH₂CH₂NHCON), 3.37 (dd, *J* = 9.3, 6.9 Hz, 2H, CH₂CH₂NHCON), 3.00 (t, *J* = 7.5 Hz, 2H, CH₂NH₃⁺), 2.87 (q, *J* = 7.0 Hz, 4H, ThiazCH₂, CH₂Ph), 2.71 (t, *J* = 7.6 Hz, 2H, TrizCH₂), 2.09 (quin, *J* = 7.6 Hz, 2H, ThiazCH₂CH₂), 1.92 (m, 2H, TrizCH₂CH₂), 1.75 (m, 2H, CH₂CH₂NH₃⁺); **¹³C-NMR (CD₃OD, 75 MHz) δ (ppm):** 165.1 (NHCON), 162.0 (OC_{Ar}), 157.8 (C_{Ar}), 157.5 (C_{Ar}), 149.3 (C_{Ar}), 143.4 (C_{Ar}), 139.6 (C_{Ar}), 130.8 (CH_{Ar}), 129.5 (CH_{Ar}), 129.4 (CH_{Ar}), 126.8 (CH_{Ar}), 123.9 (C_{Ar}), 121.6 (CH_{Ar}), 116.6 (CH_{Ar}), 113.6 (CH_{Ar}), 105.7 (CH_{Ar}), 68.5 (OCH₂), 46.9 (CH₂CH₂NHCON), 43.9 (OCH₂CH₂), 40.4 (CH₂NH₃⁺), 39.3 (CH₂CH₂NHCON), 36.4 (CH₂CH₂CH₂Ph), 32.4 (CH₂CH₂CH₂Ph), 31.7 (CH₂CH₂CH₂Ph), 28.0 (CH₂CH₂NH₃⁺), 27.1 (TrizCH₂CH₂), 25.6 (TrizCH₂); **HPLC:** Gradient 1, tᵣ = 7.7 min; **HRMS (ES, positive mode) m/z:** calculated for C₂₉H₃₅N₇O₂S 545.2573; Found 545.2573 (0.03 ppm); **Elemental analysis:** calculated for C₃₁H₃₆F₃N₇O₄S: C. 56.44; H. 5.50; N. 14.86; S. 4.86; Found: C. 56.04; H. 5.66; N. 14.59; S. 4.48

### 1-(2-(5-(4-(4-Aminobutyl)-1H-1,2,3-triazol-1-yl)-2-(4-(2-(naphthalen-2-yl)ethyl)thiazol-2-yl)phenoxy)ethyl)imidazolidin-2-one 2,2,2 trifluoroacetate (8g)

**¹H-NMR (DMSO-d₆, 400 MHz) δ (ppm):** 8.77 (s, 1H, Ar), 8.49 (d, *J* = 8.6 Hz, 1H, Ar), 7.89 - 7.66 (m, 8H, Ar, NH₃⁺), 7.52 - 7.34 (m, 3H, Ar), 7.34 (s, 1H, Ar), 4.47 (t, *J* = 5.7 Hz, 2H, OCH₂), 3.63 (t, *J* = 5.6 Hz, 2H, OCH₂CH₂), 3.48 (dd, J = 8.9 Hz, *J* = 6.7 Hz, 2H, CH₂CH₂NHCON), 3.33 - 3.04 (m, 6H, CH₂CH₂NHCON, CH₂NH₃⁺, CH₂CH₂Napth), 2.90 (m, 2H CH₂CH₂Napth), 2.77 (t, *J* = 7.2 Hz, 2H, TrizCH₂), 1.77 (m, 2H, TrizCH₂CH₂), 1.62 (m, 2H, CH₂CH₂NH₃⁺); **¹³C-NMR (DMSO-d₆, 75 MHz) δ (ppm):** 162.1 (NHCON), 159.4 (OC_{Ar}), 155.6 (C_{Ar}), 155.3 (C_{Ar}), 147.7 (C_{Ar}), 139.0 (C_{Ar}), 137.9 (C_{Ar}), 133.2 (CH_{Ar}), 131.6 (C_{Ar}), 129.1 (CH_{Ar}), 127.7 (CH_{Ar}), 127.4 (CH_{Ar}), 127.3 (C_{Ar}), 126.2 (CH_{Ar}), 125.9 (CH_{Ar}), 125.2 (CH_{Ar}), 121.4 (CH_{Ar}), 120.4 (CH_{Ar}), 116.1 (CH_{Ar}), 112.0 (CH_{Ar}), 104.3 (CH_{Ar}), 67.6 (OCH₂), 45.3 (CH₂CH₂NHCON), 42.4 (OCH₂CH₂), 37.5 (CH₂CH₂NHCON), 34.9 (CH₂CH₂Napth), 32.5 (CH₂CH₂Napth), 26.5 (CH₂CH₂NH₃⁺), 25.7 (TrizCH₂CH₂), 24.8 (TrizCH₂); **HPLC:** Gradient 1, tᵣ = 7.77 min; **HRMS (ES, positive mode) m/z:** calculated for C₃₂H₃₅N₇O₂S 581.2573; Found 581.2572 (-0.13 ppm); **Elemental analysis:** calculated for C₃₄H₃₆F₃N₇O₄S: C. 58.69; H. 5.22; N. 14.09; S. 4.61; Found: C. 58.20; H. 5.01; N. 14.09; S. 4.12

### 1-(2-(2-(4-(2-([1,1'-Biphenyl]-4-yl)ethyl)thiazol-2-yl)-5-(4-(4-aminobutyl)-1H-1,2,3-triazol-1-yl)phenoxy)ethyl)imidazolidin-2-one 2,2,2 trifluoroacetate (8h)

**¹H-NMR (DMSO-d₆, 500 MHz) δ (ppm):** 8.77 (s, 1H, Ar), 8.47 (d, *J* = 8.6 Hz, 1H, Ar), 7.75 (d, *J* = 2.1 Hz, 1H, Ar), 7.69 (dd, *J* = 8.6, 2.1 Hz, 1H, Ar), 7.66 - 7.62 (m, 2H, Ar), 7.60 - 7.56 (m, 2H, Ar), 7.48 - 7.39 (m, 3H, Ar), 7.38 - 7.29 (m, 3H, Ar), 6.41 (bs, 1H, NH), 4.47 (t, *J* = 5.7 Hz, 2H, OCH₂), 3.63 (t, *J* = 5.7 Hz, 2H, OCH₂CH₂), 3.49 (dd, *J* = 9.0, 6.7 Hz, 2H, CH₂CH₂NHCON), 3.21 (dd, *J* = 9.3, 6.5 Hz, 2H, CH₂CH₂NHCON), 3.17 - 2.96 (m, 4H, CH₂NH₃⁺, CH₂CH₂BiPh), 2.74 (t, *J* = 7.4, 2H, TrizCH₂), 2.69 (t, *J* = 7.2 Hz, 2H, CH₂CH₂BiPh), 1.72 (quin, *J* = 7.6 Hz, 2H, TrizCH₂CH₂), 1.50 (m, 2H, CH₂CH₂NH₃⁺); **¹³C-NMR (DMSO-d₆, 100 MHz) δ (ppm):** 162.1 (NHCON), 159.4 (OC_{Ar}), 155.6 (C_{Ar}), 155.3 (C_{Ar}), 148.1 (C_{Ar}), 140.7 (C_{Ar}), 140.0 (C_{Ar}), 138.0 (C_{Ar}), 137.8 (C_{Ar}), 129.0 (CH_{Ar}), 128.9 (CH_{Ar}), 128.9 (CH_{Ar}), 127.2 (CH_{Ar}), 126.5 (CH_{Ar}), 126.5 (CH_{Ar}), 121.3 (C_{Ar}), 120.3 (CH_{Ar}), 116.0 (CH_{Ar}), 112.0 (CH_{Ar}), 104.3 (CH_{Ar}), 67.6 (OCH₂), 45.4 (CH₂CH₂NHCON), 42.4 (OCH₂CH₂), 37.7 (CH₂NH₃⁺), 37.5 (CH₂CH₂NHCON), 34.3 (CH₂CH₂BiPh), 32.5 (CH₂CH₂BiPh), 30.1 (CH₂CH₂NH₃⁺), 25.9 (TrizCH₂CH₂), 24.7 (TrizCH₂); **HPLC:** Gradient 1, tᵣ = 8.4 min; **HRMS (ES, positive mode) m/z:** calculated for C₃₄H₃₇N₇O₂S 607.2729; Found 607.2740 (1.73 ppm); **Elemental analysis:** calculated for C₃₆H₃₈F₃N₇O₄S: C. 59.91; H. 5.31; N. 13.58; S. 4.44; Found: C. 60.08; H. 5.09; N. 13.18; S. 4.20

### 1-(2-(5-(4-(4-Aminobutyl)-1H-1,2,3-triazol-1-yl)-2-(4-(2-(3,4-dihydro-2H-1λ²-quinolin-5-yl)ethyl)thiazol-2-yl)phenoxy)ethyl)imidazolidin-2-one 2,2,2 trifluoroacetate (8i)

**¹H-NMR (CD₃OD, 400 MHz) δ (ppm):** 8.47 (s, 1H, Ar), 8.46 (d, *J* = 8.9 Hz, 1H, Ar), 7.71 (d, *J* = 2.1 Hz, 1H, Ar), 7.59 (dd, *J* = 8.6, 2.0 Hz, 1H, Ar), 7.15 (s, 1H, Ar), 6.93 - 6.82 (m, 2H, Ar), 6.63 (d, *J* = 8.7 Hz, 1H, Ar), 4.49 (t, *J* = 5.5 Hz, 2H, OCH₂), 3.75 (t, *J* = 5.5 Hz, 2H, OCH₂CH₂), 3.61 (dd, *J* = 9.3, 6.9 Hz, 2H, CH₂CH₂NHCON), 3.37 (dd, *J* = 9.3, 6.9 Hz, 2H, CH₂CH₂NHCON), 3.27 (t, *J* = 5.5 Hz, 2H, PhNHCH₂), 3.08 (t, *J* = 7.5 Hz, 2H, ThiazCH₂CH₂), 3.03 - 2.92 (m, 4H, ThiazCH₂CH₂, CH₂NH₃⁺), 2.88 (t, *J* = 7.2 Hz, 2H, TrizCH₂), 2.75 ( *J* = 6.5 Hz, 2H, PhNHCH₂CH₂CH₂), 1.95 (quin, *J* = 6.3 Hz, 2H, PhNHCH₂CH₂), 1.89 - 1.72 (m, 4H, TrizCH₂CH₂, CH₂CH₂NH₃⁺); **¹³C-NMR (CD₃OD, 100 MHz) δ (ppm):** 165.1 (NHCON), 161.0 (OC_{Ar}), 157.5 (C_{Ar}), 157.3 (C_{Ar}), 149.3 (C_{Ar}), 141.5 (C_{Ar}), 139.7 (C_{Ar}), 134.9 (C_{Ar}), 130.8 (CH_{Ar}), 130.8 (CH_{Ar}), 128.0 (CH_{Ar}), 125.6 (C_{Ar}), 123.8 (C_{Ar}), 121.6 (CH_{Ar}), 118.2 (CH_{Ar}), 116.9 (CH_{Ar}), 113.6 (CH_{Ar}), 105.7 (CH_{Ar}), 68.4 (OCH₂), 46.9 (CH₂CH₂NHCON), 43.8 (OCH₂CH₂), 43.8 (PhNHCH₂), 40.4 (CH₂NH₃⁺), 39.3 (CH₂CH₂NHCON), 36.0 (ThiazCH₂CH₂), 34.5 (ThiazCH₂CH₂), 28.0 (CH₂CH₂NH₃⁺), 27.5 (PhNHCH₂CH₂CH₂), 27.1 (TrizCH₂CH₂), 25.6 (TrizCH₂), 22.8 (PhNHCH₂CH₂); **HRMS (ES, positive mode) m/z:** calculated for C₃₁H₃₈N₈O₂S 586.2838; Found 586.2859 (3.60 ppm); **Elemental analysis:** calculated for C₃₅H₄₀F₆N₈O₆S: C. 51.59; H. 4.95; N. 13.75; S. 3.93; Found: C. 51.11; H. 4.86; N. 13.26; S. 3.56

### 1-(2-(5-(4-(4-Aminobutyl)-1H-1,2,3-triazol-1-yl)-2-(4-(2-(2,3-dihydrobenzofuran-6-yl)ethyl)thiazol-2-yl)phenoxy)ethyl)imidazolidin-2-one 2,2,2 trifluoroacetate (8j)

**¹H-NMR (D₂O, 400 MHz, 90 °C) δ (ppm):** 8.58 (d, *J* = 8.5 Hz, 1H, Ar), 8.55 (s, 1H, Ar), 7.84 (d, *J* = 2.0 Hz, 1H, Ar), 7.67 (dd, *J* = 8.6, 2.0 Hz, 1H, Ar), 7.35 (d, *J* = 1.9 Hz, 1H, Ar), 7.30 (s, 1H, Ar), 7.25 (dd, *J* = 8.1, 1.9 Hz, 1H, Ar), 7.06 (d, *J* = 8.0 Hz, 1H, Ar), 4.40 (t, *J* = 5.5 Hz, 2H, OCH₂CH₂N), 4.17 (t, *J* = 8.7 Hz, 2H, OCH₂CH₂Ph), 4.06 (t, *J* = 5.5 Hz, 2H, OCH₂CH₂N), 3.92 (dd, *J* = 9.7, 6.8 Hz, 2H, CH₂CH₂NHCON), 3.78 (dd, *J* = 9.5, 6.8 Hz, 2H, CH₂CH₂NHCON), 3.67 (t, *J* = 8.7 Hz, OCH₂CH₂Ph), 3.48 - 3.21 (m, 8H, ThiazCH₂CH₂, CH₂NH₃⁺, TrizCH₂), 2.30 (m, 2H, TrizCH₂CH₂, CH₂CH₂NH₃⁺); **¹³C-NMR (D₂O, 100 MHz, 90 °C) δ (ppm):** 164.5 (NHCON), 160.7 (OC_{Ar}), 158.2 (OC_{Ar}), 156.3 (C_{Ar}), 156.1 (C_{Ar}), 149.1 (C_{Ar}), 138.1 (C_{Ar}), 134.2 (C_{Ar}), 129.7 (CH_{Ar}), 127.9 (CH_{Ar}), 127.8 (CH_{Ar}), 125.3 (CH_{Ar}), 122.7 (CH_{Ar}), 120.8 (CH_{Ar}), 115.7 (C_{Ar}), 112.9 (C_{Ar}), 109.1 (CH_{Ar}), 105.2 (CH_{Ar}), 71.7 (OCH₂CH₂Ph), 68.4 (OCH₂CH₂N), 46.3 (CH₂CH₂NHCON), 43.2 (OCH₂CH₂N), 39.9 (CH₂NH₃⁺), 38.4 (CH₂CH₂NHCON), 34.7 (ThiazCH₂CH₂), 33.3 (ThiazCH₂CH₂), 29.8 (OCH₂CH₂Ph), 27.0 (CH₂CH₂NH₃⁺), 25.9 (TrizCH₂CH₂), 24.7 (TrizCH₂); **HPLC:** Gradient 1, tᵣ = 7.2 min; **HRMS (ES, positive mode) m/z:** calculated for C₃₀H₃₅N₇O₃S 573.2522; Found 573.2520 (-0.43 ppm); **Elemental analysis:** calculated for C₃₂H₃₆F₃N₇O₄S: C. 55.89; H. 5.28; N. 14.26; S. 4.66; Found: C. 54.96; H. 5.72; N. 13.67; S. 4.89

### 1-(2-(5-(4-(4-aminobutyl)-1H-1,2,3-triazol-1-yl)-2-(4-(2-(dibenzo[b,d]furan-1-yl)ethyl)thiazol-2-yl)phenoxy)ethyl)imidazolidin-2-one 2,2,2 trifluoroacetate (8k)

**¹H-NMR (DMSO-d₆, 400 MHz) δ (ppm):** 8.66 (s, 1H, Ar), 8.48 (d, *J* = 8.6 Hz, 1H, Ar), 8.09 (dd, *J* = 7.7, 1.4 Hz, 1H, Ar), 8.03 (d, *J* = 1.8 Hz, 1H, Ar), 7.80 - 7.30 (m, 11H, Ar, NH₃⁺), 6.18 (bs, 1H, NH), 4.48 (t, *J* = 5.8 Hz, 2H, OCH₂), 3.64 (t, *J* = 5.7 Hz, 2H, OCH₂CH₂), 3.49 (dd, *J* = 8.9, 6.8 Hz, 2H, CH₂CH₂NHCON), 3.27 - 3.20 (m, 4H, ThiazCH₂CH₂), 2.87 (t, *J* = 7.4 Hz, 2H, CH₂NH₃⁺), 2.79 (t, *J* = 7.2 Hz, 2H, TrizCH₂), 1.78 (quin, *J* = 7.4 Hz, 2H, TrizCH₂CH₂), 1.68 (quin, *J* = 7.6 Hz, 2H, CH₂CH₂NH₃⁺); **¹³C-NMR (CDCl₃, 75 MHz) δ (ppm):** 162.1 (NHCON), 159.4 (OC_{Ar}), 155.7 (OC_{Ar}), 155.6 (OC_{Ar}), 155.3, (NHCOO), 154.0 (C_{Ar}), 147.7 (C_{Ar}), 137.9 (C_{Ar}), 136.3 (C_{Ar}), 129.1 (CH_{Ar}), 128.0 (CH_{Ar}), 127.4 (CH_{Ar}), 123.5 (C_{Ar}), 123.0 (CH_{Ar}), 121.4 (C_{Ar}), 121.0 (CH_{Ar}), 120.5 (CH_{Ar}), 120.4 (CH_{Ar}), 116.1 (CH_{Ar}), 112.0 (CH_{Ar}), 111.6 (CH_{Ar}), 111.2 (CH_{Ar}), 104.3 (CH_{Ar}), 67.6 (OCH₂), 45.3 (CH₂CH₂NHCON), 42.4 (OCH₂CH₂), 38.7 (CH₂NH₃⁺), 37.5 (CH₂CH₂NHCON), 34.7 (ThiazCH₂CH₂), 33.2 (ThiazCH₂CH₂), 26.5 (CH₂CH₂NH₃⁺), 25.5 (TrizCH₂CH₂), 24.4 (TrizCH₂); **HRMS (ES, positive mode) m/z:** calculated for C₃₄H₃₅N₇O₃S 621.2522; Found 621.2517 (-0.78 ppm); **Elemental analysis:** calculated for C₃₆H₃₆F₃N₇O₅S: C. 58.77; H. 4.93; N. 13.33; S. 4.36; Found: C. 59.15; H. 5.09; N. 13.46; S. 4.21

### 4-(1-(4-(4-phenethylthiazol-2-yl)-3-(2-(piperidin-4-yl)ethoxy)phenyl)-1H-1,2,3-triazol-4-yl)butan-1-amine 2,2,2 trifluoroacetate (8I)

**¹H-NMR (CD₃OD, 400 MHz) δ (ppm):** 8.49 (s, 1H, Ar), 8.47 (d, *J* = 8.2 Hz, 1H, Ar), 7.73 (d, *J* = 2.1 Hz, 1H, Ar), 7.56 (dd, *J* = 8.6, 2.1 Hz, 1H, Ar), 7.33 - 7.18 (m, 5H, Ar), 7.16 (s, 1H, Ar), 4.43 (t, *J* = 5.8 Hz, 2H, OCH₂), 3.41 (d, *J* = 12.9 Hz, 2H, H'HC(NH₂⁺)CHH'), 3.22 - 3.04 (m, 4H, CH₂CH₂Ph), 3.04 - 2.94 (m, 4H, H'HC(NH₂⁺)CHH', CH₂NH₃⁺), 2.88 (t, *J* = 7.3 Hz, 2H, TrizCH₂), 2.10 - 2.02 (m, 5H, CH, OCH₂CH₂, CH(CHH')₂), 1.90 (m, 2H, TrizCH₂CH₂), 1.79 - 1.73 (m, 2H, CH₂CH₂NH₃⁺), 1.52 (m, 2H, CH(CHH')₂); **¹³C-NMR (CD₃OD, 100 MHz) δ (ppm):** 162.0 (OC_{Ar}), 157.8 (C_{Ar}), 157.1 (C_{Ar}), 149.4 (C_{Ar}), 139.7 (C_{Ar}), 130.7 (CH_{Ar}), 129.5 (CH_{Ar}), 129.4 (CH_{Ar}), 127.0 (CH_{Ar}), 123.6 (C_{Ar}), 121.7 (CH_{Ar}), 116.8 (CH_{Ar}), 113.3 (CH_{Ar}), 105.5 (CH_{Ar}), 68.1 (OCH₂), 45.3 (CH₂(NH₂⁺)CH₂), 40.4 (CH₂NH₃⁺), 36.7 (CH₂CH₂Ph), 36.1 (OCH₂CH₂), 34.3 (CH₂CH₂Ph), 32.3 (CH), 29.9 (CH(CH₂)₂), 28.0 (CH₂CH₂NH₃⁺), 27.1 (TrizCH₂CH₂), 25.6 (TrizCH₂); **HPLC:** Gradient 1, tᵣ = 6.9 min; **HRMS (ES, positive mode) m/z:** calculated for C₃₀H₃₈N₆OS 530.2829; Found 530.2846 (3.44 ppm); **Elemental analysis:** calculated for C₃₄H₄₀F₆N₆O₅S: C. 53.82; H. 5.31; N. 11.08; S. 4.23; Found: C. 54.05; H. 5.04; N. 11.32; S. 4.11

### 4-(1-(3-Methoxy-4-(4-phenethylthiazol-2-yl)phenyl)-1H-1,2,3-triazol-4-yl)butan-1-amine 2,2,2 trifluoroacetate (8m)

**¹H-NMR (CD₃OD, 400 MHz) δ (ppm):** 8.45 (s, 1H, Ar), 8.45 (d, *J* = 8.3 Hz, 1H, Ar), 7.67 (d, *J* = 2.1 Hz, 1H, Ar), 7.55 (dd, *J* = 8.6, 2.1 Hz, 1H, Ar), 7.36 - 7.16 (m, 5H, Ar), 7.15 (s, 1H, Ar), 4.12 (s, 3H, OCH₃), 3.20 - 3.02 (m, 4H, CH₂CH₂Ph), 3.00 (t, *J* = 7.5 Hz, 2H, CH₂NH₃⁺), 2.87 (t, *J* = 7.3 Hz, 2H, TrizCH₂), 1.90 (m, 2H, TrizCH₂CH₂), 1.80 (m, 2H, CH₂CH₂NH₃⁺); **¹³C-NMR (CD₃OD, 100 MHz) δ (ppm):** 162.1 (C_{Ar}), 158.6 (C_{Ar}), 156.8 (C_{Ar}), 149.4 (C_{Ar}), 142.8 (C_{Ar}), 139.8 (C_{Ar}), 130.5 (CH_{Ar}), 129.5 (CH_{Ar}), 129.4 (CH_{Ar}), 127.0 (CH_{Ar}), 123.8 (C_{Ar}), 121.6 (CH_{Ar}), 116.9 (CH_{Ar}), 113.2 (CH_{Ar}), 104.8 (CH_{Ar}), 56.7 (OCH₃), 40.4 (CH₂NH₃⁺), 36.7 (CH₂CH₂Ph), 34.2 (CH₂CH₂Ph), 28.0 (CH₂CH₂NH₃⁺), 27.1 (TrizCH₂CH₂), 25.6 (TrizCH₂); **HPLC:** Gradient 1, tᵣ = 8.1 min; **HRMS (ES, positive mode) m/z:** calculated for C₂₄H₂₇N₅OS 433.1936; Found 433.1931 (-1.25 ppm); **Elemental analysis:** calculated for C₂₆H₂₈F₃N₅O₃S: C. 57.03; H. 5.15; N. 12.79; S. 5.85; Found: C. 57.25; H. 5.31; N. 12.61; S. 5.39

### 1-(2-(5-(4-(3-aminopropyl)-1H-1,2,3-triazol-1-yl)-2-(4-phenethylthiazol-2-yl)phenoxy)ethyl)imidazolidin-2-one 2,2,2 trifluoroacetate (8n)

**¹H-NMR (CD₃OD, 400 MHz) δ (ppm):** 8.47 (s, 1H, Ar), 8.46 (d, *J* = 8.6 Hz, 1H, Ar), 7.71 (d, *J* = 2.1 Hz, 1H, Ar), 7.59 (dd, *J* = 8.6, 2.1 Hz, 1H, Ar), 7.33 - 7.17 (m, 5H, Ar), 7.15 (s, 1H, Ar), 4.49 (t, *J* = 5.5 Hz, 2H, OCH₂), 3.75 (t, *J* = 5.5 Hz, 2H, OCH₂CH₂), 3.60 (dd, *J* = 9.3, 6.9 Hz, 2H, CH₂CH₂NHCON), 3.37 (dd, *J* = 9.3, 6.9 Hz, 2H, CH₂CH₂NHCON), 3.18 - 3.03 (m, 6H, CH₂CH₂Ph, CH₂NH₃⁺), 2.93 (t, *J* = 7.4 Hz, 2H, TrizCH₂), 2.12 (quin, *J* = 7.6 Hz, 2H, CH₂CH₂NH₃⁺); **¹³C-NMR (CD₃OD, 75 MHz) δ (ppm):** 165.1 (NHCON), 162.0 (OC_{Ar}), 157.5 (C_{Ar}), 157.1 (C_{Ar}), 148.4 (C_{Ar}), 142.8 (C_{Ar}), 139.6 (C_{Ar}), 130.8 (CH_{Ar}), 129.5 (CH_{Ar}), 129.4 (CH_{Ar}), 127.0 (CH_{Ar}), 123.9 (C_{Ar}), 121.8 (CH_{Ar}), 117.0 (CH_{Ar}), 113.6 (CH_{Ar}), 105.8 (CH_{Ar}), 68.5 (OCH₂), 47.0 (CH₂CH₂NHCON), 43.8 (OCH₂CH₂), 40.2 (CH₂NH₃⁺), 39.3 (CH₂CH₂NHCON), 36.7 (CH₂CH₂Ph), 34.3 (CH₂CH₂Ph), 28.2 (CH₂CH₂NH₃⁺), 23.2 (TrizCH₂); **HPLC:** Gradient 1, tᵣ = 7.7 min; **HRMS (ES, positive mode) m/z:** calculated for C₂₇H₃₁N₇O₂S 517.2260; Found 517.2266 (1.17 ppm); **Elemental analysis:** calculated for C₂₉H₃₂F₃N₇O₄S: C. 55.14; H. 5.11; N. 15.52; S. 5.08; Found: C. 54.99; H. 5.24; N. 15.09; S. 5.00

### 1-(2-(2-(4-(2-([1,1'-Biphenyl]-4-yl)ethyl)thiazol-2-yl)-5-(4-(3-aminopropyl)-1H-1,2,3-triazol-1-yl)phenoxy)ethyl)imidazolidin-2-one 2,2,2 trifluoroacetate (8o)

**¹H-NMR (DMSO-d₆, 400 MHz) δ (ppm):** 8.77 (s, 1H, Ar), 8.49 (d, *J* = 8.6 Hz, 1H, Ar), 7.88 - 7.55 (m, 8H, Ar), 7.53 - 7.41 (m, 2H, Ar), 7.36 - 7.31 (m, 2H, Ar), 6.40 (bs, 1H, NH), 4.47 (t, *J* = 5.7 Hz, 2H, OCH₂), 3.64 (t, *J* = 5.6 Hz, 2H, OCH₂CH₂), 3.49 (dd, *J* = 9.0, 6.7 Hz, 2H, CH₂CH₂NHCON), 3.22 (dd, *J* = 9.0, 6.7 Hz, 2H, CH₂CH₂NHCON), 3.17 - 3.03 (m, 4H, CH₂CH₂BiPh), 2.93 (t, *J* = 7.5, 2H, CH₂NH₃⁺), 2.83 (t, J = 8.5 Hz, 2H, TrizCH₂), 1.98 (quin, *J* = 7.6 Hz, 2H, CH₂CH₂NH₃⁺); **¹³C-NMR (DMSO-d₆, 75 MHz) δ (ppm):** 162.2 (NHCON), 159.4 (OC_{Ar}), 155.6 (C_{Ar}), 155.4 (C_{Ar}), 147.0 (C_{Ar}), 140.7 (C_{Ar}), 140.0 (C_{Ar}), 137.9 (C_{Ar}), 137.8 (C_{Ar}), 129.0 (CH_{Ar}), 128.9 (CH_{Ar}), 128.9 (CH_{Ar}), 127.2 (CH_{Ar}), 126.6 (CH_{Ar}), 126.5 (CH_{Ar}), 121.5 (C_{Ar}), 120.6 (CH_{Ar}), 116.1 (CH_{Ar}), 112.1 (CH_{Ar}), 104.5 (CH_{Ar}), 67.6 (OCH₂), 45.4 (CH₂CH₂NHCON), 42.4 (OCH₂CH₂), 37.6 (CH₂NH₃⁺), 37.5 (CH₂CH₂NHCON), 34.3 (CH₂CH₂BiPh), 32.6 (CH₂CH₂BiPh), 26.7 (CH₂CH₂NH₃⁺), 22.0 (TrizCH₂); **HPLC:** Gradient 1, tᵣ = 8.4 min; **HRMS (ES, positive mode) m/z:** calculated for C₃₃H₃₅N₇O₂S 593.2573; Found 593.2572 (-0.09 ppm); **Elemental analysis:** calculated for C₃₅H₃₆F₃N₇O₄S: C. 59.40; H. 5.13; N. 13.85; S. 4.53; Found: C. 59.67; H. 5.41; N. 13.66; S. 4.25

### 1-(2-(5-(4-(4-Aminobutyl)-1H-1,2,3-triazol-1-yl)-2-(4,5-diphenylthiazol-2-yl)phenoxy)ethyl)imidazolidin-2-one 2,2,2 trifluoroacetate (8p)

**¹H-NMR (CD₃OD, 400 MHz) δ (ppm):** 8.56 (d, *J* = 8.6 Hz, 1H, Ar), 8.46 (s, 1H, Ar), 7.71 (d, *J* = 8.0 Hz, 1H, Ar), 7.59 (dd, *J* = 8.6, 2.0 Hz, 1H, Ar), 7.56 - 7.51 (m, 2H, Ar), 7.44 - 7.27 (m, 8H, Ar), 4.51 (t, *J* = 5.5 Hz, 2H, OCH₂), 3.76 (t, *J* = 5.5 Hz, 2H, OCH₂CH₂), 3.62 (dd, *J* = 9.3, 6.9 Hz, 2H, CH₂CH₂NHCON), 3.32 (dd, *J* = 9.3, 6.9 Hz, 2H, CH₂CH₂NHCON), 3.00 (q, *J* = 6.5 Hz, 2H, CH₂NH₃⁺), 2.86 (t, *J* = 7.2 Hz, 2H, TrizCH₂), 1.95 - 1.65 (m, 4H, TrizCH₂CH₂, CH₂CH₂NH₃⁺); **¹³C-NMR (CD₃OD, 75 MHz) δ (ppm):** 165.0 (NHCON), 159.9 (OC_{Ar}), 157.5 (C_{Ar}), 150.6 (C_{Ar}), 149.3 (C_{Ar}), 139.8 (C_{Ar}), 136.4 (C_{Ar}), 135.8 (C_{Ar}), 133.3 (CH_{Ar}), 130.6 (CH_{Ar}), 130.5 (CH_{Ar}), 130.3 (CH_{Ar}), 129.9 (CH_{Ar}), 129.3 (CH_{Ar}), 129.0 (CH_{Ar}), 129.3 (CH_{Ar}), 129.0 (CH_{Ar}), 123.7 (CH_{Ar}), 121.6 (CH_{Ar}), 113.6 (CH_{Ar}), 105.6 (CH_{Ar}), 68.2 (OCH₂), 46.9 (CH₂CH₂NHCON), 43.9 (OCH₂CH₂), 40.4 (CH₂NH₃⁺), 39.3 (CH₂CH₂NHCON), 28.0 (CH₂CH₂NH₃⁺), 27.1 (TrizCH₂CH₂), 25.6 (TrizCH₂); **HRMS (ES, positive mode) m/z:** calculated for C₃₂H₃₃N₇O₂S 579.2416; Found 579.242 (0.61 ppm); **Elemental analysis:** calculated for C₃₄H₃₄F₃N₇O₄S: C. 58.87; H. 4.94; N. 14.13; S. 4.62; Found: C. 59.15; H. 5.36 N. 14.62; S. 5.03

### 1-(2-(5-(4-(4-Aminobutyl)-1H-1,2,3-triazol-1-yl)-2-(4-(3-phenylpropyl)thiazol-2-yl)phenoxy)ethyl)imidazolidin-2-one hydrochloride salt (8q)

**¹H-NMR (CD₃OD, 400 MHz) δ (ppm):** 8.48 (s, 1H, Ar), 8.44 (d, *J* = 8.6 Hz, 1H, Ar), 7.71 (d, *J* = 1.9 Hz, 1H, Ar), 7.56 (dd, *J* = 8.5, 1.9 Hz, 1H, Ar), 7.35 - 7.15 (m, 5H, Ar), 7.16 (s, 1H, Ar), 4.48 (t, *J* = 5.7 Hz, 2H, OCH₂), 3.75 (t, *J* = 5.7 Hz, 2H, OCH₂CH₂), 3.62 (dd, *J* = 9.0, 6.8 Hz, 2H, CH₂CH₂NHCON), 3.40 (dd, *J* = 9.0, 6.8 Hz, 2H, CH₂CH₂NHCON), 3.17 - 3.00 (m, 4H, CH₂CH₂Ph), 2.96 (t, *J* = 7.6 Hz, 2H, CH₂NH₃⁺), 2.88 (t, *J* = 7.5 Hz, 2H, TrizCH₂), 1.85 (m, 2H, TrizCH₂CH₂), 1.62 (m, 2H, CH₂CH₂NH₃⁺); **¹³C-NMR (CD₃OD, 100 MHz) δ (ppm):** 165.1 (NHCON), 162.1 (OC_{Ar}), 157.3 (C_{Ar}), 157.0 (C_{Ar}), 149.2 (C_{Ar}), 142.8 (C_{Ar}), 139.8 (C_{Ar}), 130.7 (CH_{Ar}), 129.5 (CH_{Ar}), 129.3 (CH_{Ar}), 127.0 (CH_{Ar}), 123.6 (C_{Ar}), 121.8 (CH_{Ar}), 116.8 (CH_{Ar}), 113.6 (CH_{Ar}), 105.7 (CH_{Ar}), 68.5 (OCH₂), 47.1 (CH₂CH₂NHCON), 43.8 (OCH₂CH₂), 40.2 (CH₂NH₃⁺), 39.3 (CH₂CH₂NHCON), 36.7 (CH₂CH₂Ph), 34.5 (CH₂CH₂Ph), 28.0 (CH₂CH₂NH₃⁺), 27.2 (TrizCH₂CH₂), 25.6 (TrizCH₂); **HPLC:** Gradient 1, tᵣ = 4.5 min; **HRMS (ES, positive mode) m/z:** calculated for C₂₈H₃₃N₇O₂S 531.2416; Found 531.2420 (1.30 ppm); **Elemental analysis:** calculated for C₂₈H₃₄ClN₇O₂S: C. 59.19 H. 6.03; N. 17.26; S. 5.64; Found: C. 59.05; H. 5.52; N. 17.08; S. 5.62

### Example 2. Synthesis of type compounds of formula (I-B)

The synthesis of compounds of formula **(I-B)** is shown in **Scheme 3.** It involves a two-step procedure of regioselective alkylation of the 1,2,3-triazole precursors **7** with alkyl trifluoromethane sulfonates to give intermediates **9** as trifluoromethane sulfonate salts followed by treatment with TFA or catalytic hydrogenation in the presence of TFA to yield the desired unprotected triazolium di- or tricationic salts. Only N-1-alkyl-1,2,3-triazoles isomers **10** were obtained in the alkylation reaction.

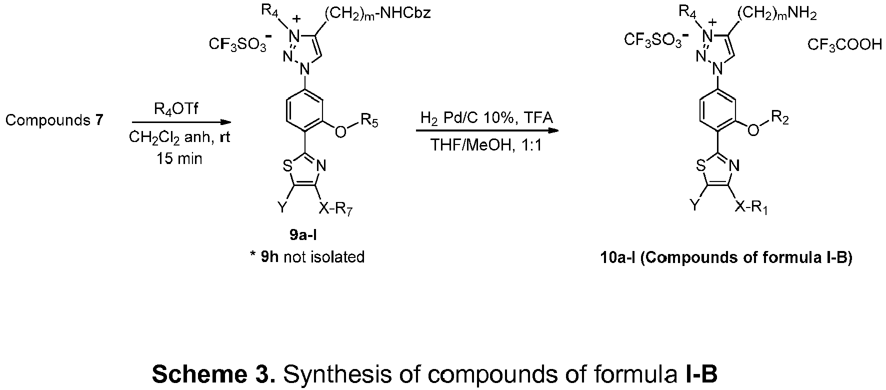

### I) Triazole alkylation

To a solution of the corresponding triazole **7** (1 mmol) in dry CH₂Cl₂ (10 mL), methyl trifluoromethanesulfonate or ethyl trifluoromethanesulfonate (1 mmol) was added and the mixture was stirred at room temperature for 15 min. Then, the reaction was quenched with MeOH (10 mL) and was evaporated to dryness. The residue was purified by Cromatotron (CH₂Cl₂/MeOH, 9:1) to give the appropriate alkylated triazoles **9** as a yellow oil in yields from 65% to 95%. In the case of intermediate **9h,** the corresponding alkylated triazole was not isolated and was immediately used in the next step without purification.

### 4-(4-(((Benzyloxy)carbonyl)amino)butyl)-3-methyl-1-(3-(2-(2-oxoimidazolidin-1-yl)ethoxy)-4-(4-phenylthiazol-2-yl)phenyl)-1H-1,2,3-triazol-3-ium, trifluoromethanosulfonate (9a)

**¹H-NMR (CD₃OD, 400 MHz) δ (ppm):** 9.14 (s, 1H, Ar), 8.62 (d, *J* = 8.5 Hz, 1H, Ar), 8.00 (dt, *J* = 6.5, 1.4 Hz, 2H, Ar), 7.92 (s, 1H, Ar), 7.69 - 7.61 (m, 2H, Ar), 7.44 (t, *J* = 7.5 Hz, 2H, Ar), 7.38 - 7.21 (m, 6H, Ar), 5.08 (s, 2H, OCH₂Ph), 4.47 (t, *J* = 5.7 Hz, 2H, OCH₂), 4.28 (s, 3H, CH₃), 3.76 (t, *J* = 5.7 Hz, 2H, OCH₂CH₂), 3.62 (dd, *J* = 9.3, 6.9 Hz, 2H, CH₂CH₂NHCON), 3.40 (dd, *J* = 9.2, 6.8 Hz, 2H, CH₂CH₂NHCON), 3.25 (t, *J* = 6.7 Hz, 2H, CH₂NHCbz), 2.90 (t, *J* = 7.7 Hz, 2H, TrizCH₂), 1.84 (quin, *J* = 7.6 Hz, 2H, TrizCH₂CH₂), 1.71 (quin, *J* = 7.1 Hz, 2H, CH₂CH₂NHCbz); **¹³C-NMR (CD₃OD, 75 MHz) δ (ppm):** 165.1 (NHCON), 161.0 (OC_{Ar}), 159.1 (C_{Ar}), 157.3 (C_{Ar}), 155.8 (C_{Ar}), 146.8 (C_{Ar}), 138.5 (C_{Ar}), 137.1 (C_{Ar}), 135.6 (CH_{Ar}), 131.0 (CH_{Ar}), 129.9 (CH_{Ar}), 129.5 (CH_{Ar}), 129.3 (CH_{Ar}), 129.0 (CH_{Ar}), 128.7 (C_{Ar}), 127.4 (CH_{Ar}), 126.2 (CH_{Ar}), 116.9 (CH_{Ar}), 114.4 (CH_{Ar}), 106.5 (CH_{Ar}), 68.4 (OCH₂), 67.4 (OCH₂Ph), 46.9 (CH₂CH₂NHCON), 43.7 (OCH₂CH₂), 40.9 (CH₂NHCbz), 39.3 (CH₂CH₂NHCON), 38.4 (CH₃), 30.2 (CH₂CH₂NHCbz), 24.8 (TrizCH₂CH₂), 23.8 (TrizCH₂); **HRMS (ES, positive mode) m/z:** calculated for C₃₅H₃₈N₇O₄S 652.2706; Found 652.2713 (1.04 ppm)

### 4-(4-(((Benzyloxy)carbonyl)amino)butyl)-3-methyl-1-(3-(2-(2-oxoimidazolidin-1-yl)ethoxy)-4-(4-phenethylthiazol-2-yl)phenyl)-1H-1,2,3-triazol-3-ium, trifluoromethanesulfonate (9b)

**¹H-NMR (CD₃OD, 400 MHz) δ (ppm):** 9.24 (s, 1H, Ar), 8.59 (d, *J* = 8.6 Hz, 1H, Ar), 7.81 (d, *J* = 2.2 Hz, 1H, Ar), 7.70 (dd, *J* = 8.6, 2.1 Hz, 1H, Ar), 7.74 - 7.08 (m, 11H, Ar), 5.07 (s, 2H, OCH₂Ph), 4.52 (t, *J* = 5.6 Hz, 2H, OCH₂), 4.35 (s, 3H, CH₃), 3.75 (t, *J* = 5.6 Hz, 2H, OCH₂CH₂), 3.60 (dd, *J* = 9.3, 6.9 Hz, 2H, CH₂CH₂NHCON), 3.36 (dd, *J* = 9.3, 6.9 Hz, 2H, CH₂CH₂NHCON), 3.25 (t, *J* = 6.8 Hz, 2H, CH₂NHCbz), 3.18 - 3.02 (m, 4H, CH₂CH₂Ph), 3.00 (t, *J* = 7.8 Hz, 2H, TrizCH₂), 1.91 (quin, *J* = 6.9 Hz, 2H, TrizCH₂CH₂), 1.70 (quin, *J* = 6.9 Hz, 2H, CH₂CH₂NHCbz); **¹³C-NMR (CD₃OD, 100 MHz) δ (ppm):** 165.0 (NHCON), 160.9 (OC_{Ar}), 159.0 (C_{Ar}), 157.5 (C_{Ar}), 157.3 (C_{Ar}), 146.9 (C_{Ar}), 146.8 (C_{Ar}), 142.7 (C_{Ar}), 138.4 (C_{Ar}), 137.3 (C_{Ar}), 131.0 (C_{Ar}), 129.5 (CH_{Ar}), 129.4 (CH_{Ar}), 129.4 (CH_{Ar}), 128.9 (CH_{Ar}), 128.7 (CH_{Ar}), 127.5 (CH_{Ar}), 127.0 (CH_{Ar}), 126.5 (CH_{Ar}), 117.9 (CH_{Ar}), 114.7 (CH_{Ar}), 107.1 (CH_{Ar}), 68.6 (OCH₂), 67.4 (OCH₂Ph), 46.8 (CH₂CH₂NHCON), 43.6 (OCH₂CH₂), 40.9 (CH₂NHCbz), 39.3 (CH₂CH₂NHCON), 38.4 (CH₃), 36.6 (CH₂CH₂Ph), 34.3 (CH₂CH₂Ph), 30.1 (CH₂CH₂NHCbz), 24.8 (TrizCH₂CH₂), 23.8 (TrizCH₂); **HRMS (ES, positive mode) m/z:** calculated for C₃₇H₄₂N₇O₄S 680.3019; Found 680.3019 (0.01 ppm)

### 4-(4-(((Benzyloxy)carbonyl)amino)butyl)-3-methyl-1-(4-(4-(2-(naphthalen-2-yl)ethyl)thiazol-2-yl)-3-(2-(2-oxoimidazolidin-1-yl)ethoxy)phenyl)-1H-1,2,3-triazol-3-ium trifluoromethanesulfonate (9c)

**¹H-NMR (CD₃OD, 400 MHz) δ (ppm):** 8.99 (s, 1H, Ar), 8.54 (d, *J* = 8.6 Hz, 1H, Ar), 7.85 - 7.77 (m, 3H, Ar), 7.73 (s, 1H, Ar), 7.64 (t, *J* = 8.4 Hz, 2H, Ar), 7.54 (d, *J* = 2.2 Hz, 1H, Ar), 7.49 - 7.41 (m, 3H, Ar), 7.41 - 7.25 (m, 6H, Ar), 7.14 (d, *J* = 15.9 Hz, 1H, ThiazCH=CH), 5.11 (s, 2H, OCH₂Ph), 4.41 (t, *J* = 5.9 Hz, 2H, OCH₂), 4.06 (s, 3H, CH₃), 3.75 (t, *J* = 5.8 Hz, 2H, OCH₂CH₂), 3.65 (dd, *J* = 9.3, 6.8 Hz, 2H, CH₂CH₂NHCON), 3.42 (dd, *J* = 9.2, 6.9 Hz, 2H, CH₂CH₂NHCON), 3.18 (t, *J* = 6.6 Hz, 2H, CH₂NHCbz), 2.49 (t, *J* = 7.7 Hz, 2H, TrizCH₂), 1.69 - 1.48 (m, 4H, TrizCH₂CH₂, CH₂CH₂NHCbz); **¹³C-NMR (CD₃OD, 100 MHz) δ (ppm):** 165.1 (NHCON), 160.9 (OC_{Ar}), 159.1 (C_{Ar}), 157.3 (C_{Ar}), 154.5 (C_{Ar}), 146.6 (C_{Ar}), 138.5 (C_{Ar}), 137.0 (C_{Ar}), 135.9 (CH_{Ar}), 135.0 (C_{Ar}), 134.4 (C_{Ar}), 131.8 (CH_{Ar}), 131.1 (CH_{Ar}), 129.5 (CH_{Ar}), 129.3 (CH_{Ar}), 129.1 (CH_{Ar}), 129.0 (CH_{Ar}), 128.8 (CH_{Ar}), 127.8 (C_{Ar}), 127.4 (CH_{Ar}), 126.6 (CH_{Ar}), 124.3 (CH_{Ar}), 123.4 (CH_{Ar}), 122.7 (CH_{Ar}), 119.7 (CH_{Ar}), 114.0 (CH_{Ar}), 106.1 (CH_{Ar}), 68.3 (OCH₂), 67.4 (OCH₂Ph), 47.0 (CH₂CH₂NHCON), 43.8 (OCH₂CH₂), 40.9 (CH₂NHCbz), 39.4 (CH₂CH₂NHCON), 38.2 (CH₃), 30.2 (CH₂CH₂NHCbz), 24.6 (TrizCH₂CH₂), 23.7 (TrizCH₂); **HRMS (ES, positive mode) m/z:** calculated for C₄₁H₄₂N₇O₄S 728.3019; Found 728.3022 (0.39 ppm)

### 4-(4-(((Benzyloxy)carbonyl)amino)butyl)-1-(4-(4-(2-(2,3-dihydrobenzofuran-6-yl)ethyl)thiazol-2-yl)-3-(2-(2-oxoimidazolidin-1-yl)ethoxy)phenyl)-3-methyl-1H-1,2,3-triazol-3-ium trifluoromethanesulfonate (9d)

**¹H-NMR (CDCl₃, 400 MHz) δ (ppm):** 9.09 (s, 1H, Ar), 8.21 (d, *J* = 8.6 Hz, 1H, Ar), 7.41 (d, *J* =8.7 Hz, 1H, Ar), 7.40 - 7.20 (m, 7H, Ar), 7.15 - 7.07 (m, 3H, Ar), 6.75 (d, *J* = 15.8 Hz, 1H, ThiazCH=CH), 6.70 (d, *J* = 8.1 Hz, 1H Ar), 5.89 (bs, 1H, NH), 5.25 (bs, 1H, NH), 5.05 (s, 2H, OCH₂Ph), 4.56 (t, *J* = 8.9 Hz, 2H, OCH₂CH₂Ph), 4.14 (m, 2H, OCH₂), 4.00 (s, 3H, CH₃), 3.60 (m, 2H, OCH₂CH₂), 3.47 (t, *J* = 7.8 Hz, 2H, CH₂CH₂NHCON), 3.31 (m, 2H, CH₂CH₂NHCON), 3.23 - 3.12 (m, 4H, CH₂NHCbz, OCH₂CH₂Ph), 2.52 (m, 2H, TrizCH₂), 1.72 (m, 2H, TrizCH₂CH₂), 1.55 (m, 2H, CH₂CH₂NHCbz); **¹³C-NMR (CDCl₃, 100 MHz) δ (ppm):** 163.0 (NHCON), 160.3 (OC_{Ar}), 159.0 (OC_{Ar}), 156.9 (C_{Ar}), 155.5 (C_{Ar}), 153.3 (C_{Ar}), 145.5 (C_{Ar}), 137.0 (C_{Ar}), 130.6 (C_{Ar}), 129.6 (CH_{Ar}), 128.5 (CH_{Ar}), 128.0 (CH_{Ar}), 127.2 (CH_{Ar}), 125.5 (CH_{Ar}), 124.0 (C_{Ar}), 123.0 (CH_{Ar}), 122.4 (CH_{Ar}), 119.2 (CH_{Ar}), 118.5 (CH_{Ar}), 117.3 (CH_{Ar}), 112.1 (CH_{Ar}), 109.3 (CH_{Ar}), 104.1 (CH_{Ar}), 71.7 (OCH₂CH₂Ph), 66.8 (OCH₂), 66.5 (OCH₂Ph), 45.6 (CH₂CH₂NHCON), 42.4 (OCH₂CH₂), 41.1 (CH₃), 40.1 (CH₂NHCbz), 38.4 (CH₂CH₂NHCON), 29.5 (OCH₂CH₂Ph), 29.4 (CH₂CH₂NHCbz), 23.6 (TrizCH₂CH₂), 23.1 (TrizCH₂); **HRMS (ES, positive mode) m/z:** calculated for C₃₉H₄₂N₇O₅S 720.2968; Found 720.2998 (4.18 ppm)

### 1-(4-(4-(2-([1,1'-Biphenyl]-4-yl)ethyl)thiazol-2-yl)-3-(2-(2-oxoimidazolidin-1-yl)ethoxy)phenyl)-4-(4-(((benzyloxy)carbonyl)amino)butyl)-3-methyl-1H-1,2,3-triazol-3-ium trifluoromethanesulfonate (9e)

**¹H-NMR (DMSO-d₆, 400 MHz) δ (ppm):** 9.54 (s, 1H, Ar), 8.70 (d, *J* = 8.6 Hz, 1H, Ar), 7.90 (d, *J* = 2.1 Hz, 1H, Ar), 7.86 (s, 1H, Ar), 7.79 (dd, *J* = 8.6, 2.1 Hz, 1H, Ar), 7.75 - 7.65 (m, 6H, Ar), 7.59 (d, *J* = 16.1 Hz, 1H, Ar), 7.48 (t, *J* = 7.7 Hz, 1H, Ar), 7.43 - 7.24 (m, 8H, Ar), 6.44 (bs, 1H, NH), 5.03 (s, 2H, OCH₂Ph), 4.52 (t, *J* = 5.6 Hz, 2H, OCH₂), 4.35 (s, 3H, CH₃), 3.68 (t, *J* = 5.5 Hz, 2H, OCH₂CH₂), 3.52 (dd, *J* = 8.9, 6.7 Hz, 2H, CH₂CH₂NHCON), 3.25 (t, *J* = 7.9 Hz, 2H, CH₂CH₂NHCON), 3.12 (q, *J* = 6.5 Hz, 2H, CH₂NHCbz), 2.94 (t, *J* = 7.6 Hz, 2H, TrizCH₂), 1.78 (quin, *J* = 7.6 Hz, 2H, TrizCH₂CH₂), 1.62 (quin, *J* = 7.2 Hz, 2H, CH₂CH₂NHCbz); **¹³C-NMR (DMSO-d₆, 75 MHz) δ (ppm):** 162.2 (NHCON), 159.4 (OC_{Ar}), 156.2 (C_{Ar}), 155.8 (C_{Ar}), 152.9 (C_{Ar}), 145.1 (C_{Ar}), 139.5 (C_{Ar}), 139.5 (C_{Ar}), 137.2 (CH_{Ar}), 135.9 (C_{Ar}), 130.1 (CH_{Ar}), 129.6 (CH_{Ar}), 129.0 (CH_{Ar}), 128.4 (CH_{Ar}), 128.0 (CH_{Ar}), 127.8 (CH_{Ar}), 127.6 (CH_{Ar}), 126.5 (C_{Ar}), 123.8 (CH_{Ar}), 121.7 (CH_{Ar}), 119.6 (CH_{Ar}), 113.3 (CH_{Ar}), 106.0 (CH_{Ar}), 68.1 (OCH₂), 65.3 (OCH₂Ph), 45.4 (CH₂CH₂NHCON), 42.4 (OCH₂CH₂), 37.9 (CH₂NHCbz), 37.6 (CH₂CH₂NHCON), 28.6 (CH₂CH₂NHCbz), 23.4 (TrizCH₂CH₂), 22.3 (TrizCH₂); **HRMS (ES, positive mode) m/z:** calculated for C₄₃H₄₄N₇O₄S 754.3176; Found 754.3197 (2.81 ppm)

### 1-(4-(4-(2-([1,1'-Biphenyl]-4-yl)ethyl)thiazol-2-yl)-3-(2-(2-oxoimidazolidin-1-yl)ethoxy)phenyl)-4-(4-(((benzyloxy)carbonyl)amino)butyl)-3-ethyl-1H-1,2,3-triazol-3-ium trifluoromethanesulfonate (9f)

**¹H-NMR (CD₃OD, 400 MHz) δ (ppm):** 9.20 (s, 1H, Ar), 8.66 (d, *J* = 8.5 Hz, 1H, Ar), 7.79 - 7.54 (m, 9H, Ar), 7.52 - 7.40 (m, 3H, Ar), 7.39 - 7.24 (m, 5H, Ar), 7.22 (d, *J* = 16.0 Hz, 1H, Ar), 5.08 (s, 2H, OCH₂Ph), 4.62 (q, *J* = 7.3 Hz, 2H, CH₂CH₃), 4.51 (t, *J* = 5.7 Hz, 2H, OCH₂), 3.78 (t, *J* = 5.7 Hz, 2H, OCH₂CH₂), 3.68 (dd, *J* = 9.3, 6.8 Hz, 2H, CH₂CH₂NHCON), 3.40 (dd, *J* = 9.2, 6.9 Hz, 2H, CH₂CH₂NHCON), 3.23 (t, *J* = 6.7 Hz, 2H, CH₂NHCbz), 2.89 (t, *J* = 7.8 Hz, 2H, TrizCH₂), 1.84 (quin, *J* = 7.6 Hz, 2H, TrizCH₂CH₂), 1.72 -1.62 (m, 5H, CH₂CH₂NHCbz, CH₂CH₃); **¹³C-NMR (CD₃OD, 100 MHz) δ (ppm):** 165.1 (NHCON), 161.2 (OC_{Ar}), 159.1 (C_{Ar}), 157.5 (C_{Ar}), 154.9 (C_{Ar}), 146.4 (C_{Ar}), 141.7 (C_{Ar}), 141.6 (C_{Ar}), 138.5 (C_{Ar}), 137.5 (C_{Ar}), 137.4 (C_{Ar}), 131.8 (C_{Ar}), 131.3 (CH_{Ar}), 130.0 (CH_{Ar}), 129.5 (CH_{Ar}), 129.0 (CH_{Ar}), 128.7 (CH_{Ar}), 128.6 (CH_{Ar}), 128.2 (C_{Ar}), 128.2 (CH_{Ar}), 127.8 (CH_{Ar}), 127.7 (CH_{Ar}), 127.4 (CH_{Ar}), 126.2 (CH_{Ar}), 122.4 (CH_{Ar}), 119.5 (CH_{Ar}), 114.6 (CH_{Ar}), 106.8 (CH_{Ar}), 68.5 (OCH₂), 67.4 (OCH₂Ph), 47.0 (CH₂CH₂NHCON), 43.7 (OCH₂CH₂), 40.9 (CH₂NHCbz), 39.3 (CH₂CH₂NHCON), 30.2 (CH₂CH₂NHCbz), 25.0 (TrizCH₂CH₂), 23.7 (TrizCH₂), 14.2 (CH₃); **HRMS (ES, positive mode) m/z:** calculated for C₄₄H₄₆N₇O₄S 768.3332; Found 768.3329 (-0.44 ppm)

### 4-(4-(((Benzyloxy)carbonyl)amino)butyl)-1-(4-(4-(4-methoxyphenyl)thiazol-2-yl)-3-(2-(2-oxoimidazolidin-1-yl)ethoxy)phenyl)-3-methyl-1 H-1,2,3-triazol-3-ium trifluoromethanesulfonate (9g)

**¹H-NMR (CDCl₃, 400 MHz) δ (ppm):** 9.11 (s, 1H, Ar), 8.56 (d, *J* = 8.5 Hz, 1H, Ar), 7.90 (d, *J* = 8.7 Hz, 2H, Ar), 7.74 (s, 1H, Ar), 7.63 - 7.58 (m, 2H, Ar), 7.38 - 7.25 (m, 5H, Ar), 6.99 (d, *J* = 8.7 Hz, 2H, Ar), 5.08 (s, 2H, OCH₂Ph), 4.45 (t, *J* = 5.7 Hz, 2H, OCH₂), 4.27 (s, 3H, N⁺CH₃), 3.85 (s, 3H, OMe), 3.75 (t, *J* = 5.7 Hz, 2H, OCH₂CH₂), 3.62 (dd, *J* = 9.3, 6.8 Hz, 2H, CH₂CH₂NHCON), 3.39 (dd, *J* = 9.2, 6.9 Hz, 2H, CH₂CH₂NHCON), 3.24 (t, *J* = 6.7 Hz, 2H, CH₂NHCbz), 2.87 (t, *J* = 7.7 Hz, 2H, TrizCH₂), 1.82 (quin, *J* = 7.9 Hz, 2H, TrizCH₂CH₂), 1.70 (quin, *J* = 7.0 Hz, 2H, CH₂CH₂NHCbz); **¹³C-NMR (CDCl₃,** 75 **MHz) δ (ppm):** 165.0 (NHCON), 161.3 (OC_{Ar}), 160.7 (C_{Ar}), 159.0 (C_{Ar}), 157.1 (C_{Ar}), 155.6 (C_{Ar}), 146.8 (C_{Ar}), 138.4 (C_{Ar}), 136.9 (C_{Ar}), 130.9 (CH_{Ar}), 129.5 (CH_{Ar}), 129.0 (CH_{Ar}), 128.7 (CH_{Ar}), 128.7 (CH_{Ar}), 128.4 (CH_{Ar}), 126.9 (CH_{Ar}), 126.1 (C_{Ar}), 115.2 (CH_{Ar}), 114.2 (CH_{Ar}), 106.3 (CH_{Ar}), 68.3 (OCH₂), 67.4 (OCH₂Ph), 55.9 (OCH₃), 46.8 (CH₂CH₂NHCON), 43.7 (OCH₂CH₂), 40.9 (CH₂NHCbz), 39.3 (CH₂CH₂NHCON), 38.3 (N⁺CH₃), 30.2 (CH₂CH₂NHCbz), 24.8 (TrizCH₂CH₂), 23.8 (TrizCH₂); **HRMS (ES, positive mode) m/z:** calculated for C₃₆H₄₀N₇O₅S 682.2812; Found 682.2809 (-0.32 ppm)

### 4-(4-(((Benzyloxy)carbonyl)amino)butyl)-1-(4-(4-(4-(diethylamino)phenyl)thiazol-2-yl)-3-(2-(2-oxoimidazolidin-1-yl)ethoxy)phenyl)-3-methyl-1H-1,2,3-triazol-3-ium trifluoromethanesulfonate (9h)

Following the general alkylation procedure, compound **9h** was obtained but it was not isolated and it was used directly in the next step without purification.

### 4-(3-(((Benzyloxy)carbonyl)amino)propyl)-3-methyl-1-(3-(2-(2-oxoimidazolidin-1-yl)ethoxy)-4-(4-phenethylthiazol-2-yl)phenyl)-1H-1,2,3-triazol-3-ium trifluoromethanesulfonate (9i)

**¹H-NMR (CD₃OD, 400 MHz) δ (ppm):** 9.28 (s, 1H, Ar), 8.59 (d, *J* = 8.6 Hz, 1H, Ar), 7.80 (d, *J* = 2.1 Hz, 1H, Ar), 7.70 (dd, *J* = 8.7, 2.1 Hz, 1H, Ar), 7.36 - 7.08 (m, 11H, Ar), 5.07 (s, 2H, OCH₂Ph), 4.51 (t, *J* = 5.6 Hz, 2H, OCH₂), 4.32 (s, 3H, CH₃), 3.75 (t, *J* = 5.6 Hz, 2H, OCH₂CH₂), 3.60 (dd, *J* = 9.3, 6.9 Hz, 2H, CH₂CH₂NHCON), 3.39 - 3.28 (m, 4H, CH₂CH₂NHCON, CH₂NHCbz), 3.18 - 3.04 (m, 4H, CH₂CH₂Ph), 3.00 (t, *J* = 7.5 Hz, 2H, TrizCH₂), 2.08 (quin, *J* = 6.9 Hz, 2H, TrizCH₂CH₂); **¹³C-NMR (CD₃OD, 75 MHz) δ (ppm):** 165.6 (NHCON), 160.9 (OC_{Ar}), 159.1 (C_{Ar}), 157.5 (C_{Ar}), 157.3 (C_{Ar}), 146.6 (C_{Ar}), 142.7 (C_{Ar}), 138.3 (C_{Ar}), 137.3 (C_{Ar}), 131.0 (CH_{Ar}), 129.5 (CH_{Ar}), 129.4 (CH_{Ar}), 129.0 (CH_{Ar}), 128.8 (CH_{Ar}), 127.6 (CH_{Ar}), 127.0 (CH_{Ar}), 126.5 (CH_{Ar}), 117.9 (CH_{Ar}), 114.7 (CH_{Ar}), 107.1 (CH_{Ar}), 68.6 (OCH₂), 67.6 (OCH₂Ph), 46.9 (CH₂CH₂NHCON), 43.6 (OCH₂CH₂), 40.4 (CH₂NHCbz), 39.3 (CH₂CH₂NHCON), 38.4 (CH₃), 36.6 (CH₂CH₂Ph), 34.3 (CH₂CH₂Ph), 24.8 (TrizCH₂CH₂), 21.6 (TrizCH₂); **HRMS (ES, positive mode) m/z:** calculated for C₃₆H₄₉N₇O₄S 666.2862; Found 666.2873 (1.59 ppm)

### (E)-1-(4-(4-(2-([1,1'-Biphenyl]-4-yl)vinyl)thiazol-2-yl)-3-(2-(2-oxoimidazolidin-1-yl)ethoxy)phenyl)-4-(3-(((benzyloxy)carbonyl)amino)propyl)-3-methyl-1H-1,2,3-triazol-3-ium trifluoromethanesulfonate (9j)

**¹H-NMR (DMSO-d₆, 400 MHz) δ (ppm):** 9.56 (s, 1H, Ar), 8.71 (d, *J* = 8.6 Hz, 1H, Ar), 8.01 - 7.68 (m, 9H, Ar), 7.60 (d, *J* = 16.1 Hz, 1H, ThiazCH=CH), 7.52 - 7.25 (m, 9H, Ar), 6.43 (bs, 1H, NH), 5.04 (s, 2H, OCH₂Ph), 4.52 (t, *J* = 5.9 Hz, 2H, OCH₂), 4.32 (s, 3H, CH₃), 3.68 (t, *J* = 5.6 Hz, 2H, OCH₂CH₂), 3.52 (dd, *J* = 9.0, 6.6 Hz, 2H, CH₂CH₂NHCON), 3.27 - 3.16 (m, 4H, CH₂CH₂NHCON, CH₂NHCbz), 2.94 (t, *J* = 7.7 Hz, 2H, TrizCH₂), 1.96 (quin, *J* = 7.3 Hz, 2H, TrizCH₂CH₂); **¹³C-NMR (DMSO-d₆, 100 MHz) δ (ppm):** 162.1 (NHCON), 159.4 (OC_{Ar}), 156.3 (C_{Ar}), 155.8 (C_{Ar}), 152.9 (C_{Ar}), 144.8 (C_{Ar}), 139.5 (C_{Ar}), 139.5 (C_{Ar}), 137.1 (C_{Ar}), 135.8 (CH_{Ar}), 129.0 (CH_{Ar}), 129.0 (CH_{Ar}), 128.4 (CH_{Ar}), 127.8 (CH_{Ar}), 127.8 (CH_{Ar}), 127.2 (CH_{Ar}), 127.0 (CH_{Ar}), 126.6 (CH_{Ar}), 126.5 (CH_{Ar}), 126.4 (CH_{Ar}), 123.8 (CH_{Ar}), 119.6 (CH_{Ar}), 113.3 (CH_{Ar}), 106.1 (CH_{Ar}), 68.1 (OCH₂), 65.4 (OCH₂Ph), 45.4 (CH₂CH₂NHCON), 42.4 (OCH₂CH₂), 39.9 (CH₂NHCbz), 37.9 (CH₂CH₂Ph), 37.6 (CH₂CH₂Ph), 26.6 (TrizCH₂CH₂), 20.3 (TrizCH₂); **HRMS (ES, positive mode) m/z:** calculated for C₄₂H₄₂N₇O₄S 740.3019; Found 740.3023 (0.51 ppm)

### 4-(4-(((Benzyloxy)carbonyl)amino)butyl)-1-(3-(2-(1-((benzyloxy)carbonyl)piperidin-4-yl)ethoxy)-4-(4-phenethylthiazol-2-yl)phenyl)-3-methyl-1H-1,2,3-triazol-3-ium trifluoromethanesulfonate (9k)

**¹H-NMR (CDCl₃, 400 MHz) δ (ppm):** 9.24 (s, 1H, Ar), 8.53 (d, *J* = 8.6 Hz, 1H, Ar), 7.66 (s, 1H, Ar), 7.45 (dd, *J* = 8.7, 2.2 Hz, 1H, Ar), 7.36 - 7.05 (m, 15H, Ar), 6.88 (s, 1H, Ar), 5.54 (t, *J* = 5.9 Hz, 1H, NHCbz), 5.05 (s, 2H, NCOOCH₂), 4.99 (s, 2H, NCOOCH₂), 4.27 (t, *J* = 6.4 Hz, 2H, OCH₂), 4.19 - 4.05 (m, 5H, CH₃, H'HCN(Cbz)CHH'), 3.19 (q, *J* = 6.4 Hz, 2H, CH₂NHCbz), 3.12 - 2.98 (m, 4H, CH₂CH₂Ph), 2.87 (t, J = 7.9 Hz, 2H, TrizCH₂), 2.71 (t, *J* = 11.9 Hz, 2H, H'HCN(Cbz)CHH'), 1.90 - 1.68 (m, 7H, TrizCH₂CH₂, CH, CH(CHH')₂, OCH₂CH₂), 1.59 (m, 2H, CH₂CH₂NHCbz), 1.13 (m, 2H, CH(CHH')₂); **¹³C-NMR (CDCl₃, 75 MHz) δ (ppm):** 159.3 (OC_{Ar}), 157.1 (NCOO), 156.7 (NCOO), 156.3 (C_{Ar}), 155.4 (C_{Ar}), 145.9 (C_{Ar}), 141.6 (C_{Ar}), 137.1 (C_{Ar}), 137.0 (C_{Ar}), 135.4 (CH_{Ar}), 130.0 (CH_{Ar}), 128.6 (CH_{Ar}), 128.6 (CH_{Ar}), 128.5 (CH_{Ar}), 128.0 (CH_{Ar}), 127.9 (CH_{Ar}), 126.9 (CH_{Ar}), 126.2 (CH_{Ar}), 125.6 (C_{Ar}), 116.4 (CH_{Ar}), 112.5 (CH_{Ar}), 105.5 (CH_{Ar}), 68.3 (OCH₂Ph), 67.1 (OCH₂Ph), 66.6 (OCH₂), 44.3 (CH₂N(Cbz)CH₂), 39.8 (CH₂NHCbz), 37.9 (N⁺CH₃), 35.7 (OCH₂CH₂), 35.6 (CH₂CH₂Ph), 33.4 (CH), 33.0 (CH₂CH₂Ph), 32.1 (CH(CH)₂), 29.4 (CH₂CH₂NHCbz), 24.1 (TrizCH₂CH₂), 23.2 (TrizCH₂); **HRMS (ES, positive mode) m/z:** calculated for C₄₇H₅₃N₆O₅S 813.3798; Found 813.3810 (1.44 ppm)

### 4-(4-(((Benzyloxy)carbonyl)amino)butyl)-1-(4-(4,5-diphenylthiazol-2-yl)-3-(2-(2-oxoimidazolidin-1-yl)ethoxy)phenyl)-3-methyl-1H-1,2,3-triazol-3-ium trifluoromethanesulfonate (9l)

**¹H-NMR (CDCl₃, 400 MHz) δ (ppm):** 9.24 (s, 1H, Ar), 8.59 (d, *J* = 8.7 Hz, 1H, Ar), 7.76 (d, *J* = 2.2 Hz, 1H, Ar), 7.56 - 7.49 (m, 3H, Ar), 7.33 - 7.13 (m, 12H, Ar), 5.62 (t, *J* =6.0 Hz, 1H, NHCbz), 4.99 (s, 2H, OCH₂Ph), 4.74 (bs, 1H, NH), 4.42 (t, *J* = 7.1 Hz, 2H, OCH₂), 4.18 (s, 3H, CH₃), 3.64 (t, *J* = 6.2 Hz, 2H, OCH₂CH₂), 3.45 (dd, *J* = 9.1, 6.7 Hz, 2H, CH₂CH₂NHCON), 3.26 - 3.17 (m, 4H, CH₂NHCbz, CH₂CH₂NHCON), 2.81 (t, *J* = 8.0 Hz, 2H, TrizCH₂), 1.88 (m, 2H, TrizCH₂CH₂), 1.61 (m, 2H, CH₂CH₂NHCbz); **¹³C-NMR (CDCl₃, 75 MHz) δ (ppm):** 162.9 (NHCON), 157.2 (OC_{Ar}), 157.1 (C_{Ar}), 156.1 (C_{Ar}), 149.7 (C_{Ar}), 146.0 (C_{Ar}), 136.9 (C_{Ar}), 135.5 (C_{Ar}), 135.0 (C_{Ar}), 132.0 (CH_{Ar}), 129.9 (CH_{Ar}), 129.7 (CH_{Ar}), 129.2 (CH_{Ar}), 128.9 (CH_{Ar}), 128.6 (CH_{Ar}), 128.5 (CH_{Ar}), 128.1 (CH_{Ar}), 128.0 (CH_{Ar}), 126.6 (C_{Ar}), 125.5 (CH_{Ar}), 122.5 (CH_{Ar}), 113.0 (CH_{Ar}), 105.7 (CH_{Ar}), 67.3 (OCH₂), 66.6 (OCH₂Ph), 46.0 (CH₂CH₂NHCON), 42.7 (OCH₂CH₂), 40.0 (CH₂NHCbz), 38.4 (CH₃), 38.0 (CH₂CH₂NHCON), 29.2 (CH₂CH₂NHCbz), 23.8 (TrizCH₂CH₂), 23.2 (TrizCH₂); **HRMS (ES, positive mode) m/z:** calculated for C₄₁H₄₂N₇O₄S 728.3019; Found 728.3048 (3.91 ppm)

### H') Catalytic hydrogenation

*General procedure.* A solution of the corresponding Cbz protected compounds **9a-l** (0.15 mmol) in THF/MeOH 1:1 (30 mL) containing Pd/C (10%) (20% wt/wt) (20 mg) was hydrogenated (hydrogen balloon) in the presence of TFA (0.5 mL) at room temperature for 2 h. The reaction mixture was filtered (PTFE membrane filters) and the filtrate was evaporated to dryness under reduced pressure. The final residues were purified on a SP1 Isolera Biotage instrument using reverse phase columns to give type **II** compounds **10a-l** (yellow oils) in yields ranging from 13% to 55%.

### 4-(4-Ammoniobutyl)-3-methyl-1-(3-(2-(2-oxoimidazolidin-1-yl)ethoxy)-4-(4-phenylthiazol-2-yl)phenyl)-1H-1,2,3-triazol-3-ium 2,2,2-trifluoroacetate trifluoromethanesulfonate (10a)

**¹H-NMR (CD₃OD, 400 MHz) δ (ppm):** 9.27 (s, 1H, Ar), 8.75 (d, *J* = 8.6 Hz, 1H, Ar), 8.05 (d, *J* = 7.1 Hz, 2H, Ar), 7.97 (s, 1H, Ar), 7.81 (s, 1H, Ar), 7.73 (dd, *J* = 8.2, 2.2 Hz, 1H, Ar), 7.57 - 7.19 (m, 3H, Ar), 4.56 (t, *J* = 5.6 Hz, 2H, OCH₂), 4.38 (s, 3H, CH₃), 3.80 (t, *J* = 5.6 Hz, 2H, OCH₂CH₂), 3.65 (dd, *J* = 9.2, 6.9 Hz, 2H, CH₂CH₂NHCON), 3.40 (dd, *J* = 9.3, 6.9 Hz, 2H, CH₂CH₂NHCON), 3.11 - 2.99 (m, 4H, CH₂NH₃⁺, TrizCH₂), 2.05 - 1.86 (m, 4H, TrizCH₂CH₂, CH₂CH₂NH₃⁺); **¹³C-NMR (CD₃OD, 100 MHz) δ (ppm):** 165.1 (NHCON), 161.1 (OC_{Ar}), 157.5 (C_{Ar}), 156.1 (C_{Ar}), 146.4 (C_{Ar}), 137.3 (C_{Ar}), 135.7 (C_{Ar}), 131.2 (CH_{Ar}), 129.8 (CH_{Ar}), 129.3 (CH_{Ar}), 127.5 (CH_{Ar}), 127.4 (C_{Ar}), 126.5 (CH_{Ar}), 117.0 (CH_{Ar}), 114.8 (CH_{Ar}), 107.0 (CH_{Ar}), 68.5 (OCH₂), 46.8 (CH₂CH₂NHCON), 43.7 (OCH₂CH₂), 40.2 (CH₂NH₃⁺), 39.3 (CH₂CH₂NHCON), 38.5 (CH₃), 27.9 (CH₂CH₂NH₃⁺), 24.7 (TrizCH₂CH₂), 23.8 (TrizCH₂); **HPLC:** Gradient 1, tᵣ = 6.6 min; **HRMS (ES, positive mode) m/z:** calculated for C₂₇H₃₂N₇O₂S 518.2338; Found 518.2354 (3.1 ppm); **Elemental analysis:** calculated for C₃₀H₃₃F₆N₇O₇S₂: C. 46.09; H. 4.26; N. 12.54; S. 8.20; Found: C. 46.31; H. 4.38; N. 12.76; S. 8.24

### 4-(4-Ammoniobutyl)-3-methyl-1-(3-(2-(2-oxoimidazolidin-1-yl)ethoxy)-4-(4-phenethylthiazol-2-yl)phenyl)-1H-1,2,3-triazol-3-ium 2,2,2-trifluoroacetate trifluoromethanesulfonate (10b)

**¹H-NMR (CD₃OD, 500 MHz) δ (ppm):** 9.32 (s, 1H, Ar), 8.61 (d, *J* = 8.6 Hz, 1H, Ar), 7.84 (d, *J* = 2.2 Hz, 1H, Ar), 7.72 (dd, *J* = 8.6, 2.2 Hz, 1H, Ar), 7.35 - 6.98 (m, 6H, Ar), 4.54 (t, *J* = 5.5 Hz, 2H, OCH₂), 4.40 (s, 3H, CH₃), 3.76 (t, *J* = 5.6 Hz, 2H, OCH₂CH₂), 3.58 (dd, *J* = 9.2, 6.8 Hz, 2H, CH₂CH₂NHCON), 3.39 (dd, *J* = 9.2, 6.8 Hz, 2H, CH₂CH₂NHCON), 3.21 - 2.99 (m, 8H, CH₂CH₂Ph, CH₂NH₃⁺, TrizCH₂), 1.97 (m, 2H, TrizCH₂CH₂), 1.85 (m, 2H, CH₂CH₂NH₃⁺); **¹³C-NMR (CD₃OD, 125 MHz) δ (ppm):** 165.1 (NHCON), 160.0 (C_{Ar}), 157.7 (C_{Ar}), 157.5 (C_{Ar}), 146.4 (C_{Ar}), 142.8 (C_{Ar}), 137.3 (C_{Ar}), 131.2 (CH_{Ar}), 129.5 (CH_{Ar}), 129.3 (CH_{Ar}), 127.0 (CH_{Ar}), 126.9 (CH_{Ar}), 123.4 (C_{Ar}), 117.9 (CH_{Ar}), 114.9 (CH_{Ar}), 107.4 (CH_{Ar}), 68.7 (OCH₂), 47.0 (CH₂CH₂NHCON), 43.8 (OCH₂CH₂), 40.3 (CH₂NH₃⁺), 39.3 (CH₂CH₂NHCON), 38.5 (CH₃), 36.6 (CH₂CH₂Ph), 34.2 (CH₂CH₂Ph), 27.9 (CH₂CH₂NH₃⁺), 24.8 (TrizCH₂CH₂), 23.8 (TrizCH₂); **HPLC:** Gradient 1, tᵣ = 6.6 min; **HRMS (ES, positive mode) m/z:** calculated for C₂₉H₃₆N₇O₂S 546.2651; Found 546.2660 (1.59 ppm); **Elemental analysis:** calculated for C₃₂H₃₇F₆N₇O₇ S₂: C. 47.46; H. 4.61; N. 12.11; S. 7.92; Found: C. 47.84; H. 4.73; N. 12.05; S. 7.53

### 4-(4-Ammoniobutyl)-3-methyl-1-(4-(4-(2-(naphthalen-2-yl)ethyl)thiazol-2-yl)-3-(2-(2-oxoimidazolidin-1-yl)ethoxy)phenyl)-1H-1,2,3-triazol-3-ium 2,2,2-trifluoroacetate trifluoromethanesulfonate (10c)

**¹H-NMR (CD₃OD, 400 MHz) δ (ppm):** 9.26 (s, 1H, Ar), 8.58 (d, *J* = 8.6 Hz, 1H, Ar), 7.86 - 7.66 (m, 5H, Ar), 7.62 (s, 1H, Ar), 7.46 - 7.30 (m, 3H, Ar), 7.22 (s, 1H, Ar), 4.50 (t, *J* = 5.7 Hz, 2H, OCH₂), 4.38 (s, 3H, CH₃), 3.73 (t, *J* = 5.5 Hz, 2H, OCH₂CH₂), 3.56 (dd, *J* = 9.0, 6.7 Hz, 2H, CH₂CH₂NHCON), 3.31 (m, 2H, CH₂CH₂NHCON), 3.24 - 3.22 (m, 4H CH₂CH₂Napth), 3.09 - 3.00 (m, 4H, CH₂NH₃⁺, TrizCH₂), 2.03 (m, 2H, TrizCH₂CH₂), 1.85 (m, 2H, CH₂CH₂NH₃⁺); **¹³C-NMR (CD₃OD, 100 MHz) δ (ppm):** 165.0 (NHCON), 160.9 (OC_{Ar}), 157.4 (C_{Ar}), 157.3 (C_{Ar}), 146.4 (C_{Ar}), 140.3 (C_{Ar}), 137.3 (C_{Ar}), 135.1 (C_{Ar}), 133.6 (CH_{Ar}), 131.0 (CH_{Ar}), 128.9 (CH_{Ar}), 128.6 (CH_{Ar}), 128.5 (C_{Ar}), 128.3 (CH_{Ar}), 127.6 (CH_{Ar}), 126.9 (CH_{Ar}), 126.5 (CH_{Ar}), 126.2 (CH_{Ar}), 118.0 (CH_{Ar}), 114.8 (CH_{Ar}), 107.2 (CH_{Ar}), 68.6 (OCH₂), 46.8 (CH₂CH₂NHCON), 43.6 (OCH₂CH₂), 40.2 (CH₂NH₃⁺), 39.2 (CH₂CH₂NHCON), 38.5 (CH₃), 36.7 (CH₂CH₂Napth), 34.0 (CH₂CH₂Napth), 27.8 (CH₂CH₂NH₃⁺), 24.6 (TrizCH₂CH₂), 23.8 (TrizCH₂); **HPLC:** Gradient 1, tᵣ = 7.1 min; **HRMS (ES, positive mode) m/z:** calculated for C₃₃H₃₈N₇O₂S 596.2808; Found 596.2787 (-3.4 ppm); **Elemental analysis:** calculated for C₃₆H₃₉F₆N₇O₇S₂: C. 50.29; H. 4.57; N. 11.40; S. 7.46; Found: C. 50.05; H. 5.08; N. 10.92; S. 7.83

### 4-(4-Ammoniobutyl)-1-(4-(4-(2-(2,3-dihydrobenzofuran-5-yl)ethyl)thiazol-2-yl)-3-(2-(2-oxoimidazolidin-1-yl)ethoxy)phenyl)-3-methyl-1H-1,2,3-triazol-3-ium 2,2,2-trifluoroacetate trifluoromethanesulfonate (10d)

**¹H-NMR (DMSO-d₆, 400 MHz) δ (ppm):** 9.54 (s, 1H, Ar), 8.61 (d, *J* = 8.7 Hz, 1H, Ar), 7.89 (d, *J* = 2.2 Hz, 1H, Ar), 7.75 (dd, *J* = 8.7, 2.1 Hz, 1H, Ar), 7.65 (bs, 3H, NH₃⁺), 7.48 (s, 1H, Ar), 7.13 (s, 1H, Ar), 6.94 (dd, *J* = 8.1, 1.9 Hz, 1H, Ar), 6.65 (d, *J* = 8.1 Hz, 1H, Ar), 6.41 (bs, 1H, NH), 4.56 - 4.41 (m, 4H, OCH₂CH₂N, OCH₂CH₂Ph), 4.37 (s, 3H, CH₃), 3.66 (t, *J* = 5.5 Hz, 2H, OCH₂CH₂N), 3.50 (dd, *J* = 9.0, 6.7 Hz, 2H, CH₂CH₂NHCON), 3.23 (dd, *J* = 9.0, 6.7 Hz, 2H, CH₂CH₂NHCON), 3.13 (t, *J* = 8.7 Hz, 2H, OCH₂CH₂Ph), 3.08 - 2.72 (m, 8H, ThiazCH₂CH₂, CH₂NH₃⁺, TrizCH₂), 1.82 (quin, *J* = 7.6 Hz, 2H, TrizCH₂CH₂), 1.71 (quin, *J* = 8.1 Hz, 2H, CH₂CH2NH₃⁺); **¹³C-NMR (DMSO-d₆, 100 MHz) δ (ppm):** 162.2 (NHCON), 158.5 (OCAr), 157.9 (OCAr), 156.0 (CAr), 155.5 (C_{Ar}), 144.8 (C_{Ar}), 135.5 (C_{Ar}), 133.1 (C_{Ar}), 129.4 (CH_{Ar}), 127.5 (CH_{Ar}), 127.2 (CH_{Ar}), 126.5 (CH_{Ar}), 125.5 (C_{Ar}), 124.2 (CH_{Ar}), 122.3 (CH_{Ar}), 119.1 (C_{Ar}), 117.1 (C_{Ar}), 113.4 (C_{Ar}), 108.5 (CH_{Ar}), 106.1 (CH_{Ar}), 70.7 (OCH₂CH₂Ph), 68.1 (OCH₂CH₂N), 45.4 (CH₂CH₂NHCON), 42.4 (OCH₂CH₂N), 38.4 (CH₃), 37.9 (CH₂NH₃⁺), 37.6 (CH₂CH₂NHCON), 34.3 (ThiazCH₂CH₂), 33.2 (ThiazCH₂CH₂), 29.2 (OCH₂CH₂Ph), 26.3 (CH₂CH₂NH₃⁺), 23.0 (TrizCH₂CH₂), 22.0 (TrizCH₂); **HPLC:** Gradient 1, tᵣ = 6.4 min; **HRMS (ES, positive mode) m/z:** calculated for C₃₁H₃₈N₇O₃S 588.2757; Found 588.2765 (1.4 ppm); **Elemental analysis:** calculated for C₃₄H₃₉F₆N₇O₈S₂: C. 47.94; H. 4.61; N. 11.51; S. 7.53; Found: C. 47.57; H. 4.85; N. 11.16; S. 7.46

### 1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-methyl-1H-1,2,3-triazol-3-ium 2,2,2-trifluoroacetate trifluoromethanesulfonate (10e)

**¹H-NMR (CD₃OD, 400 MHz) δ (ppm):** 9.28 (s, 1H, Ar), 8.61 (d, *J* = 8.6 Hz, 1H, Ar), 7.82 (d, *J* = 2.2 Hz, 1H, Ar), 7.71 (dd, *J* = 8.6, 2.2 Hz, 1H, Ar), 7.60 - 7.54 (m, 2H, Ar), 7.52 - 7.49 (m, 2H, Ar), 7.46 - 7.37 (m, 2H, Ar), 7.33 - 7.22 (m, 4H, Ar), 4.53 (t, *J* = 5.6 Hz, 2H, OCH₂), 4.39 (s, CH₃), 3.76 (t, *J* = 5.6 Hz, 2H, OCH₂CH₂), 3.60 (dd, *J* = 9.1, 6.8 Hz, 2H, CH₂CH₂NHCON), 3.35 (dd, *J =* 9.3, 6.9 Hz, 2H, CH₂CH₂NHCON), 3.27 - 3.09 (m, 4H, CH₂CH₂BiPh), 3.09 - 3.02 (m, 4H, CH₂NH₃⁺, TrizCH₂), 2.02 - 1.85 (m, 4H, TrizCH₂CH₂, CH₂CH₂NH₃⁺); **¹³C-NMR (CD₃OD, 100 MHz) δ (ppm):** 165.1 (NHCON), 160.9 (OC_{Ar}), 157.5 (C_{Ar}), 157.4 (C_{Ar}), 146.3 (C_{Ar}), 142.3 (C_{Ar}), 141.9 (C_{Ar}), 140.2 (C_{Ar}), 137.3 (C_{Ar}), 131.0 (CH_{Ar}), 130.0 (CH_{Ar}), 129.8 (CH_{Ar}), 128.1 (CH_{Ar}), 127.9 (CH_{Ar}), 127.8 (CH_{Ar}), 126.6 (C_{Ar}), 120.2 (CH_{Ar}), 118.0 (CH_{Ar}), 114.8 (CH_{Ar}), 107.2 (CH_{Ar}), 68.6 (OCH₂), 46.8 (CH₂CH₂NHCON), 43.6 (OCH₂CH₂), 40.2 (CH₂NH₃⁺), 39.3 (CH₂CH₂NHCON), 38.5 (CH₃), 36.2 (CH₂CH₂Ph), 34.1 (CH₂CH₂Ph), 27.9 (CH₂CH₂NH₃⁺), 24.7 (TrizCH₂CH₂), 23.7 (TrizCH₂); **HPLC:** Gradient 1, tᵣ = 7.5 min; **HRMS (ES, positive mode) m/z:** calculated for C₃₅H₄₀N₇O₂S 622.2964; Found 622.2952 (-2.02 ppm); **Elemental analysis:** calculated for C₃₈H₄₁F₆N₇O₇S₂: C. 51.52; H. 4.67; N. 11.07; S. 7.24; Found: C. 51.88; H. 5.25; N. 10.78; S. 7.61

### 1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-ethyl-1H-1,2,3-triazol-3-ium 2,2,2-trifluoroacetate trifluoromethanesulfonate (10f)

**¹H-NMR (CD₃OD, 400 MHz) δ (ppm):** 9.26 (s, 1H, Ar), 8.62 (d, *J* = 8.6 Hz, 1H, Ar), 7.84 (d, *J* = 2.2 Hz, 1H, Ar), 7.72 (dd, *J* = 8.6, 2.1 Hz, 1H, Ar), 7.55 - 7.50 (m, 2H, Ar), 7.49 - 7.44 (m, 2H, Ar), 7.43 - 7.39 (m, 2H, Ar), 7.35 - 7.25 (m, 4H, Ar), 4.74 (q, *J* = 7.3 Hz, 2H, N⁺CH₂CH₃), 4.55 (t, *J* = 5.6 Hz, 2H, OCH₂), 3.77 (t, *J* = 5.5 Hz, 2H, OCH₂CH₂), 3.61 (dd, *J* = 9.3, 6.9 Hz, 2H, CH₂CH₂NHCON), 3.33 (dd, *J* = 9.3, 6.9 Hz, 2H, CH₂CH₂NHCON), 3.27 - 3.12 (m, 4H, CH₂CH₂BiPh), 3.12 - 3.04 (m, 4H, CH₂NH₃⁺, TrizCH₂), 2.06 - 1.85 (m, 4H, TrizCH₂CH₂, CH₂CH₂NH₃⁺), 1.74 (t, *J* = 7.3 Hz, 3H, CH₃); **¹³C-NMR (CD₃OD, 100 MHz) δ (ppm):** 165.1 (NHCON), 161.0 (OC_{Ar}), 157.5 (C_{Ar}), 157.4 (C_{Ar}), 145.9 (C_{Ar}), 142.3 (C_{Ar}), 141.9 (C_{Ar}), 140.2 (C_{Ar}), 137.4 (C_{Ar}), 131.1 (CH_{Ar}), 130.1 (CH_{Ar}), 129.8 (CH_{Ar}), 128.1 (CH_{Ar}), 127.9 (CH_{Ar}), 127.8 (CH_{Ar}), 127.7 (C_{Ar}), 126.6 (CH_{Ar}), 118.0 (CH_{Ar}), 114.9 (CH_{Ar}), 107.2 (CH_{Ar}), 68.6 (OCH₂), 46.9 (CH₂CH₂NHCON), 43.6 (OCH₂CH₂), 40.2 (CH₂NH₃⁺), 39.3 (CH₂CH₂NHCON), 36.3 (CH₂CH₂Ph), 34.2 (CH₂CH₂Ph), 27.9 (CH₂CH₂NH₃⁺), 24.9 (TrizCH₂CH₂), 23.7 (TrizCH₂), 14.1 (CH₃); **HPLC:** Gradient 1, tᵣ = 7.8 min; **HRMS (ES, positive mode) m/z:** calculated for C₃₆H₄₂N₇O₂S 636.3121; Found 636.3132 (1.71 ppm); **Elemental analysis:** calculated for C₃₉H₄₃F₆N₇O₇S₂: C. 52.05; H. 4.82; N. 10.90; S. 7.13; Found: C. 51.86; H. 4.87; N. 10.50; S. 7.26

### 4-(4-Ammoniobutyl)-1-(4-(4-(4-methoxyphenyl)thiazol-2-yl)-3-(2-(2-oxoimidazolidin-1-yl)ethoxy)phenyl)-3-methyl-1H-1,2,3-triazol-3-ium 2,2,2-trifluoroacetate trifluoromethanesulfonate (10g)

**¹H-NMR (CD₃OD, 400 MHz) δ (ppm):** 9.07 (s, 1H, Ar), 8.50 (d, *J* = 8.5 Hz, 1H, Ar), 7.83 (d, *J* = 8.7 Hz, 2H, Ar), 7.66 (s, 1H, Ar), 7.59 - 7.47 (m, 2H, Ar), 6.90 (d, *J* = 8.7 Hz, 2H, Ar), 4.38 (t, *J* = 5.7 Hz, 2H, OCH₂), 4.23 (s, 3H, N⁺CH₃), 3.76 (s, 3H, OCH₃), 3.67 (t, *J* = 5.6 Hz, 2H, OCH₂CH₂), 3.53 (dd, *J* = 9.3, 6.9 Hz, 2H, CH₂CH₂NHCON), 3.30 (dd, *J* = 9.0, 6.9 Hz, 2H, CH₂CH₂NHCON), 2.97 (t, *J* = 7.4 Hz, 2H, CH₂NH₃⁺), 2.87 (t, *J* = 7.5 Hz, 2H, TrizCH₂), 1.90 - 1.71 (m, 4H, CH₂CH₂NH₃⁺, TrizCH₂CH₂); **¹³C-NMR (CD₃OD, 75 MHz) δ (ppm):** 165.1 (NHCON), 161.3 (OCAr), 160.7 (C_{Ar}), 157.2 (C_{Ar}), 155.7 (C_{Ar}), 146.4 (C_{Ar}), 137.0 (C_{Ar}), 130.9 (C_{Ar}), 128.7 (CH_{Ar}), 128.5 (CH_{Ar}), 127.2 (CH_{Ar}), 126.2 (C_{Ar}), 115.7 (CH_{Ar}), 115.2 (CH_{Ar}), 114.4 (CH_{Ar}), 106.5 (CH_{Ar}), 68.4 (OCH₂), 55.9 (OCH₃), 46.8 (CH₂CH₂NHCON), 43.6 (OCH₂CH₂), 40.2 (CH₂NH₃⁺), 39.3 (CH₂CH₂NHCON), 38.4 (N⁺CH₃), 27.9 (CH₂CH₂NH₃⁺), 24.6 (TrizCH₂CH₂), 23.8 (TrizCH₂); **HRMS (ES, positive mode) m/z:** calculated for C₂₈H₃₄N₇O₃S 548.2444; Found 548.2439 (-0.88 ppm); **Elemental analysis:** calculated for C₃₁H₃₅F₆N₇O₈S₂: C. 45.87; H. 4.35; N. 12.08; S. 7.90; Found: C. 45.88; H. 4.40; N. 12.11; S. 7.99

### 4-(4-Ammoniobutyl)-1-(4-(4-(4-(diethylamino)phenyl)thiazol-2-yl)-3-(2-(2-oxoimidazolidin-1-yl)ethoxy)phenyl)-3-methyl-1H-1,2,3-triazol-3-ium 2,2,2-trifluoroacetate trifluoromethanesulfonate (10h)

**¹H-NMR (CD₃OD, 400 MHz) δ (ppm):** 8.76 (d, *J* = 8.6 Hz, 1H, Ar), 7.89 - 7.80 (m, 3H, Ar), 7.44 (t, *J* = 7.5 Hz, 2H, Ar), 7.74 (dd, *J* = 8.6, 2.1 Hz, 1H, Ar), 7.67 (s, 1H, Ar), 6.78 (d, *J* = 9.0 Hz, 1H, Ar), 4.47 (t, *J* = 5.5 Hz, 2H, OCH₂), 4.40 (s, 3H, CH₃), 3.80 (t, *J* = 5.5 Hz, 2H, OCH₂CH₂), 3.66 (dd, *J* = 9.3, 6.9 Hz, 2H, CH₂CH₂NHCON), 3.44 (q, J = 7.1 Hz, 4H, N(CH₂CH₃)₂), 3.39 (dd, *J* = 9.2, 6.9 Hz, 2H, CH₂CH₂NHCON), 3.03 (t, *J* = 7.7 Hz, 2H, CH₂NH₂), 2.94 (t, *J* = 7.4 Hz, 2H, TrizCH₂), 1.93 (m, 2H, TrizCH₂CH₂), 1.80 (quin, *J* = 7.7 Hz, 2H, CH₂CH₂NH₂), 1.20 (t, *J* = 7.0 Hz, 6H, N(CH₂CH₃)₂); **¹³C-NMR (CD₃OD, 100 MHz) δ (ppm):** 165.1 (NHCON), 160.5 (OC_{Ar}), 157.5 (C_{Ar}), 157.2 (C_{Ar}), 149.4 (C_{Ar}), 146.4 (C_{Ar}), 137.1 (C_{Ar}), 131.3 (CH_{Ar}), 128.6 (CH_{Ar}), 127.0 (C_{Ar}), 123.4 (C_{Ar}), 114.8 (CH_{Ar}), 113.3 (CH_{Ar}), 113.1 (CH_{Ar}), 107.2 (CH_{Ar}), 68.7 (OCH₂), 47.0 (CH₂CH₂NHCON), 43.8 (OCH₂CH₂), 40.3 (CH₂NH₂), 39.3 (CH₂CH₂NHCON), 38.5 (CH₃), 30.7 (CH₂CH₂NH₂), 28.2 (N(CH₂CH₃)₂), 24.8 (TrizCH₂CH₂), 23.8 (TrizCH₂), 13.0 (N(CH₂CH₃)₂)

### 4-(3-Ammoniopropyl)-3-methyl-1-(3-(2-(2-oxoimidazolidin-1-yl)ethoxy)-4-(4-phenethylthiazol-2-yl)phenyl)-1H-1,2,3-triazol-3-ium 2,2,2-trifluoroacetate trifluoromethanesulfonate (10i)

**¹H-NMR (CD₃OD, 400 MHz) δ (ppm):** 9.29 (s, 1H, Ar), 8.61 (d, *J* = 8.6 Hz, 1H, Ar), 7.84 (d, *J* = 2.2 Hz, 1H, Ar), 7.72 (dd, *J* = 8.6, 2.2 Hz, 1H, Ar), 7.31 - 6.92 (m, 6H, Ar), 4.54 (t, *J* = 5.6 Hz, 2H, OCH₂), 4.41 (s, 3H, CH₃), 3.76 (t, *J* = 5.6 Hz, 2H, OCH₂CH₂), 3.61 (dd, *J* = 9.3, 6.9 Hz, 2H, CH₂CH₂NHCON), 3.37 (dd, *J* = 9.2, 6.9 Hz, 2H, CH₂CH₂NHCON), 3.22 - 2.92 (m, 8H, CH₂CH₂Ph, CH₂NH₃⁺, TrizCH₂), 2.25 (quin, *J* = 7.8 Hz, 2H, CH₂CH₂NH₃⁺); **¹³C-NMR (CD₃OD, 75 MHz) δ (ppm):** 165.1 (NHCON), 160.0 (OC_{Ar}), 157.5 (C_{Ar}), 157.4 (C_{Ar}), 145.5 (C_{Ar}), 142.7 (C_{Ar}), 137.3 (C_{Ar}), 131.0 (CH_{Ar}), 129.5 (CH_{Ar}), 129.4 (CH_{Ar}), 127.8 (CH_{Ar}), 127.0 (CH_{Ar}), 126.6 (C_{Ar}), 118.0 (CH_{Ar}), 114.9 (CH_{Ar}), 107.3 (CH_{Ar}), 68.6 (OCH₂), 46.8 (CH₂CH₂NHCON), 43.6 (OCH₂CH₂), 39.7 (CH₂NH₃⁺), 39.3 (CH₂CH₂NHCON), 38.6 (CH₃), 36.6 (CH₂CH₂Ph), 34.3 (CH₂CH₂Ph), 25.7 (TrizCH₂CH₂), 21.5 (TrizCH₂); **HPLC:** Gradient 1, tᵣ = 8.3 min; **HRMS (ES, positive mode) m/z:** calculated for C₂₈H₃₄N₇O₂S 532.2495; Found 532.2500 (1.07 ppm); **Elemental analysis:** calculated for C₃₁H₃₅F₆N₇O₇S₂: C. 46.79; H. 4.43; N. 12.32; S. 8.06; Found: C. 46.86; H. 4.87; N. 12.35; S. 8.05

### 1-(4-(4-(2-([1,1'-Biphenyl]-4-yl)ethyl)thiazol-2-yl)-3-(2-(2-oxoimidazolidin-1-yl)ethoxy)phenyl)-4-(3-ammoniopropyl)-3-methyl-1H-1,2,3-triazol-3-ium 2,2,2-trifluoroacetate trifluoromethanesulfonate (10j)

**¹H-NMR (DMSO-d₆, 400 MHz) δ (ppm):** 9.52 (s, 1H, Ar), 8.63 (d, *J* = 8.6 Hz, 1H, Ar), 7.89 (d, *J* = 2.2 Hz, 1H, Ar), 7.81 - 7.68 (m, 3H, Ar), 7.66 - 7.54 (m, 4H, Ar), 7.45 (t, *J* = 7.5 Hz, 2H, Ar), 7.42 - 7.17 (m, 2H, Ar), 6.41 (bs, 1H, NH), 4.50 (t, *J* = 5.6 Hz, 2H, OCH₂), 4.35 (s, 3H, CH₃), 3.66 (t, *J* = 5.6 Hz, 2H, OCH₂CH₂), 3.50 (dd, *J* = 9.0, 6.7 Hz, 2H, CH₂CH₂NHCON), 3.23 (dd, *J* = 8.9, 6.8 Hz, 2H, CH₂CH₂NHCON), 3.09 - 2.95 (m, 8H, CH₂CH₂BiPh, CH₂NH₃⁺, TrizCH₂), 2.04 (quin, *J* = 7.5 Hz, 2H, CH₂CH₂NH₃⁺); **¹³C-NMR (DMSO-d₆, 75 MHz) δ (ppm):** 162.2 (NHCON), 158.5 (C_{Ar}), 155.8 (C_{Ar}), 155.5 (C_{Ar}), 143.9 (C_{Ar}), 140.6 (C_{Ar}), 140.0 (C_{Ar}), 137.8 (C_{Ar}), 135.6 (C_{Ar}), 129.4 (CH_{Ar}), 129.0 (CH_{Ar}), 128.9 (CH_{Ar}), 127.2 (CH_{Ar}), 126.7 (CH_{Ar}), 126.6 (CH_{Ar}), 124.2 (C_{Ar}), 122.0 (CH_{Ar}), 119.4 (CH_{Ar}), 113.5 (CH_{Ar}), 106.1 (CH_{Ar}), 68.1 (OCH₂), 45.5 (CH₂CH₂NHCON), 42.4 (OCH₂CH₂), 38.0 (CH₂NH₃⁺), 37.5 (CH₂CH₂NHCON), 37.6 (CH₃), 34.3 (CH₂CH₂Ph), 32.5 (CH₂CH₂Ph), 24.2 (TrizCH₂CH₂), 19.9 (TrizCH₂); **HPLC:** Gradient 1, tᵣ = 9.7 min; **HRMS (ES, positive mode) m/z:** calculated for C₃₄H₃₈N₇O₂S 608.2808; Found 608.2814 (0.99 ppm); **Elemental analysis:** calculated for C₃₇H₃₉F₆N₇O₇S₂: C. 50.97; H. 4.51; N. 11.25; S. 7.35; Found: C. 50.32; H. 4.83; N. 11.38; S. 7.34

### Mono (4-(2-(5-(4-(4-ammoniobutyl)-3-methyl-1H-1,2,3-triazol-3-ium-1-yl)-2-(4-phenethylthiazol-2-yl)phenoxy)ethyl)piperidin-1-ium) mono(2,2,2-trifluoroacetate) mono(trifluoromethanesulfonate) (10k)

**¹H-NMR (CD₃OD, 400 MHz) δ (ppm):** 9.36 (s, 1H, Ar), 8.63 (d, *J* = 8.6 Hz, 1H, Ar), 7.84 (d, *J* = 2.2 Hz, 1H, Ar), 7.72 (dd, *J* = 8.6, 2.2 Hz, 1H, Ar), 7.35 - 7.07 (m, 6H, Ar), 4.48 (t, *J* = 5.7 Hz, 2H, OCH₂), 4.40 (s, 3H, CH₃), 3.41 (d, *J* = 12.9 Hz, 2H, CHH'(NH₂⁺)CHH'), 3.25 - 2.89 (m, 10H, CH₂CH₂Ph, CHH'(NH₂⁺)CHH', CH₂NH₃⁺, TrizCH₂), 2.20 - 1.71 (m, 9H, CH, OCH₂CH₂, CHH'(CH)CHH,' CH₂CH₂NH₃⁺, TrizCH₂CH₂), 1.53 (m, 2H, CHH'(CH)CHH'); **¹³C-NMR (CD₃OD, 100 MHz) δ (ppm):** 160.0 (OC_{Ar}), 157.8 (C_{Ar}), 157.6 (C_{Ar}), 146.5 (C_{Ar}), 146.4 (C_{Ar}), 142.7, 131.0 (CH_{Ar}), 129.5 (CH_{Ar}), 129.4 (CH_{Ar}), 127.7 (CH_{Ar}), 127.0 (C_{Ar}), 126.4 (CH_{Ar}), 117.7 (CH_{Ar}), 114.5 (CH_{Ar}), 107.0 (CH_{Ar}), 68.7 (OCH₂), 45.3 (CH₂(NH₂⁺)CH₂), 40.1 (CH₂NH₃⁺), 38.4 (CH₃), 36.7 (CH₂CH₂Ph), 36.1 (OCH₂CH₂), 34.3 (CH₂CH₂Ph), 32.2 (CH), 29.8 (CH₂(CH)CH₂), 27.9 (CH₂CH₂NH₃⁺), 24.7 (TrizCH₂CH₂), 23.8 (TrizCH₂); **HPLC:** Gradient 1, tᵣ = 6.1 min; **HRMS (ES, positive mode) m/z:** calculated for C₃₁H₄₁N₆OS 545.3063; Found 545.3067 (0.85 ppm); **Elemental analysis:** calculated for C₃₆H₄₃F₉N₆O₈S₂: C. 46.85; H. 4.70; N. 9.11; S. 6.95; Found: C. 46.53; H. 4.69; N. 8.69; S. 6.69

### 4-(4-Ammoniobutyl)-1-(4-(4,5-diphenylthiazol-2-yl)-3-(2-(2-oxoimidazolidin-1-yl)ethoxy)phenyl)-3-methyl-1H-1,2,3-triazol-3-ium 2,2,2-trifluoroacetate trifluoromethanesulfonate (10I)

**¹H-NMR (CD₃OD, 500 MHz) δ (ppm):** 9.28 (s, 1H, Ar), 8.70 (d, *J* = 8.7 Hz, 1H, Ar), 7.86 (d, *J* = 2.2 Hz, 1H, Ar), 7.73 (dd, *J* = 8.6, 2.1 Hz, 1H, Ar), 7.59 - 7.53 (m, 2H, Ar), 7.45 - 7.25 (m, 8H, Ar), 4.58 (t, *J* = 5.6 Hz, 2H, OCH₂), 4.39 (s, 3H, CH₃), 3.78 (t, *J* = 5.5 Hz, 2H, OCH₂CH₂), 3.63 (dd, *J* = 9.1, 6.7 Hz, 2H, CH₂CH₂NHCON), 3.37 (dd, J = 9.0, 7.2 Hz, CH₂CH₂NHCON), 3.12 - 3.00 (m, 4H, TrizCH₂, CH₂NH₃⁺), 1.99 (quin, *J* = 8.5 Hz, 2H, TrizCH₂CH₂), 1.89 (m, 2H, CH₂CH₂NH₃⁺); **¹³C-NMR (CD₃OD, 125 MHz) δ (ppm):** 165.0 (NHCON), 159.0 (OC_{Ar}), 157.4 (C_{Ar}), 151.0 (C_{Ar}), 146.5 (C_{Ar}), 137.5 (CH_{Ar}), 136.8 (CH_{Ar}), 136.2 (CH_{Ar}), 133.1 (CH_{Ar}), 130.8 (CH_{Ar}), 130.7 (CH_{Ar}), 130.2 (CH_{Ar}), 130.0 (C_{Ar}), 129.5 (CH_{Ar}), 129.4 (CH_{Ar}), 129.1 (CH_{Ar}), 114.9 (CH_{Ar}), 107.2 (CH_{Ar}), 68.4 (OCH₂), 46.7 (CH₂CH₂NHCON), 43.7 (OCH₂CH₂), 40.2 (CH₂NH₃⁺), 39.3 (CH₂CH₂NHCON), 38.5 (CH₃), 27.9 (CH₂CH₂NH₃⁺), 24.7 (TrizCH₂CH₂), 23.8 (TrizCH₂); **HRMS (ES, positive mode) m/z:** calculated for C₃₃H₃₆N₇O₂S 594.2651; Found 594.2649 (-0.43 ppm); **Elemental analysis:** calculated for C₃₆H₃₇F₆N₇O₇S₂: C. 50.40; H. 4.35; N. 11.43; S. 7.47; Found: C. 49.86; H. 4.31; N. 11.63; S. 7.36

### Example 3. Biological evaluation

### 3.2.1. Enzymatic assays

### Li-TryR oxidoreductase activity

Oxidoreductase activity was determined according to the method described by Hamilton et al. (Biochem. J., 2003, 369, 529-537). Briefly, reactions were carried out at 26 °C (250 µL) of HEPES pH 8.0 (40 mM) buffer containing EDTA (1 mM), NADPH (150 µM), NADP+ (30 µM), DTNB (25 µM), T[S]₂ (1 µM), glicerol (0.02%), DMSO (1.5%) and recombinant Li-TryR (7 nM). For IC₅₀ determinations the enzyme was pre-incubated with the peptides (concentrations ranging from 75 µM to 0.29 µM) for 10 min prior the addition of T[S]₂ and NADPH. Enzyme activity was monitored by the increase in absorbance at 412 nm for 1 h at 26 °C in a VERSAmax microplate reader (Molecular Devices, California, USA). All the assays were conducted in triplicate in at least three independent experiments. Data were analyzed using a non-linear regression model with the Grafit6 software (Erithacus, Horley, Surrey, UK).

### Dimer quantitation assay

The stability of the Li-TryR dimeric form in the presence of the compounds was evaluated using an Enzyme-Linked ImmunoSorbent Assay (ELISA). Briefly a dual (HIS/FLAG) tagged Li-TryR (400 nM) was incubated in a dimerization buffer (200 µL 300 mM NaCl, 50 mM Tris pH 8.0) for 16 h at 37 °C with agitation and in a humid atmosphere in the presence of different peptide concentration (10 to 90 µM) in 1.5ml tubes. Next, the tubes were centrifuged at 30000 rcf and supernatants were disposed in anti-flag coated 96 well plates and incubated 30 min, at 37°C with gentle shaking. Then the plates were washed ten times with TTBS (Tween 0.1%, 2 mM Tris, 138 mM NaCl 138 pH 7.6) and incubated with diluted monoclonal α-HIS HRP conjugated antibody (200 µL, Abcam, Cambridge, UK) in BSA (5%) in TTBS for 1 h at room temperature. The plates were washed once again as previously described and 1,2-phenylenediamine dihydrochloride (OPD) substrate (100 µL, Dako, Glostrup, Denmark) prepared according to manufacturer's instructions was added. The enzymatic reaction was stopped after 10 min with H₂SO₄ (100 µL, 0.5 M) and the absorbances were measured at 490 nm in a VERSAmax microplate reader (Molecular Devices, California, USA). All the assays were conducted in triplicate in at least three independent experiments. Data were analyzed using a non-linear regression model with the Grafit6 software (Erithacus, Horley, Surrey, UK).

**Table 1. IC₅₀ ± SEM values for target compounds of formula I-A and I-B in the oxidoreductase activity assays and in the Li-TryR monomer displacement**

| | | | | | | | |
|---|---|---|---|---|---|---|---|
| 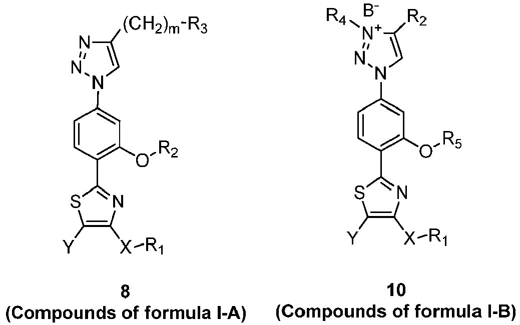 | | | | | | | |

| **COMPOUNDS OF FORMULA I-A (8)** | | | | | | | |
|---|---|---|---|---|---|---|---|
| **Comp. (code)** | **(CH₂)ₘ-R₃** | **R₂** | **X-R₁** | | **Y** | **IC₅₀ act (µM)^{a}** | **IC₅₀ dim (µM)^{b}** |
| **8a** or **model comp. A** (TRL133) | (CH₂)₄NH₃⁺ | 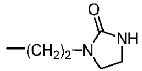 | OiPr | | H | 50.25±2.37 | > 75 |
| **8b** (TRL145) | (CH₂)₄NH₃⁺ | 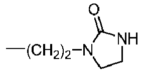 | CH₂^{t}Su | | H | 17.22±1.69 | > 75 |
| **8c** (TRL191) | (CH₂)₄NH₃⁺ | 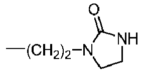 | Ph | | H | 23.97±1.69 | 29.02±1.51 |
| **8d** (TRL139) | (CH₂)₄NH₃⁺ | 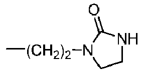 | CH₂Ph | | H | 43.64±1.15 | 55.26±2.39 |
| **8e** (TRL146) | (CH₂)₄NH₃⁺ | 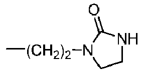 | (CH₂)₂Ph | | H | 14.6±0.95 | 44.13±1.87 |
| **8f** (TRL176) | (CH₂)₄NH₃⁺ | 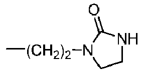 | (CH₂)₃Ph | | H | 14.58±1.73 | 19.07±1.06 |
| **8g** (TRL148) | (CH₂)₄NH₃⁺ | 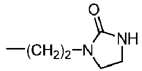 | (CH₂)₂Naphthyl | | H | 6.22±1.01 | 11.18±0.41 |
| **8h** (TRL159) | (CH₂)₄NH₃ ⁺ | 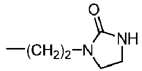 | (CH₂)₂Biphenyl | | H | 11.59±2.05 | 5.94±0.99 |
| **8j** (TRL165) | (CH₂)₄NH₃⁺ | 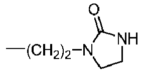 | (CH₂)₂Dihydrobe nzofuranyl | | H | 11.88±0.58 | 10.34±0.52 |
| **8l** (TRL190) | (CH₂)₄NH₃⁺ | 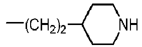 | (CH₂)₂Ph | | H | 5.78 ± 0.89 | > 75 |
| **8m** (TRL157) | (CH₂)₄NH₃⁺ | Me | (CH₂)₂Ph | | H | 17.52±2.91 | 89.22±29.65 |
| **8n** (TRL177) | (CH₂)₃NH₃⁺ | 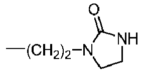 | (CH₂)₂Ph | | H | 41.21±8.48 | > 75 |
| **80** (TRL178) | (CH₂)₃NH₃⁺ | 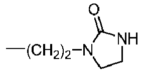 | (CH₂)₂Biphenyl | | H | 21.80±5.49 | 11.09±0.44 |
| **8p** (TRL188) | (CH₂)₄NH₃⁺ | 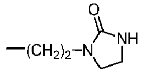 | Ph | | Ph | 6.07±1.01 | 4.15±1.92 |

| **COMPOUNDS OF FORMULA I-B (10)** | | | | | | | |
|---|---|---|---|---|---|---|---|
| **Comp. (code)** | **(CH₂)ₘ -R₃** | **R₂** | **X-R₁** | **Y** | **R₄** | **IC₅₀ act (µM)^{a}** | **IC₅₀ dim (µM)^{b}** |
| **10a** (TRL192) | (CH₂)₄ NH₃⁺ | 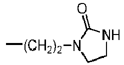 | Ph | H | Me | 22.80 ±0.98 | > 75 |
| **10b** (TRL164) | (CH₂)₄ NH₃⁺ | 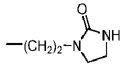 | (CH₂)₂Ph | H | Me | 12.86±0.98 | > 75 |
| **10c** (TRL158) | (CH₂)₄ NH₃⁺ | 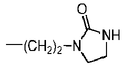 | (CH₂)₂Naphthyl | H | Me | 2.67±0.13 | 82.7±2.4 |
| **10d** (TRL169) | (CH₂)₄ NH₃⁺ | 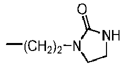 | (CH₂)₂Dihydrobe nzofuranyl | H | Me | 5.42±0.23 | > 75 |
| **10e** (TRL160) | (CH₂)₄ NH₃⁺ | 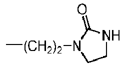 | (CH₂)₂Biphenyl | H | Me | 5.19±0.82 | 23.79±0.96 |
| **10f** (TRL168) | (CH₂)₄ NH₃⁺ | 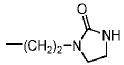 | (CH₂)₂Biphenyl | H | Et | 3.88±0.55 | 87.06±0.1 |
| **10i** (TRL179) | (CH₂)₃ NH₃⁺ | 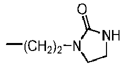 | (CH₂)₂Ph | H | Me | 21.52±2.87 | > 75 |
| **10j** (TRL180) | (CH₂)₃ NH₃⁺ | 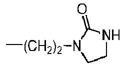 | (CH₂)₂Biphenyl | H | Me | 6.99±0.59 | 30.20±3.47 |
| **10k** (TRL187) | (CH₂)₄ NH₃⁺ | 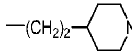 | (CH₂)₂Ph | H | Me | 3.35±0.3 | > 75 |
| **10l** (TRL189) | (CH₂)₄ NH₃⁺ | 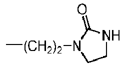 | Ph | Ph | Me | 9.31±0.92 | > 75 |

| | | | | | | | |
|---|---|---|---|---|---|---|---|
| ^{a} Enzymatic activity > 75 indicates that the IC₅₀ is higher than 75 µM (maximum assayed). Results are representative of three independent experiments each performed in triplicate. ^{b} Dimer quantitation assay (ELISA) is described in ChemBioChem, 2013, 14, 1212-1217. | | | | | | | |

### 3.2.2. In vitro anti-leishmanial activity

### Leishmanicidal activity

*L. infantum* axenic amastigotes were grown in M199 (Invitrogen, Leiden, The Netherlands) medium supplemented with 10% heat inactivated FCS, 1 g/L β-alanine, 100 mg/L L-asparagine, 200 mg/L sacarose, 50 mg/L sodium pyruvate, 320 mg/L malic acid, 40 mg/L fumaric acid, 70 mg/L succinic acid, 200 mg/L α-ketoglutaric acid, 300 mg/L citric acid, 1.1 g/L sodium bicarbonate, 5 g/L MES, 0.4 mg/L hemin, 10 mg/L gentamicine pH 5.4 at 37 °C. THP-1 cells were grown in RPMI-1640 medium (Gibco, Leiden, The Netherlands) supplemented with 10% heat inactivated FCS, antibiotics, 1 mM HEPES, 2 mM glutamine and 1 mM sodium pyruvate, pH 7.2 at 37 °C and 5% CO₂.

*L. infantum* promastigotes (MCAN/ES/ 89/IPZ229/1/89) were grown in RPMI-1640 medium (Sigma-Aldrich, St. Louis, MO, USA) supplemented with 10% heat-inactivated fetal calf serum (FCS), antibiotics, and 25 mm HEPES (pH 7.2) at 26 °C.

Drug treatment of amastigotes was performed during the logarithmic growth phase at a concentration of 1×10⁶ parasites/mL at 37 °C for 24 h. Drug treatment of promastigotes was performed during the logarithmic growth phase at a concentration of 2×10⁶ parasites/mL at 26 °C for 24 h. The percentage of living cells was evaluated by flow cytometry by the propidium iodide (PI) exclusion method.¹⁷ Drug concentrations ranged from 0.2 M to 25 M.

### Leishmania infection assay

Human THP-1 monocytic cells were seeded at 1.2 x 10⁵ cells/ml in 24-multidish plates (Nunc, Roskilde, Denmark) and differentiated to macrophages for 24 h in 1 ml of RPMI 1640 medium containing 10 ng/ml of phorbol 12-myristate 13-acetate (PMA) (Sigma-Aldrich, St. Louis, MO). Medium culture was removed, and 1.2 x 10⁶ *Leishmania* amastigotes in 1 ml of THP-1 medium were added to each well. Four hours later, the medium with non-infective amastigotes was completely removed, washed 3 times with 1 phosphate-buffered saline (PBS), and replaced with new THP-1 medium and the corresponding treatment. After 48 h of treatment, medium was removed; THP-1 cells were washed 3 times with 1xPBS and detached with TrypLE Express (Invitrogen, Leiden, The Netherlands) according to the manufacturer's indications. Infection quantification was measured by flow cytometry. Drug concentrations ranged from 0.2 M to 25 M.

### Cytotoxicity assays

Human THP-1 monocytic cells were seeded at 1.2 x 10⁵ cells/ml in 24-multidish plates (Nunc, Roskilde, Denmark) and differentiated to macrophages for 24 h in 1 ml of RPMI 1640 medium containing 10 ng/ml of phorbol 12-myristate 13-acetate (PMA) (Sigma-Aldrich, St. Louis, MO). Human HepG2 cells were seeded at 8 x 10⁵ cells/ml in 24-multidish plates (Nunc, Roskilde, Denmark). After 24 h of drug treatment, both cell lines were washed with PBS and stained with violet crystal solution (0.2% in 2% ethanol) in for 10 min. at room temperature. After dye removal, cells were washed twice with water. Once dried, the wells were treated with 1% SDS for 30 min and the absorbance at 570nm was monitored in a plate reader.

**Table 2. EC₅₀± SEM values for the compounds of formula I-A and I-B on L. infantum promastigotes, extracellular and intracellular amastigotes and cytotoxic activity in macrophage-differentiated THP-1 monocytes and HepG2 cell lines**

| | | | | | |
|---|---|---|---|---|---|
| 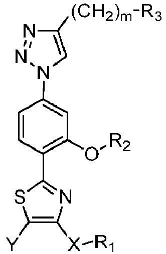 | | | 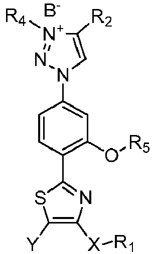 | | |
| **8 (Compounds of formula I-A)** | | | **10 (Compounds of formula I-B)** | | |

| **COMPOUNDS OF FORMULA I-A (8)** | | | | | |
|---|---|---|---|---|---|
| **Compound** | **EC₅₀ (µM) Promastigotes** | **EC₅₀ (µM) axenic Amastigotes** | **EC₅₀(µM) intracellular Amastigotes** | **EC₅₀ (µM) THP-1 dif** | **EC₅₀ (µM) HepG2** |
| **8b** (TRL145) | 44.96 ± 3.51 | 18.24 ± 0.4 | 30.2 ± 1.7 | ND | ND |
| **8e** (TRL146) | 30.54 ± 0.16 | 13.86± 0.6 | 33.6 ± 1.5 | 27.18 ± 1.2 | 20.35 ± 2.37 |
| **8g** (TRL148) | 8.79 ± 0.75 | 22.97 ± 1.86 | 31.2 ± 1.9 | 47.55 ± 3.3 | 33.28 ± 2.7 |
| **8h** (TRL159) | 5.44 ± 0.44 | 13.93 ± 0.93 | ND | 45.73 ± .6 | 43.06 ± 2.65 |
| **8j** (TRL165) | 23.85 ± 0.52 | 25.08 ±0.6 | ND | 28.73 ± 1.6 | 29.47 ± 1.27 |
| **8m** (TRL157) | 9.56 ± 0.44 | 7.1 ± 0.9 | ND | ND | ND |

| **COMPOUNDS OF FORMULA I-B (10)** | | | | | |
|---|---|---|---|---|---|
| **Compound** | **EC₅₀ (µM) Promastigotes** | **EC₅₀ (µM) axenic Amastigotes** | **EC₅₀(µM) intracellular Amastigotes** | **EC₅₀ (µM) THP-1 dif** | **EC₅₀ (µM) HepG2** |
| **10c** (TRL158) | 17.93 ± 3.76 | 12.91 ± 0.51 | ND | ≥75 | ≥75 |
| **10d** (TRL169) | 47.02 ± 2.08 | ≥75 | ND | ≥75 | ≥75 |
| **10e** (TRL160) | 18.71 ± 1.26 | 11.56 ± 1.14 | 19.5 ± 1.0 | 21.55 ± 0.7 | ≥75 |
| **10f** (TRL168) | 6.03 ± 0.49 | 15.51 ± 0.7 | ND | 19.93 ± 1.1 | 69.93 ± 2.97 |

| | | | | | |
|---|---|---|---|---|---|
| *ND non determined | | | | | |

## Claims

1. Compound of formula (**I**), a pharmaceutically acceptable salt, or solvate thereof, 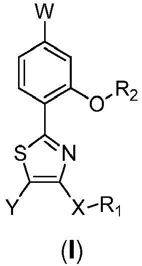 wherein:
X is selected from O, and -(CH₂)ₙ-, wherein n is a whole number in the range of from 0 to 3, and wherein n is 0 it is considered that X is absent;
Y is selected from H, substituted or unsubstituted (C₁-C₁₀)alkyl, substituted or unsubstituted aryl, and substituted or unsubstituted heteroaryl;
R₁ is selected from substituted or unsubstituted (C₁-C₁₀)alkyl, substituted or unsubstituted aryl, and substituted or unsubstituted heteroaryl;
R₂ is selected from substituted or unsubstituted (C₁-C₄)alkyl, wherein the substituent is an heterocyclylalkyl, NHCONH₂, and NHCONHR, wherein R is selected from (C₁-C₄)alkyl; and
W is a substituted or unsubstituted triazol.

2. Compound according to claim 1, wherein X is -(CH₂)ₙ- and n is selected from 0 to 3.

3. Compound according to any preceding claim, wherein Y is H or substituted or unsubstituted aryl, preferably Y is H or phenyl.

4. Compound according to any preceding claim, wherein R₁ is selected from phenyl, naphthyl, biphenyl, dihydrobenzofuranyl, tetrahydroquinolinyl, and dibenzofuranyl.

5. Compound according to any preceding claim, wherein R₂ is selected from CH₃,

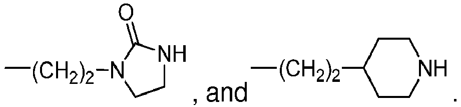

6. Compound according to any preceding claim, wherein W is 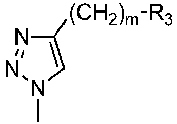 or 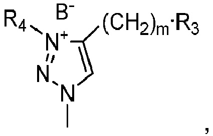 and wherein:
R₃ is selected from NH₃⁺A⁻, and an optionally substituted guanidinium salt;
R₄ is selected from substituted or unsubstituted (C₁-C₅)alkyl and substituted or unsubstituted aryl;
m is selected from 1 to 5, preferably m is 3 or 4; and
A⁻ and B⁻ independently are a counter-ion, preferably selected from trifluoroacetic acid anion, hydrochloric acid anion, or sulphuric acid anion, acetic acid anion, trifluoromethanesulfonic acid anion, hydroiodic acid anion, and benzenesulfonic acid anion.

7. Compound according to claim 1, of formula (**I-A**) or (**I-B**), a pharmaceutically acceptable salt, or solvate thereof, 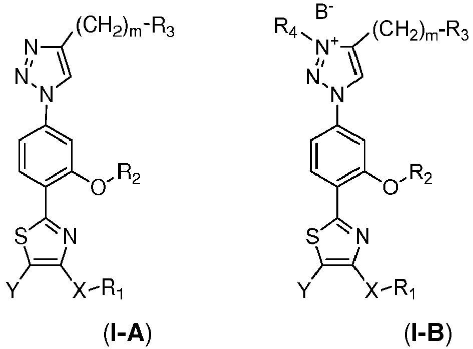 wherein:
X, Y, R₁, R₂, m, R₃, R₄, and B⁻ are as defined in claims 1 to 6.

8. Compound according to claims 7, wherein R₃ is NH₃⁺TFA⁻.

9. Compound according to any of claims 8 to 9, wherein R₄ is selected from methyl, ethyl, propyl, and butyl, preferably R₄ is methyl or ethyl.

10. Compound according to claim 1, selected from the group as listed:
1-(2-(5-(4-(4-Aminobutyl)-1H-1,2,3-triazol-1 -yl)-2-(4-isopropoxythiazol-2-yl)phenoxy)ethyl)imidazolidin-2-one 2,2,2 trifluoroacetate,
1-(2-(5-(4-(4-Aminobutyl)-1 H-1,2,3-triazol-1 -yl)-2-(4-neopentylthiazol-2-yl)phenoxy)ethyl)imidazolidin-2-one 2,2,2 trifluoroacetate,
1-(2-(5-(4-(4-aminobutyl)-1H-1,2,3-triazol-1-yl)-2-(4-phenylthiazol-2-yl)phenoxy)ethyl)imidazolidin-2-one 2,2,2 trifluoroacetate,
1-(2-(5-(4-(4-Aminobutyl)-1H-1,2,3-triazol-1 -yl)-2-(4-benzylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-phenethylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(3-phenylpropyl)thiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(naphthalen-2-yl)ethyl)thiazol-2-yl)phenoxy)ethyl)imidazolidin-2-one 2,2,2 trifluoroacetate,
1-(2-(2-(4-(2-([1,1'-Biphenyl]-4-yl)ethyl)thiazol-2-yl)-5-(4-(4-aminobutyl)-1H-1,2,3-triazol-1-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(3,4-dihydro-2H-1λ²-quinolin-5-yl)ethyl)thiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(2-(2,3-dihydrobenzofuran-6-yl)ethyl)thiazol-2-yl)phenoxy)ethyl)imidazolidin-2-one 2,2,2 trifluoroacetate,
1-(2-(5-(4-(4-aminobutyl)-1H-1,2,3-triazol-1-yl)-2-(4-(2-(dibenzo[b,d]furan-1-yl)ethyl)thiazol-2-yl)phenoxy)ethyl)imidazolidin-2-one 2,2,2 trifluoroacetate,
4-(1-(4-(4-phenethylthiazol-2-yl)-3-(2-(piperidin-4-yl)ethoxy)phenyl)-1H-1,2,3-triazol-4-yl)butan-1-amine 2,2,2 trifluoroacetate,
4-(1-(3-Methoxy-4-(4-phenethylthiazol-2-yl)phenyl)-1H-1,2,3-triazol-4-yl)butan-1-amine 2,2,2 trifluoroacetate,
1-(2-(5-(4-(3-aminopropyl)-1H-1,2,3-triazol-1-yl)-2-(4-phenethylthiazol-2-yl)phenoxy)ethyl)imidazolidin-2-one 2,2,2 trifluoroacetate,
1-(2-(2-(4-(2-([1,1'-Biphenyl]-4-yl)ethyl)thiazol-2-yl)-5-(4-(3-aminopropyl)-1H-1,2,3-triazol-1-yl)phenoxy)ethyl)imidazolidin-2-one 2,2,2 trifluoroacetate,
1-(2-(5-(4-(4-Aminobutyl)-1H-1,2,3-triazol-1-yl)-2-(4,5-diphenylthiazol-2-yl)phenoxy)ethyl)imidazolidin-2-one 2,2,2 trifluoroacetate,
1-(2-(5-(4-(4-Aminobutyl)-1H-1,2,3-triazol-1-yl)-2-(4-(3-phenylpropyl)thiazol-2-yl)phenoxy)ethyl)imidazolidin-2-one hydrochloride salt,
4-(4-Ammoniobutyl)-3-methyl-1-(3-(2-(2-oxoimidazolidin-1-yl)ethoxy)-4-(4-phenylthiazol-2-yl)phenyl)-1*H*-1,2,3-triazol-3-ium 2,2,2-trifluoroacetate trifluoromethanesulfonate,
4-(4-Ammoniobutyl)-3-methyl-1-(3-(2-(2-oxoimidazolidin-1-yl)ethoxy)-4-(4-phenethylthiazol-2-yl)phenyl)-1*H*-1,2,3-triazol-3-ium 2,2,2-trifluoroacetate trifluoromethanesulfonate,
4-(4-Ammoniobutyl)-3-methyl-1-(4-(4-(2-(naphthalen-2-yl)ethyl)thiazol-2-yl)-3-(2-(2-oxoimidazolidin-1-yl)ethoxy)phenyl)-1*H*-1,2,3-triazol-3-ium 2,2,2-trifluoroacetate trifluoromethanesulfonate,
4-(4-Ammoniobutyl)-1-(4-(4-(2-(2,3-dihydrobenzofuran-5-yl)ethyl)thiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-methyl-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(4-ammoniobutyl)-3-ethyl-1*H*-1,2,3-triazol-3-ium 2,2,2-trifluoroacetate trifluoromethanesulfonate,
4-(4-Ammoniobutyl)-1-(4-(4-(4-methoxyphenyl)thiazol-2-yl)-3-(2-(2-oxoimidazolidin-1-yl)ethoxy)phenyl)-3-methyl-1 H-1,2,3-triazol-3-ium 2,2,2-trifluoroacetate trifluoromethanesulfonate,
4-(4-Ammoniobutyl)-1-(4-(4-(4-(diethylamino)phenyl)thiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate,
4-(3-Ammoniopropyl)-3-methyl-1-(3-(2-(2-oxoimidazolidin-1-yl)ethoxy)-4-(4-phenethylthiazol-2-yl)phenyl)-1*H*-1,2,3-triazol-3-ium 2,2,2-trifluoroacetate trifluoromethanesulfonate,
1-(4-(4-(2-([1,1'-Biphenyl]-4-yl)ethyl)thiazol-2-yl)-3-(2-(2-oxoimidazolidin-1-yl)ethoxy)phenyl)-4-(3-ammoniopropyl)-3-methyl-1*H*-1,2,3-triazol-3-ium 2,2,2-trifluoroacetate trifluoromethanesulfonate,
Mono (4-(2-(5-(4-(4-ammoniobutyl)-3-methyl-1*H*-1,2,3-triazol-3-ium-1-yl)-2-(4-phenethylthiazol-2-yl)phenoxy)ethyl)piperidin-1-ium) mono(2,2,2-trifluoroacetate) mono(trifluoromethanesulfonate), and
4-(4-Ammoniobutyl)-1-(4-(4,5-diphenylthiazol-2-yl)-3-(2-(2-oxoimidazolidin-1-yl)ethoxy)phenyl)-3-methyl-1*H*-1,2,3-triazol-3-ium 2,2,2-trifluoroacetate trifluoromethanesulfonate.

11. Compound of formula (**VI**) or (**VII**), a pharmaceutically acceptable salt, or solvate thereof, 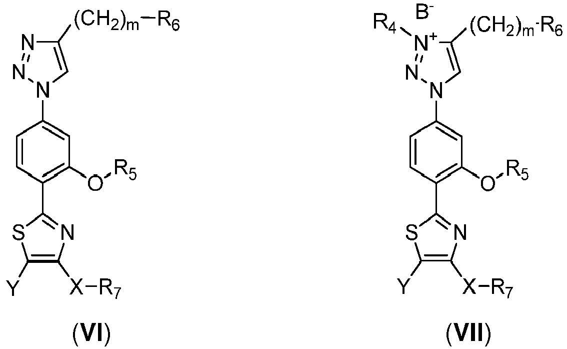 wherein:
X is absent or is selected from O, -(CH₂)ₙ-, wherein n is a whole number in the range of from 1 to 3, -(CH=CH)-, and -(CH=CH)-CH₂-;
Y is selected from H, substituted or unsubstituted (C₁-C₁₀)alkyl, substituted or unsubstituted aryl, and substituted or unsubstituted heteroaryl;
m is a whole number in the range of from 1 to 5, preferably m is 3 or 4;
R₄ is selected from substituted or unsubstituted (C₁-C₅)alkyl and substituted or unsubstituted aryl;
R₅ is selected from substituted or unsubstituted (C₁-C₄)alkyl, wherein the substituent is an heterocyclylalkyl, NHCONH₂, and NHCONHR, wherein R is selected from (C₁-C₄)alkyl;
R₆ is selected from NHPG, in which PG is an amino-protecting group, and a protected guanidinium group; and
R₇ is selected from substituted or unsubstituted (C₁-C₁₀)alkyl, substituted or unsubstituted aryl, and substituted or unsubstituted heteroaryl; and
B- is a counter-ion selected from trifluoromethanesulfonic acid anion, benzenesulfonic acid anion, trifluoroacetic acid, or sulphuric acid anion.

12. Pharmaceutical composition comprising at least one of the compounds of formula (**I**), (**I-A**) or (**I-B**) as defined in any of claims 1 to 10, a pharmaceutically acceptable salt, or solvate thereof, and one or more pharmaceutically acceptable excipients and/or carriers.

13. Compound of formula (**I**), (**I-A**) or (**I-B**), as defined in claims 1 to 10, their pharmaceutically acceptable salts, or solvates thereof, for use as a medicament.

14. Compound of formula (**I**), (**I-A**) or (**I-B**), as defined in claims 1 to 10, their pharmaceutically acceptable salts, or solvates thereof, for use in the treatment of a disease related to an infection caused by a parasite.

15. Compound for use according to claim 14, wherein the disease is selected from Chagas disease, trypanosomiasis, and leishmaniasis.
